# EUROPEAN PATENT APPLICATION

(11) **EP 4 400 504 A1**
(43) Date of publication of application: **17.07.2024**
(21) Application number: 22866640.0
(22) Date of filing: 07.09.2022
(51) Int. Cl.: C07D 495/16, A61K 31/4985, A61P 25/00, A61P 15/10, A61P 35/00

(54) **THIOPHENE RING COMPOUND, PREPARATION METHOD THEREFOR AND APPLICATION THEREOF**

(30) Priority: 07.09.2021 CN 202111052987
(71) Applicant: Center for Excellence in Molecular Cell Science, Chinese Academy of Sciences, Shanghai 200031 (CN); ShanghaiTech University, Shanghai 201210 (CN)
(72) Inventor: WANG, Sheng, Shanghai 200031 (CN); CHENG, Jianjun, Shanghai 201210 (CN); FAN, Luyu, Shanghai 200031 (CN); WANG, Huan, Shanghai 201210 (CN); CHEN, Zhangcheng, Shanghai 200031 (CN); YU, Jing, Shanghai 200031 (CN); DUAN, Wenwen, Shanghai 201210 (CN); CAO, Dongmei, Shanghai 200031 (CN)
(74) Representative: Calysta NV
(86) International application number: PCT/CN2022/117539
(87) International publication number: WO 2023/036177

(57) **Abstract**

Disclosed in the present invention are a thiophene ring compound, a preparation method therefor and an application thereof. The structure of the thiophene ring compound of the present invention is as shown in formula I. The compound of the present invention has good affinity and agonistic activity against at least one of a dopamine receptor and a 5-hydroxytryptamine receptor.

## Description

The present application claims the priority of Chinese patent application No. 2021110529878 with an application date of September 7, 2021. The contents of the above Chinese patent application are incorporated herein by reference in its entirety.

### TECHNICAL FIELD

The present disclosure relates to a thiophene-fused cyclic compound, a preparation method therefor, and a use thereof.

### BACKGROUND

G protein-coupled receptors (GPCRs) are the most important family of drug targets. According to statistics, as of 2017, 34% of all small molecule drugs approved by the U.S. FDA target GPCRs (Nature Review Drug Discovery, 2017, 16, 19-34). As the largest receptor family encoded by the human genome, GPCRs comprise approximately 1,000 different receptors, including more than 300 potential drug targets. Research has confirmed that GPCRs are closely related to the onset and progression of numerous diseases, including neuropsychiatric disorders (such as schizophrenia and pain), cardiovascular diseases (such as hypertension and heart failure), metabolic diseases (such as diabetes and obesity), immune diseases, and cancer. Among the many GPCR receptors, monoamine GPCR receptors, such as dopamine receptors and 5-hydroxytryptamine receptors, are validated drug targets. Many drugs, especially those for psychiatric disorders, primarily target these receptors.

Dopamine receptors have five subtypes (D₁₋₅), where D₁ and D₅ belong to the D₁-like class of receptors, which are mainly coupled with Gₛ proteins, and increase intracellular cAMP levels after activation; D₂, D₃, and D₄ belong to the D₂-like class of receptors, which are mainly coupled with Gᵢ proteins, and decrease intracellular cAMP levels after activation. The dopaminergic signaling pathway has been a focus of neuroscience research in recent decades, with abnormalities in this pathway being associated with various diseases, including schizophrenia and Parkinson's disease. Small molecule antagonists or partial agonists targeting dopamine D₂ receptors are effective antipsychotics, such as haloperidol, olanzapine, aripiprazole, and cariprazine, all of which act at dopamine D₂ receptors as their primary targets. Dopamine D₃ receptors, which belong to the same subfamily as D₂, are also important targets for many antipsychotics. Highly selective D₃ receptor antagonists or partial agonists also have potential for the treatment of drug addiction. Dopamine D₁, D₄, and D₅ receptors have also been identified as potential drug targets.

The 5-hydroxytryptamine receptor includes 14 subtypes, and except for 5-HT₃, which is an ion channel, the other 13 subtypes are all GPCR receptors. Among them, the 5-hydroxytryptamine 2A receptor (5-HT_{2A}) is another important drug target for the treatment of schizophrenia. Antagonist or inverse agonist activity at 5-HT_{2A} is an important pharmacological characteristic of "atypical" antipsychotics. In addition, selective 5-HT_{2A} receptor inverse agonists, such as pimavanserin, which was approved by the U.S. FDA in 2018, have been proven to have a good therapeutic effect in controlling hallucination symptoms in patients with Parkinson's disease. Similarly, the 5-HT_{1A} receptor has also been proven to be related to physiological functions such as mood, pain, and movement. Many anxiolytics (*e.g*., buspirone), antidepressants (*e.g.,* vortioxetine), and antipsychotics (*e.g.,* aripiprazole) also possess pharmacological characteristics of agonists or partial agonists of the 5-HT_{1A} receptor.

For monoamine GPCR receptors such as dopamine and 5-hydroxytryptamine receptors, the "4-arylpiperazine" or "4-arylpiperidine" substructures have been found to be the most commonly used privileged scaffolds. For example, drugs such as risperidone, ziprasidone, aripiprazole, and cariprazine all feature the "4-arylpiperazine" substructures. In recent years, a large number of similarly structured drug-like small molecules have been reported (ChemMedChem 2011, 6, 1152-1162; Chem. Rev., 2013, 113, PR123-PR178).

Krogsgaard-Larsen et al. reported a compound A with an aza-ergoline skeleton, whose derivative A1 has binding activity to the 5-HT₆ receptor, derivative A2 has agonist activity to the dopamine D₂ receptor, and derivative A3 has partial agonist activity to the dopamine D₂ receptor (Krogsgaard-Larsen et al., J. Med. Chem. 2014, 57, 5823-5828; WO2011088836; WO2011088838). The target selectivity, *in vivo* activity in animals, and other drug-like properties of these compounds have not been reported.

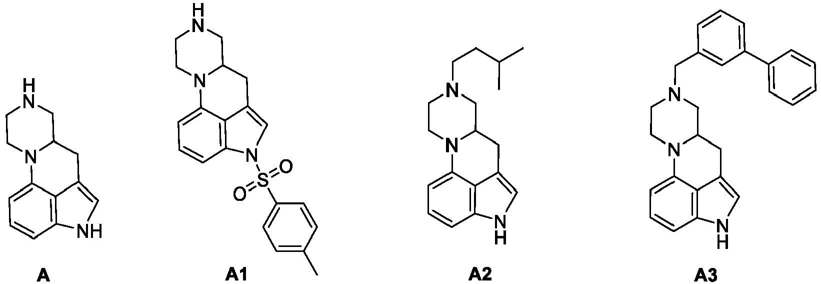

### CONTENT OF THE PRESENT INVENTION

The technical problem addressed by the present disclosure is the limited classes of compounds with affinity and agonistic activity for dopamine receptors and/or 5-hydroxytryptamine receptors. To this end, the present disclosure provides a thiophene-fused cyclic compound, a preparation method therefor, and a use thereof. The compounds of the present disclosure exhibit good affinity and/or agonistic activity for at least one of the dopamine receptors and 5-hydroxytryptamine receptors.

The present disclosure provides a compound of formula I, a pharmaceutically acceptable salt thereof, a solvate thereof, or a solvate of the pharmaceutically acceptable salt thereof: 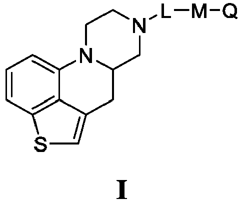
wherein L is absent, C₁₋₁₀ alkylene, C₂₋₁₀ alkenylene, C₂₋₁₀ alkynylene, or -C₁₋₆ alkylene-C₃₋₆ cycloalkylene-;
M is absent, -O-, -NH-, -CH₂-, -(CH-OH)-, or -C(=O)-;
Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, 5- to 12-membered heteroaryl substituted by one or more than one Q¹⁻², or - C(=O)R¹; the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3;
Q¹⁻¹ is halogen, C₁₋₄ alkyl, C₁₋₄ alkoxy, C₆₋₁₈ aryl, 5- to 12-membered heterocycloalkyl, 5- to 12-membered oxo-heterocycloalkyl, hydroxyl, or C₆₋₁₈ aryl substituted by one or more than one Q^{1-1-1.}, the heteroatoms in the 5- to 12-membered heterocycloalkyl are one or more than one of N, S, or O, and the number is 1, 2, or 3;
Q¹⁻² is halogen, C₁₋₄ alkyl, C₁₋₄ alkoxy, 5- to 12-membered heterocycloalkyl, C₆₋₁₈ aryl substituted by one or more than one Q¹⁻²⁻¹, C₆₋₁₈ aryl, or hydroxyl; the heteroatoms in the 5- to 12-membered heterocycloalkyl are one or more than one of N, S, or O, and the number is 1, 2, or 3;
R¹ is -NR¹⁻¹R¹⁻², 3- to 6-membered heterocycloalkyl, C₆₋₁₈ aryl, 5- to 12-membered heteroaryl, 5- to 12-membered heteroaryl substituted by one or more than one R¹⁻⁴, or C₆₋₁₈ aryl substituted by one or more than one R¹⁻³; the heteroatoms in the 3- to 6-membered heterocycloalkyl are one or more than one of N, S, or O, and the number is 1, 2, or 3; the heteroatoms in the 5- to 12-membered heteroaryl are one or more than one of N, S, or O, and the number is 1, 2, or 3;
R¹⁻¹ and R¹⁻² are independently hydrogen or C₁₋₄ alkyl;
R¹⁻³, Q¹⁻¹⁻¹, and Q¹⁻²⁻¹ are independently halogen;
R¹⁻⁴ is halogen or oxo.

In one embodiment, in the compound of formula I, the pharmaceutically acceptable salt thereof, the solvate thereof, or the solvate of the pharmaceutically acceptable salt thereof, some groups are defined as follows and other groups are as defined in any embodiment of the present disclosure, which is hereinafter referred to as "in one embodiment".

In one embodiment, the compound of formula I is a compound of formula Ie, a compound of formula If, or a mixture thereof (such as an equimolar mixture of the compound of formula Ie and the compound of formula If);

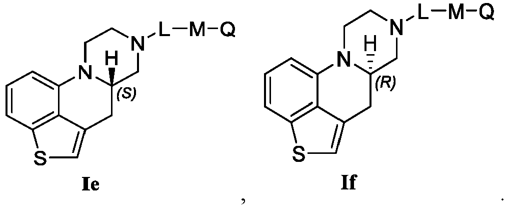

In one embodiment, the compound of formula I is a compound of formula Ia, Ib, Ic, or Id:

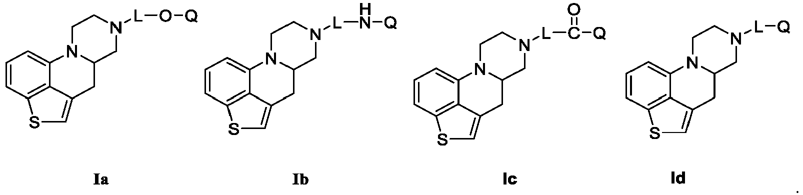

In one embodiment, the compound of formula I is a compound of formula Ia: 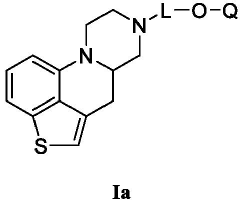
L is C₁₋₁₀ alkylene;
Q is hydrogen, C₆₋₁₈ aryl, or 5- to 12-membered heteroaryl; the heteroatoms in the 5-to 12-membered heteroaryl are one or more than one of N, S, or O, and the number is 1, 2, or 3.

In one embodiment, the molecular structure of formula I is as shown in formula Ib: 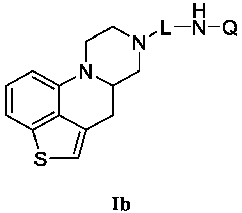
L is C₁₋₁₀ alkylene or -C₁₋₆ alkylene-C₃₋₆ cycloalkylene-;
Q is -C(=O)R¹;
R¹ is -NR¹R¹⁻², 3- to 6-membered heterocycloalkyl, C₆₋₁₈ aryl, 5- to 12-membered heteroaryl, 5- to 12-membered heteroaryl substituted by one or more than one R¹⁻⁴, or C₆₋₁₈ aryl substituted by one or more than one R¹⁻³; the heteroatoms in the 3- to 6-membered heterocycloalkyl are one or more than one of N, S, or O, and the number is 1, 2, or 3; the heteroatoms in the 5- to 12-membered heteroaryl are one or more than one of N, S, or O, and the number is 1, 2, or 3;
R¹⁻¹ and R¹⁻² are independently C₁₋₄ alkyl;
R¹⁻³ and R¹⁻⁴ are independently halogen.

In one embodiment, the molecular structure of formula I is as shown in formula Ic: 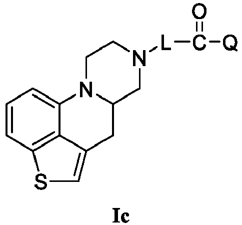
L is C₁₋₁₀ alkylene;
Q is C₆₋₁₈ aryl or C₆₋₁₈ aryl substituted by one or more than one Q¹⁻¹, Q¹⁻¹ is halogen.

In one embodiment, the molecular structure of formula I is as shown in formula Id: 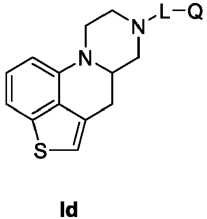
L is absent, C₁₋₁₀ alkylene, or -C₁₋₆ alkylene-C₃₋₆ cycloalkylene-;
Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, or 5- to 12-membered heteroaryl substituted by one or more than one Q^{1-2.}, the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3;
Q¹⁻¹ is halogen, C₁₋₄ alkyl, C₁₋₄ alkoxy, C₆₋₁₈ aryl, 5- to 12-membered oxo-heterocycloalkyl, 5- to 12-membered heterocycloalkyl, or C₆₋₁₈ aryl substituted by one or more than one Q^{1-1-1.}, the heteroatoms in the 5- to 12-membered heterocycloalkyl are one or more than one of N, S, or O, and the number is 1, 2, or 3;
Q¹⁻² is halogen, C₁₋₄ alkyl, C₁₋₄ alkoxy, 5- to 12-membered heterocycloalkyl, C₆₋₁₈ aryl substituted by one or more than one Q¹⁻²⁻¹, C₆₋₁₈ aryl, or hydroxyl; the heteroatoms in the 5- to 12-membered heterocycloalkyl are one or more than one of N, S, or O, and the number is 1, 2, or 3;
Q¹⁻¹⁻¹ and Q¹⁻²⁻¹ are independently halogen.

In one embodiment, L is C₁₋₁₀ alkylene or -C₁₋₆ alkylene-C₃₋₆ cycloalkylene-;
M is absent, -O-, -NH-, -CH₂-, or -C(=O)-;
Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, 5- to 12-membered heteroaryl substituted by one or more than one Q¹⁻², or - C(=O)R¹; the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3;
Q¹¹ is halogen, C₁₋₄ alkyl, C₆₋₁₈ aryl, 5- to 12-membered heterocycloalkyl, 5- to 12-membered oxo-heterocycloalkyl, or C₆₋₁₈ aryl substituted by one or more than one Q^{1-1-1.}, the heteroatoms in the 5- to 12-membered heterocycloalkyl are one or more than one of N, S, or O, and the number is 1, 2, or 3;
Q¹⁻² is C₁₋₄ alkyl, C₁₋₄ alkoxy, C₆₋₁₈ aryl substituted by one or more than one Q¹⁻²⁻¹, or C₆₋₁₈ aryl;
R¹ is -NR¹⁻¹R¹⁻², 3- to 6-membered heterocycloalkyl, C₆₋₁₈ aryl, or C₆₋₁₈ aryl substituted by one R¹⁻³; the heteroatom in the 3- to 6-membered heterocycloalkyl is N, and the number is 1;
R¹⁻¹ and R¹⁻² are independently C₁₋₄ alkyl;
R¹⁻³, Q^{1-1-1.}, and Q¹⁻²⁻¹ are independently halogen;
R¹⁻⁴ is halogen or oxo.

In one embodiment, L is C₁₋₁₀ alkylene or -C₁₋₆ alkylene-C₃₋₆ cycloalkylene-.

In one embodiment, M is absent, -O-, -NH-, -CH₂-, or -C(=O)-.

In one embodiment, Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, 5- to 12-membered heteroaryl substituted by one or more than one Q¹⁻², or -C(=O)R¹; the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3.

In one embodiment, Q¹⁻¹ is halogen, C₁₋₄ alkyl, C₆₋₁₈ aryl, 5- to 12-membered heterocycloalkyl, 5- to 12-membered oxo-heterocycloalkyl, or C₆₋₁₈ aryl substituted by one or more than one Q^{1-1-1.}, the heteroatoms in the 5- to 12-membered heterocycloalkyl are one or more than one of N, S, or O, and the number is 1, 2, or 3.

In one embodiment, Q¹⁻² is C₁₋₄ alkyl, C₁₋₄ alkoxy, C₆₋₁₈ aryl substituted by one or more than one Q¹⁻²⁻¹, or C₆₋₁₈ aryl.

In one embodiment, R¹ is -NR¹⁻¹R¹⁻², 3- to 6-membered heterocycloalkyl, C₆₋₁₈ aryl, or C₆₋₁₈ aryl substituted by one R¹⁻³; the heteroatom in the 3- to 6-membered heterocycloalkyl is N, and the number is 1.

In one embodiment, in L, the C₁₋₁₀ alkylene is C₁₋₄ alkylene, such as methylene,

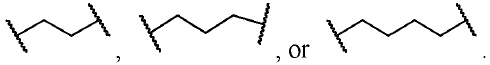

In one embodiment, in case of the -C₁₋₆ alkylene-C₃₋₆ cycloalkylene- in L, the C₁₋₆ alkylene is connected to N, and the C₃₋₆ cycloalkylene is connected to Q.

In one embodiment, in case of the -C₁₋₆ alkylene-C₃₋₆ cycloalkylene- in L, the C₁₋₆ alkylene is C₁₋₄ alkylene, such as methylene or ethylene.

In one embodiment, in case of the -C₁₋₆ alkylene-C₃₋₆ cycloalkylene- in L, the C₃₋₆ cycloalkylene is cyclopropylene, cyclobutylene, cyclopentylene, or cyclohexylene, such as cyclopropylene or cyclohexylene. Furthermore, the -C₁₋₆ alkylene-C₃₋₆ cycloalkylene is - methylene-cyclopropylene- or -ethylene-cyclohexylene-, such as 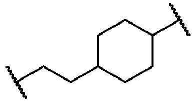 or

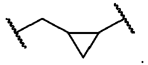

In one embodiment, in Q, the C₆₋₁₈ aryl is C₆₋₁₀ aryl (such as phenyl or naphthyl), and can further be phenyl.

In one embodiment, in Q, the C₆₋₁₈ aryl, as described in the C₆₋₁₈ aryl substituted by one or more than one Q¹⁻¹, is C₆₋₁₀ aryl (such as phenyl or naphthyl). Furthermore, the C₆₋₁₈ aryl substituted by one or more than one Q¹⁻¹ can be phenyl substituted by fluorine or phenyl substituted by imidazolidinone, such as

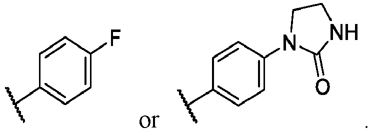

In one embodiment, in Q, the 5- to 12-membered heteroaryl has 1 or 2 heteroatoms. Furthermore, the 5- to 12-membered heteroaryl can be thienyl, pyridyl, benzothiazolyl, or benzodioxolane, such as

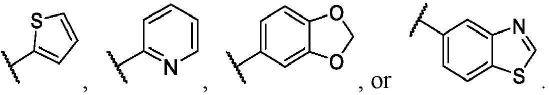

In one embodiment, in Q, the 5- to 12-membered heteroaryl, as described in the 5- to 12-membered heteroaryl substituted by one or more than one Q¹⁻¹, can be pyridyl, tetrahydro-benzazepine, dihydroisoquinolinyl, or indolyl. Furthermore, the 5- to 12-membered heteroaryl substituted by one or more than one Q¹⁻¹⁻¹ can be pyridyl substituted by fluorinesubstituted phenyl, indolyl substituted by methoxy, indolyl substituted by oxo, dihydroisoquinolinyl substituted by oxo, or tetrahydro-benzazepine substituted by oxo, such as

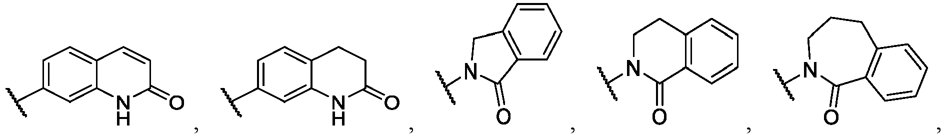

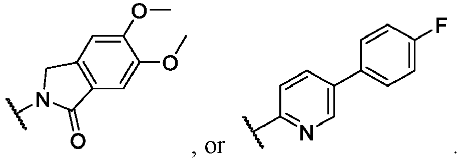

In one embodiment, in Q, the 5- to 12-membered heterocycloalkyl can be piperidinyl, pyrrolidinyl, imidazolidinyl, or hexahydroisoindolyl, such as 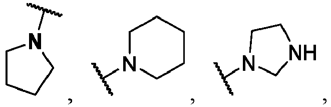 or

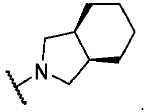

In one embodiment, in Q, the 5- to 12-membered oxo-heterocycloalkyl can be oxopiperidinyl, oxo-pyrrolidinyl, oxo-imidazolidinyl, or oxo-hexahydroisoindolyl; the 5- to 12-membered oxo-heterocycloalkyl can be

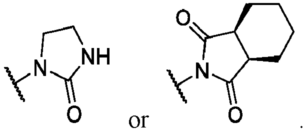

In one embodiment, in Q¹⁻¹, the halogen can be F, Cl, Br, or I, such as F.

In one embodiment, in Q¹⁻¹, the C₁₋₄ alkyl is methyl, ethyl, *n*-propyl, isopropyl, *n*-butyl, isobutyl, *sec-*butyl, or *tert*-butyl, such as methyl or ethyl.

In one embodiment, in Q¹⁻¹, the C₁₋₄ alkoxy is methoxy, ethoxy, *n*-propoxy, isopropoxy, *n-*butoxy, isobutoxy, *sec*-butoxy, or *tert*-butoxy, such as methoxy or ethoxy.

In one embodiment, in Q¹⁻¹, the 5- to 12-membered heterocycloalkyl can be imidazolidinyl, such as

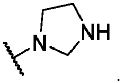

In one embodiment, in Q¹⁻¹, the 5- to 12-membered oxo-heterocycloalkyl can be oxo-imidazolidinyl, such as

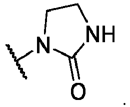

In one embodiment, in Q¹⁻¹, the C₆₋₁₈ aryl is C₆₋₁₀ aryl (such as phenyl or naphthyl), and can further be phenyl.

In one embodiment, in Q¹⁻¹, the C₆₋₁₈ aryl, as described in the C₆₋₁₈ aryl substituted by one or more than one Q^{1-1-1.}, is C₆₋₁₀ aryl (such as phenyl or naphthyl). Furthermore, the C₆₋₁₈ aryl substituted by one or more than one Q¹⁻¹⁻¹ can be phenyl substituted by fluorine, such as

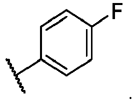

In one embodiment, in Q¹⁻², the C₁₋₄ alkyl is methyl, ethyl, *n*-propyl, isopropyl, *n*-butyl, isobutyl, *sec*-butyl, or *tert*-butyl, such as methyl or ethyl.

In one embodiment, in Q¹⁻², the C₁₋₄ alkoxy is methoxy, ethoxy, *n*-propoxy, isopropoxy, *n*-butoxy, isobutoxy, sec-butoxy, or *tert*-butoxy, such as methoxy or ethoxy.

In one embodiment, in Q¹⁻², the C₆₋₁₈ aryl is C₆₋₁₀ aryl (such as phenyl or naphthyl), and can further be phenyl.

In one embodiment, in Q¹⁻¹, the C₆₋₁₈ aryl, as described in the C₆₋₁₈ aryl substituted by one or more than one Q¹⁻²⁻¹, is C₆₋₁₀ aryl (such as phenyl or naphthyl). Furthermore, the C₆₋₁₈ aryl substituted by one or more than one Q¹⁻²⁻¹ can be phenyl substituted by fluorine, such as

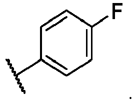

In one embodiment, in R¹, the 3- to 6-membered heterocycloalkyl can be piperidinyl, tetrahydropyranyl, or pyrrolidinyl, such as

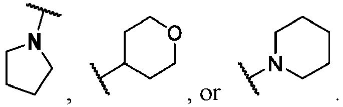

In one embodiment, in R¹, the C₆₋₁₈ aryl is C₆₋₁₀ aryl (such as phenyl or naphthyl), and can further be phenyl.

In one embodiment, in R¹, the C₆₋₁₈ aryl, as described in the C₆₋₁₈ aryl substituted by one or more than one R¹⁻³, is C₆₋₁₀ aryl (such as phenyl or naphthyl). Furthermore, the C₆₋₁₈ aryl substituted by one or more than one R¹⁻³ can be phenyl substituted by fluorine, such as

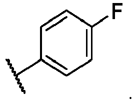

In one embodiment, in R¹⁻¹, the C₁₋₄ alkyl is methyl, ethyl, *n*-propyl, isopropyl, *n*-butyl, isobutyl, *sec*-butyl, or *tert*-butyl, such as methyl or ethyl.

In one embodiment, in R¹⁻², the C₁₋₄ alkyl is methyl, ethyl, *n*-propyl, isopropyl, *n*-butyl, isobutyl, *sec*-butyl, or *tert*-butyl, such as methyl or ethyl.

In one embodiment, in R¹⁻³, the halogen can be F, Cl, Br, or I, such as F.

In one embodiment, in Q^{1-1-1.}, the halogen can be F, Cl, Br, or I, such as F.

In one embodiment, in Q¹⁻²⁻¹, the halogen can be F, Cl, Br, or I, such as F.

In one embodiment, in R¹⁻⁴, the halogen is F, Cl, Br, or I, such as F.

In one embodiment, L is C₁₋₄ alkylene or -C₁₋₄ alkylene-C₃₋₆ cycloalkylene.

In one embodiment, M is absent, -O-, -NH-, -CH₂-, or -C(=O)-.

In one embodiment, -L-M- is absent,

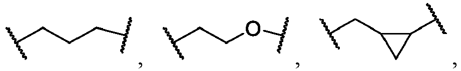

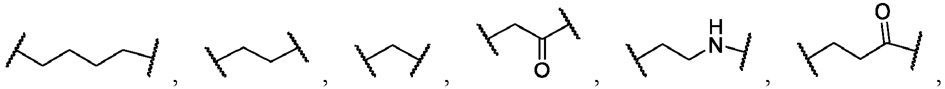

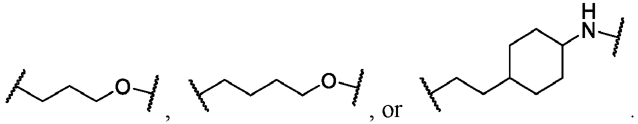

In one embodiment, Q is hydrogen, hydroxyl, cyclopropyl,

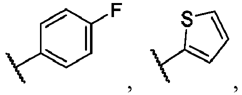

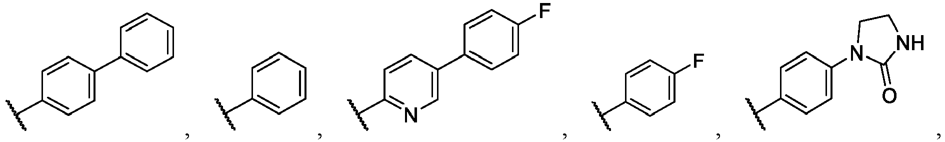

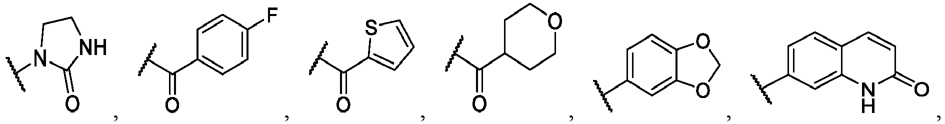

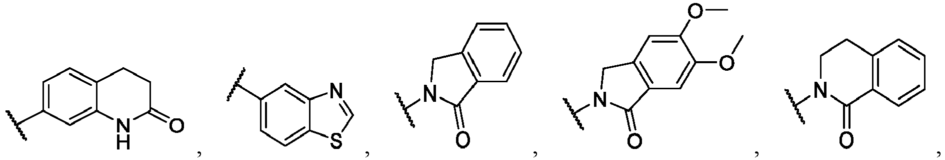

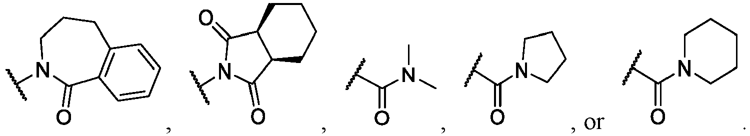

In one embodiment, the compound of formula I is selected from any one of the following compounds: 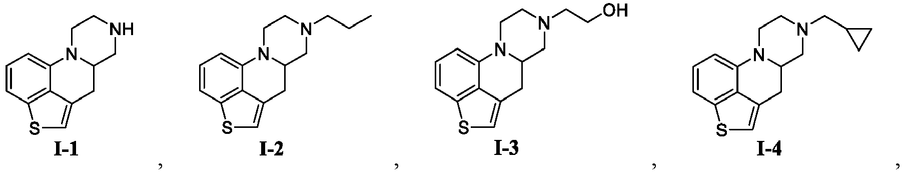 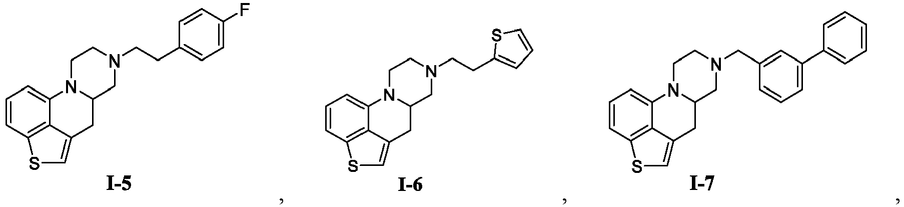 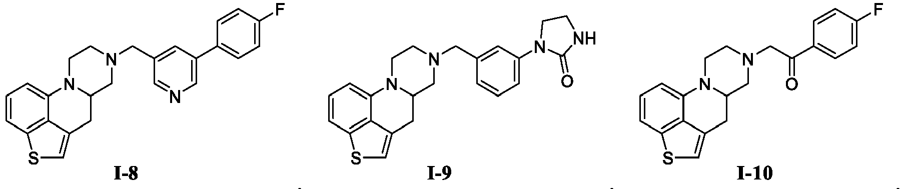 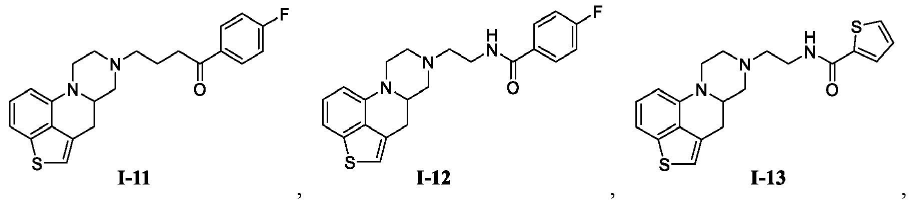 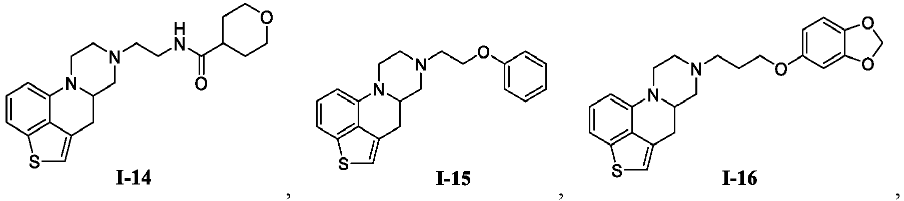 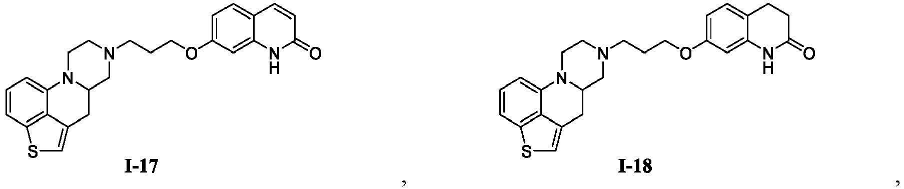 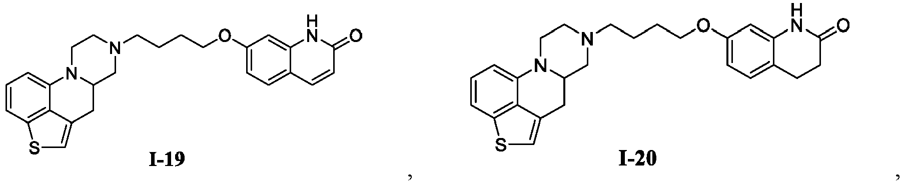 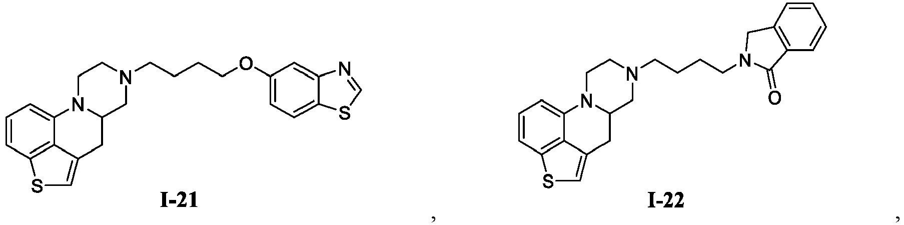 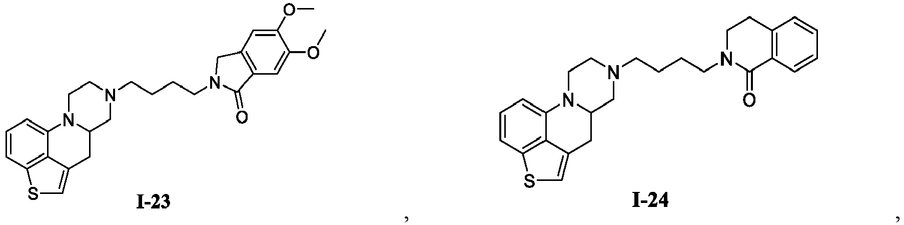 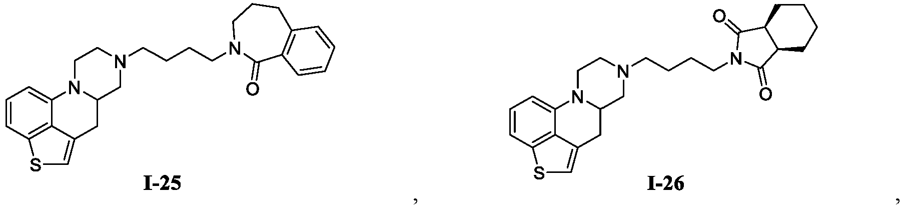 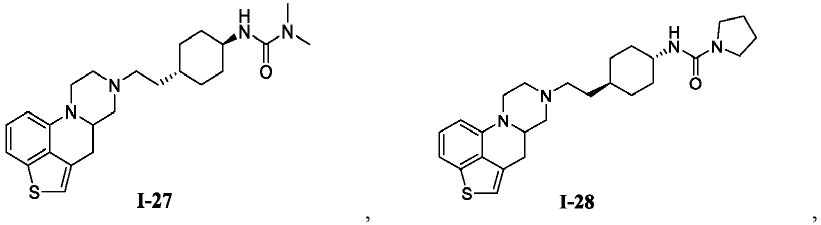 or

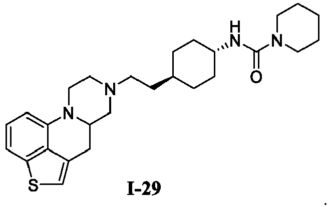

The present disclosure also provides a compound of any one of the following formulas:

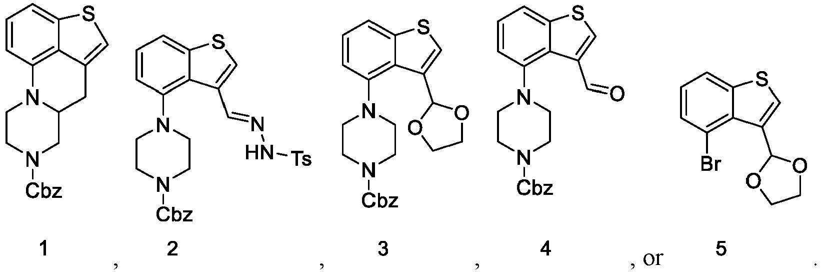

The present disclosure provides a preparation method for the compound of formula I, the pharmaceutically acceptable salt thereof, the solvate thereof, or the solvate of the pharmaceutically acceptable salt thereof, comprising the following steps:

in the presence of an alkaline reagent, carrying out an alkylation reaction between a compound of formula II and a compound of formula III in a solvent as shown below to obtain the compound of formula I; 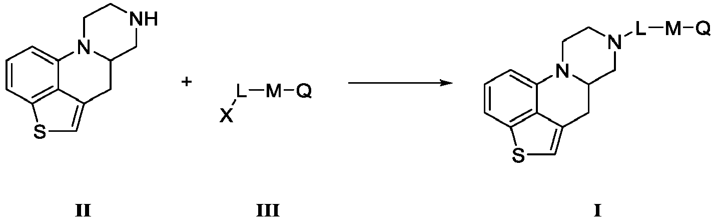 wherein X is halogen; L, M, Q, and R are as previously defined.

The conditions and operations for the alkylation reaction can be those which are conventional for such reactions in the art, with the following conditions particularly preferred in the present disclosure:

The alkaline reagent is, for example, K₂CO₃ (*e.g.,* the molar ratio of the alkaline reagent to the compound of formula II is 6: 1).

The solvent is, for example, tetrahydrofuran and dimethyl sulfoxide (*e.g*., the volume ratio of the two is 3: 1).

The temperature for the alkylation reaction is, for example, 60°C.

The halogen is F, Cl, Br, or I.

The present disclosure also provides a pharmaceutical composition comprising the compound of formula I, the pharmaceutically acceptable salt thereof, the solvate thereof, or the solvate of the pharmaceutically acceptable salt thereof, and a pharmaceutical excipient.

The present disclosure also provides a use of substance **A** in the preparation of a G protein-coupled receptor agonist or partial agonist, wherein the substance **A** is the compound of formula I, the pharmaceutically acceptable salt thereof, the solvate thereof, or the solvate of the pharmaceutically acceptable salt thereof, or the pharmaceutical composition; the G protein-coupled receptor is a dopamine receptor and/or a 5-hydroxytryptamine receptor.

Preferably, the dopamine receptor can be a dopamine D₂ receptor.

Preferably, the 5-hydroxytryptamine receptor can be a 5-hydroxytryptamine 5-HT_{2A} receptor and/or a 5-hydroxytryptamine 5-HT_{1A} receptor.

In one embodiment, the G protein-coupled receptor agonist can be used *in vivo, in vitro,* and *ex vivo.*

The present disclosure also provides a use of substance **A** in the preparation of a medicament for treating and/or preventing a disease associated with a G protein-coupled receptor; the substance **A** is the compound of formula I, the pharmaceutically acceptable salt thereof, the solvate thereof, or the solvate of the pharmaceutically acceptable salt thereof, or the pharmaceutical composition; the G protein-coupled receptor is a dopamine receptor and/or a 5-hydroxytryptamine receptor.

Preferably, the dopamine receptor can be a dopamine D2 receptor.

Preferably, the 5-hydroxytryptamine receptor is a 5-hydroxytryptamine 5-HT_{2A} receptor and/or a 5-hydroxytryptamine 5-HT_{1A} receptor.

Preferably, the disease associated with a G protein-coupled receptor refers to one or more than one of a neurodegenerative disease, a mental disorder, and a metabolic disease related to a mental disorder, such as Parkinson's disease, Alzheimer's disease, dementia, schizophrenia, bipolar disorder, depression, attention deficit hyperactivity disorder (ADHD), restless legs syndrome, Huntington's disease, erectile dysfunction, prolactinoma, or drug addiction.

The present disclosure also provides a use of substance **A** in the preparation of a medicament for treating and/or preventing disease **M;** the substance **A** is the compound of formula I, the pharmaceutically acceptable salt thereof, the solvate thereof, or the solvate of the pharmaceutically acceptable salt thereof, or the pharmaceutical composition; the disease **M** refers to one or more than one of a neurodegenerative disease, a mental disorder, and a metabolic disease related to a mental disorder.

In the use, the disease **M** is preferably Parkinson's disease, Alzheimer's disease, dementia, schizophrenia, bipolar disorder, depression, attention deficit hyperactivity disorder, restless legs syndrome, Huntington's disease, erectile dysfunction, prolactinoma, or drug addiction.

Unless otherwise specified, terms used in the present disclosure have the following meanings:
As used herein, a substituent employed may be preceded by a single dash "-" to indicate that the named substituent is connected to the parent moiety by a single bond.

In the present disclosure, when the connection direction of the listed linking groups is not specified, their connection direction is assumed to follow the same direction as the reading order from left to right. For example, the linking group L₁ in 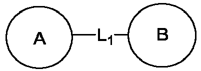 is -C-D-, then -C-D- connects ring A and ring B in the same direction as the reading order from left to right to form 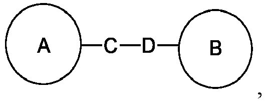 but not 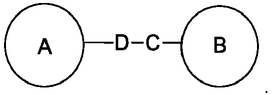 Specifically, in the present disclosure, L being "-C₁₋₆ alkylene-C₃₋₆ cycloalkylene-" means that C₁₋₆ alkylene is connected to N in the parent moiety by a single bond, rather than C₃₋₆ cycloalkylene being connected to N in the parent moiety.

The terms "compound" and "pharmaceutically acceptable salt", if tautomers exist, may exist as a single tautomer or as a mixture of tautomers, preferably in the form in which a relatively stable tautomer is dominant.

If a linking group is indicated as "absent," then the structures on both sides of the linking group are directly connected by a single bond. For example, in the structure -A-B-C-, if B is absent, then -A-B-C- is -A-C-.

The term "halogen" refers to fluorine, chlorine, bromine, or iodine.

The term "alkyl" refers to a straight or branched alkyl group with a specified number of carbon atoms (*e.g*., C₁ to C₁₀). Alkyl includes, but is not limited to, methyl, ethyl, *n*-propyl, isopropyl, *n*-butyl, *tert*-butyl, isobutyl, *sec*-butyl, *n*-pentyl, *n*-hexyl, *n*-heptyl, *n*-octyl, *etc.*

The term "alkylene" refers to a divalent group of a straight or branched saturated aliphatic hydrocarbon group with a specified number of carbon atoms. Two valences can be on the same atom, such as methylene (-CH₂-), ethylene 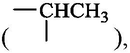 and two valences can also be connected to two atoms respectively, such as 1,2-ethylene (-CH₂CH₂-).

The term "alkenylene" refers to a divalent group of a straight or branched aliphatic hydrocarbon group containing one or more double bonds with a specified number of carbon atoms (*e.g.*, C₂ to C₁₀). Two valences can be on the same atom, such as 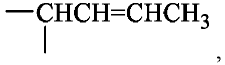 and two valences can also be connected to two atoms respectively, such as -CH₂CH=CHCH₂-.

The term "alkynylene" refers to a divalent group of a straight or branched aliphatic hydrocarbon group containing one or more triple bonds with a specified number of carbon atoms (*e.g*., C₂ to C₁₀). Two valences can be on the same atom, such as 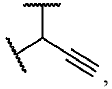 and two valences can also be connected to two atoms respectively, such as

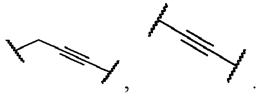

The term "cycloalkyl" refers to a straight or branched alkyl group with a specified number of carbon atoms (*e.g*., C₃ to C₆), which is a saturated monocyclic, bridged, or spirocyclic ring group consisting solely of carbon atoms. Monocyclic cycloalkyl includes, but is not limited to, cyclopropyl, cyclobutyl, cyclopentyl, cyclohexyl, *etc.*

The term "cycloalkylene" refers to a divalent group of saturated cyclic alkylene, for example, cyclopentylene (*e.g*., 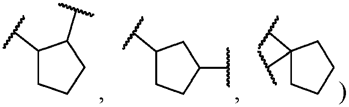 or cyclohexylene.

The term "heterocycloalkyl" refers to a cyclic group with a specified number of ring atoms *(e.g.,* 5- to 10-membered), a specified number of heteroatoms (*e.g*., 1, 2, or 3), and a specified type of heteroatoms (one or more than one of N, O, and S). It can be a monocyclic, bridged, or spiro ring, and each ring is saturated. Heterocycloalkyl includes, but is not limited to, azetidinyl, tetrahydropyrrolyl, tetrahydrofuranyl, morpholinyl, piperidinyl, *etc.*

The term "aryl" refers to a cyclic group consisting solely of carbon atoms with a specified number of carbon atoms (*e.g*., C₆ to C₁₀). It can be a monocyclic or polycyclic ring, and at least one ring is aromatic (conforming to Huckel's rule). Aryl is connected to other moieties in the molecule through an aromatic ring or a non-aromatic ring. Aryl includes, but is not limited to, phenyl, naphthyl, *etc.*

The term "heteroaryl" refers to a cyclic group with a specified number of ring atoms (*e.g.,* 5- to 10-membered), a specified number of heteroatoms (*e.g*., 1, 2, or 3), and a specified type of heteroatoms (one or more than one of N, O, and S). It can be a monocyclic or polycyclic ring, and at least one ring is aromatic (conforming to Huckel's rule). Heteroaryl is connected to other moieties in the molecule through an aromatic ring or a non-aromatic ring. Heteroaryl includes, but is not limited to, furyl, pyrrolyl, thienyl, pyrazolyl, imidazolyl, oxazolyl, thiazolyl, pyridyl, pyrimidinyl, indolyl, *etc.*

"-" at the end of a group indicates that the group is connected to other moieties in the molecule through this site. For example, CH₃-C(=O)- refers to acetyl. 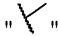 in a structural moiety indicates that the structural moiety is connected to other moieties in the molecule through this site. For example, 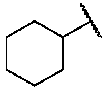 refers to cyclohexyl.

The term "more than one" refers to 2, 3, 4, or 5.

When any variable (*e.g*., the group R¹⁻¹) appears multiple times in the definition of a compound, their definitions are independent of each other and do not interfere with each other. For example, C₆-C₁₀ aryl substituted by 3 R¹⁻¹ means that the C₆-C₁₀ aryl will be substituted by 3 R¹⁻¹, and the definitions of the 3 R¹⁻¹ are independent of each other and do not interfere with each other.

The term "pharmaceutically acceptable salt" refers to a salt obtained by reacting a compound with a pharmaceutically acceptable (relatively non-toxic, safe, and suitable for patient use) acid or base. When a compound contains relatively acidic functional groups, base addition salts can be obtained by contacting the free form of the compound with a sufficient amount of a pharmaceutically acceptable base in a suitable inert solvent. Pharmaceutically acceptable base addition salts include, but are not limited to, sodium salts, potassium salts, calcium salts, aluminum salts, magnesium salts, bismuth salts, ammonium salts, *etc.* When a compound contains relatively basic functional groups, acid addition salts can be obtained by contacting the free form of the compound with a sufficient amount of a pharmaceutically acceptable acid in a suitable inert solvent. Pharmaceutically acceptable acid addition salts include, but are not limited to, hydrochlorides, sulfates, methanesulfonates, *etc.* For details, see Handbook of Pharmaceutical Salts: Properties, Selection, and Use (P. Heinrich Stahl, 2002). The term "solvate" refers to a substance formed by crystallization of a compound with a solvent (including but not limited to water, methanol, ethanol, *etc*.). Solvates are divided into stoichiometric solvates and non-stoichiometric solvates.

The term "solvate of the pharmaceutically acceptable salt" refers to a substance formed by the combination of a compound with a pharmaceutically acceptable (relatively non-toxic, safe, and suitable for patient use) acid or base, and a solvent (including but not limited to water, methanol, ethanol, *etc.*), wherein the pharmaceutically acceptable salt has the same meaning as the term "pharmaceutically acceptable salt" described above, and the solvent is either stoichiometric or non-stoichiometric. The solvate of the pharmaceutically acceptable salt includes, but is not limited to, hydrochloride monohydrate.

The term "pharmaceutical excipient" refers to formulating agents and additives used in the manufacture of drugs and compounding of prescriptions. They are all substances contained in a pharmaceutical formulation except for active ingredients. For details, see the Pharmacopoeia of the People's Republic of China (2020 Edition) or Handbook of Pharmaceutical EMcipients (Raymond C Rowe, 2009).

The term "treat/treatment" refers to any one of the following: (1) alleviating one or more biological manifestations of a disease; (2) interfering with one or more points in the biological cascade that causes a disease; (3) slowing the progression of one or more biological manifestations of a disease.

The term "prevent/prevention" refers to reducing the risk of developing a disease.

The term "patient" refers to any animal, preferably a mammal, most preferably a human, who has already received or is about to receive treatment. Mammals include, but are not limited to, cattle, horses, sheep, pigs, cats, dogs, mice, rats, rabbits, guinea pigs, monkeys, humans, *etc.*

Without departing from common knowledge in the art, the above various preferred conditions can be arbitrarily combined to obtain the various preferred examples of the present disclosure.

The reagents and raw materials used in the present disclosure are commercially available.

The positive effects of the present disclosure include: The compounds of the present disclosure exhibit good affinity and agonistic activity for at least one of the dopamine receptors and 5-hydroxytryptamine receptors. Some compounds of the present disclosure exhibit good affinity and agonistic activity for two of the following receptors: dopamine D₂ receptor, 5-hydroxytryptamine 5-HT_{2A} receptor, and 5-hydroxytryptamine 5-HT_{1A} receptor.

### DETAILED DESCRIPTION OF THE PREFERRED EMBODIMENT

The present disclosure is further described below by examples, but is not limited to the scope of the described examples. In the following examples, experimental methods without specified conditions are selected according to conventional methods and conditions, or according to the product instructions.

### Example 1: Synthesis of 6,6a,7,8,9,10-hexahydropyrazino[1,2-a]thieno[4,3,2-de]quinoline (compound I-1):

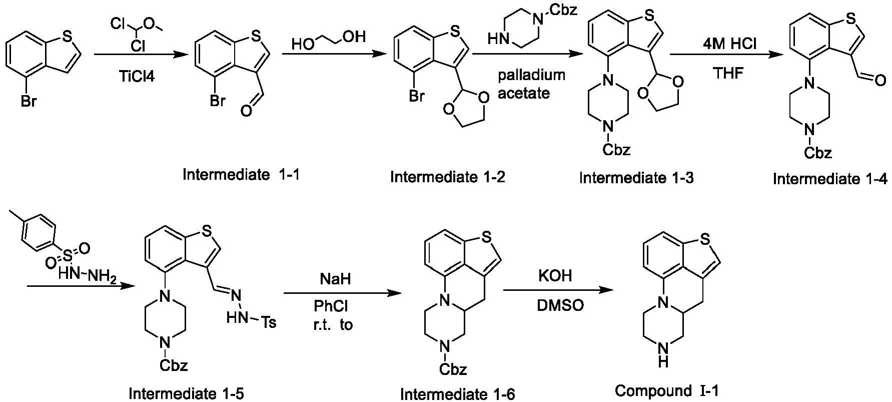

Step 1: 4-Bromobenzo[b]thiophene (500 mg, 2.35 mmol) was dissolved in dry dichloromethane (100 mL). 1,1-Dichlorodimethyl ether (162 mg, 1.41 mmol) was then added thereto, followed by the addition of titanium tetrachloride (670 mg, 3.54 mmol). The reaction mixture was stirred at room temperature in a water bath for 4 hours. Water was added to quench the reaction. Sodium hydroxide pellets were slowly added thereto in batches, monitoring the pH to 9 to 10 with test paper, and the reaction mixture was stirred for another 30 minutes. The mixture was filtered through diatomite, and the resulting filtrate was directly separated into different phases. The aqueous phase was extracted twice with DCM. The organic phase was concentrated, and the residue was purified by flash column chromatography (10 to 15% ethyl acetate/petroleum ether) to obtain **intermediate 1-1** (260 mg, yield of 46%) as a yellow solid. ¹H NMR (800 MHz, DMSO-d₆) δ 10.90 (s, 1H), 8.80 (s, 1H), 8.22 (d, *J* = 8.0 Hz, 1H), 7.83 (d, *J* = 8.5 Hz, 1H), 7.41 (t, *J* = 7.9 Hz, 1H). ¹³C NMR (201 MHz, DMSO) δ 185.87, 142.86, 139.59, 136.06, 134.43, 130.66, 126.27, 123.23, 114.98.

Step 2: M-1-057 (200 mg, 0.83 mmol) was dissolved in toluene (6 mL). Ethylene glycol (515 mg, 8.3 mmol) and *p*-toluenesulfonic acid (40 mg, 0.23 mmol) were sequentially added thereto. The reaction mixture was refluxed for 5 hours. The reaction mixture was diluted with water, extracted with ethyl acetate, washed with saturated brine, and concentrated. The residue was purified by flash column chromatography (5 to 10% ethyl acetate/petroleum ether) to obtain **intermediate 1-2** (180 mg, yield of 76%) as a yellow oil. ¹HNMR (800 MHz, DMSO) δ 8.09 - 8.05 (m, 2H), 7.67 (d, *J* = 7.7 Hz, 1H), 7.29 (t, *J* = 7.8 Hz, 1H), 6.67 (s, 1H), 4.07 - 4.03 (m, 2H), 4.01 - 3.97 (m, 2H). ¹³C NMR (201 MHz, DMSO) δ 142.91, 134.70, 133.85, 129.80, 128.38, 125.37, 122.71, 115.03, 98.34, 64.39. HRMS (ESI) C₁₁H₁₀BrO₂S⁺ [M+H]⁺ calculated: 284.9579, found: 284.9575.

Step 3: M-1-059 (700 mg, 2.45 mmol) was dissolved in anhydrous toluene (12 mL). 1-(Benzyloxycarbonyl)piperazine (1.08 g, 4.91 mmol), sodium *tert*-butoxide (354 mg, 3.68 mmol), BINAP (2,2'-bis(diphenylphosphino)-1,1'-binaphthyl) (230 mg, 0.37 mmol), and palladium acetate (30 mg, 0.13 mmol) were sequentially added thereto. The reaction mixture was evacuated and replaced with argon three times, and then refluxed at 110°C for 4 hours. The reaction mixture was diluted with water, extracted with ethyl acetate, washed with saturated brine, and concentrated. The residue was purified by flash column chromatography (10 to 15% ethyl acetate/petroleum ether) to obtain **intermediate 1-3** (530 mg, yield of 51%) as a yellow oil. ¹H NMR (800 MHz, DMSO) δ 7.87 (s, 1H), 7.78 (d, *J* = 8.9 Hz, 1H), 7.42 - 7.38 (m, 4H), 7.35 - 7.32 (m, 2H), 7.25 (d, *J* = 8.7 Hz, 1H), 6.77 (s, 1H), 5.12 (s, 2H), 4.10 - 4.07 (m, 2H), 4.04 (d, *J* = 12.5 Hz, 2H), 4.01 - 3.98 (m, 2H), 3.23 - 3.04 (m, 4H), 2.78 - 2.73 (m, 2H). ¹³C NMR (201 MHz, DMSO) δ 154.50, 149.38, 141.76, 136.86, 134.51, 132.96, 128.38(2), 127.81, 127.59(2), 126.04, 125.00, 119.88, 117.42, 98.70, 66.26, 64.49(2), 52.96(2), 43.80(2). HRMS (ESI) C₂₃H₂₅N₂O₄S⁺ [M+H]⁺ calculated: 425.1530, found: 425.1535.

Step 4: M-1-061 (700 mg, 1.65 mmol) was dissolved in dry THF (5 mL), and 4 M HCl (6 mL) was added thereto at room temperature. The reaction mixture was reacted at room temperature for 2 hours. Sodium hydroxide pellets were slowly added thereto in batches, monitoring the pH to approximately 10 with test paper. The reaction mixture was extracted with ethyl acetate, concentrated, and the residue was purified by flash column chromatography (15% ethyl acetate/petroleum ether) to obtain **intermediate 1-4** (510 mg, yield of 81%) as an orange-yellow oil. ¹H NMR (800 MHz, DMSO) δ 10.77 (s, 1H), 8.60 (s, 1H), 7.85 (d, *J* = 8.8 Hz, 1H), 7.42 (t, *J* = 7.9 Hz, 1H), 7.40 - 7.37 (m, 4H), 7.34 - 7.31 (m, 1H), 7.30 (d, *J* = 7.7 Hz, 1H), 5.12 (s, 2H), 4.06 (d, *J* = 9.5 Hz, 2H), 3.17 (s, 4H), 2.79 (s, 2H). ¹³C NMR (201 MHz, DMSO) δ 187.77, 154.48, 149.16, 141.95, 136.83, 136.38, 135.59, 131.08, 128.43(2), 127.86, 127.58(2), 125.98, 119.52, 116.77, 66.33, 52.31(2), 43.46(2). HRMS (ESI) C₂₁H₂₁N₂O₃S⁺ [M+H]⁺ calculated: 381.1267, found: 381.1264.

Step 5: M-1-062 (350 mg, 0.92 mmol) was dissolved in toluene (8 mL), and *p-*toluenesulfonyl hydrazide (188 mg, 1.01 mmol) was added thereto. The reaction mixture was sonicated and reacted for 1.5 hours. The reaction mixture was directly filtered, and the filter cake was rinsed with petroleum ether and dried under reduced pressure to obtain **intermediate 1-5** (350 mg, yield of 69%) as a white solid. ¹H NMR (800 MHz, DMSO) δ 11.55 (s, 1H), 8.86 (s, 1H), 7.94 (s, 1H), 7.75 - 7.73 (m, 2H), 7.72 (d, *J* = 2.0 Hz, 1H), 7.43 - 7.38 (m, 4H), 7.38 - 7.34 (m, 1H), 7.34 - 7.30 (m, 3H), 7.15 - 7.12 (m, 1H), 5.24 - 5.04 (m, 2H), 3.92 - 3.70 (m, 2H), 3.16 (s, 2H), 2.99 - 2.82 (m, 2H), 2.72 - 2.56 (m, 2H), 2.31 (s, 3H). ¹³C NMR (201 MHz, DMSO) δ 154.71, 149.09, 143.91, 143.35, 141.56, 136.85, 136.22, 131.22, 130.41, 129.65, 128.89, 128.50(2), 128.19, 127.99, 127.81(2), 127.00, 125.94, 125.47, 119.36, 115.83, 66.37, 52.83, 52.22, 42.92(2), 20.94. HRMS (ESI) C₂₈H₂₉N₄O₄S₂⁺ [M+H]⁺ calculated: 549.1625, found: 549.1629.

Step 6: M-1-068 (1 g, 1.82 mmol) was dissolved in dry chlorobenzene (30 mL), and sodium hydride (68 mg, 2 mmol) was added thereto in an ice bath. The reaction mixture was reacted at room temperature for 1 hour and then refluxed for 2 hours. Water was added to quench the reaction. The reaction mixture was extracted three times with dichloromethane, washed with saturated brine, concentrated, and the residue was purified by flash column chromatography (5% ethyl acetate/petroleum ether) to obtain **intermediate 1-6** (148 mg, yield of 24%) as a white solid. ¹H NMR (800 MHz, DMSO) δ 7.42 - 7.35 (m, 4H), 7.34 - 7.28 (m, 2H), 7.21 (t, *J* = 7.9 Hz, 1H), 7.10 (s, 1H), 6.69 (d, *J* = 8.0 Hz, 1H), 5.12 (s, 2H), 4.20 - 4.07 (m, 2H), 3.86 (d, *J* = 9.6 Hz, 1H), 3.21 (d, *J* = 15.6 Hz, 2H), 2.99 - 2.79 (m, 2H), 2.71 - 2.58 (m, 2H). ¹³C NMR (201 MHz, DMSO) δ 154.25, 144.44, 138.85, 136.81, 128.47(2), 128.43, 127.91, 127.62(2), 127.29, 126.26, 116.15, 112.60, 105.03, 67.04, 66.42, 54.50, 48.47, 45.60, 29.44. HRMS (ESI) C₂₁H₂₁N₂O₂S⁺ [M+H]⁺ calculated: 365.1318, found: 365.1316.

Step 7: M-1-069 (100 mg, 0.27 mmol) was dissolved in dimethyl sulfoxide (5 mL). 6 M KOH (1.5 mL) was added thereto with stirring, and the reaction mixture was reacted at 90°C for 1 hour. The reaction mixture was diluted with water, extracted with dichloromethane, washed with saturated brine, and concentrated. The residue was purified by flash column chromatography (0 to 5% methanol/dichloromethane) to obtain **compound I-1** as an off-white crystal. ¹H NMR (800 MHz, CDCl₃) δ 7.30 (d, *J* = 8.0 Hz, 1H), 7.26 (t, *J* = 7.8 Hz, 1H), 6.84 (d, *J* = 1.9 Hz, 1H), 6.64 (d, *J* = 7.7 Hz, 1H), 3.81 (dt, *J* = 11.7, 2.5 Hz, 1H), 3.27 - 3.23 (m, 2H), 3.10 - 3.06 (m, 2H), 3.04 (dd, *J* = 15.6, 3.4 Hz, 1H), 2.82 - 2.78 (m, 3H). HRMS (ESI) C₁₃H₁₅N₂S⁺ [M+H]⁺ calculated: 231.0950, found: 231.0949.

### Example 2: Synthesis of 8-propyl-6,6a,7,8,9,10-hexahydropyrazino[1,2-a]thieno[4,3,2-de]quinoline (compound I-2):

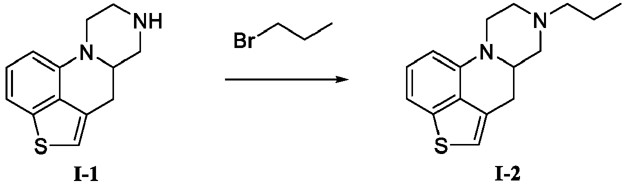

6,6a,7,8,9,10-Hexahydropyrazino[1,2-*a*]thieno[4,3,2-*de*]quinoline (compound **I-1**) (46 mg, 0.2 mmol), 1-bromopropane (0.5 mL, 2 mmol), and diisopropylethylamine (0.08 mL, 0.4 mmol) were placed in a reaction flask and dissolved in dimethyl sulfoxide (5 mL). The reaction mixture was heated to 60°C and stirred for 4 hours. The reaction mixture was added with water and extracted with ethyl acetate. The organic phase was dried over anhydrous sodium sulfate, concentrated, and purified by column chromatography (dichloromethane/methanol = 95:5). **I-2** (38 mg, yield of 76%) was obtained as a brown solid. ¹H NMR (800 MHz, CDCl₃) δ 7.26 (t, *J* = 7.8 Hz, 1H), 7.22 (t, *J* = 7.8 Hz, 1H), 6.79 (s, 1H), 6.60 (d, *J* = 7.7 Hz, 1H), 3.76 (m, 1H), 3.23 - 3.16 (m, 1H), 3.10 - 3.03 (m, 2H), 3.00 (dd, *J* = 15.6, 3.4 Hz, 1H), 2.90 (td, *J* = 11.8, 3.2 Hz, 1H), 2.78 (m, 1H), 2.39 - 2.34 (m, 2H), 2.29 (td, *J* = 11.7, 3.1 Hz, 1H), 2.03 (t, *J* = 10.7 Hz, 1H), 1.60 - 1.53 (m, 2H), 0.94 (t, *J* = 7.4 Hz, 3H). ¹³C NMR (201 MHz, CDCl₃) δ 145.10, 139.68, 129.26, 127.95, 126.27, 115.40, 112.70, 104.46, 60.60, 59.97, 55.14, 52.87, 46.23, 31.12, 20.07, 12.06. HRMS (ESI, m/z): C₁₆H₂₁N₂S⁺ [M+H]⁺ calculated: 273.1420, found: 273.1521.

### Example 3: Synthesis of 2-(6a,7,9,10-tetrahydropyrazino[1,2-a]thieno[4,3,2-de]quinolin-8(6H)-yl)ethan-1-ol (compound I-3)

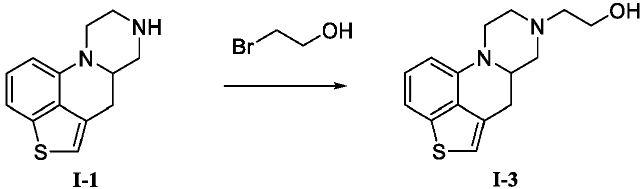

The synthesis was conducted in a manner similar to the synthesis of compound **I-2,** with 1-bromopropane replaced by 2-bromoethanol. Compound **I-3** (13 mg, yield of 48%) was obtained as a pale yellow solid. ¹H NMR (800 MHz, CDCl₃) δ 7.28 (d, *J* = 8.0 Hz, 1H), 7.24 (t, *J* = 7.8 Hz, 1H), 6.83 (s, 1H), 6.61 (d, *J* = 7.7 Hz, 1H), 3.80 (d, *J* = 11.7 Hz, 1H), 3.71 (t, *J* = 5.3 Hz, 2H), 3.23 (tt, *J* = 11.1, 3.1 Hz, 1H), 3.11 (m, 2H), 3.02 (dd, *J* = 15.6, 3.4 Hz, 1H), 2.93 (td, *J* = 11.9, 3.2 Hz, 1H), 2.80 (m, 1H), 2.66 (m, 2H), 2.50 (td, *J* = 11.7, 3.1 Hz, 1H), 2.24 (t, *J* = 10.8 Hz, 1H). ¹³C NMR (201 MHz, CDCl₃) δ 144.62, 139.68, 128.74, 127.82, 126.19, 115.65, 112.90, 104.50, 59.33, 59.22, 57.76, 54.98, 52.40, 46.03, 30.81. HRMS (ESI, m/z): C₁₅H₁₉N₂OS⁺ [M+H]⁺ calculated: 275.1213, found: 275.1324.

### Example 4: Synthesis of 8-(cyclopropylmethyl)-6,6a,7,8,9,10-hexahydropyrazino[1,2-a]thieno[4,3,2-de]quinoline (compound I-4)

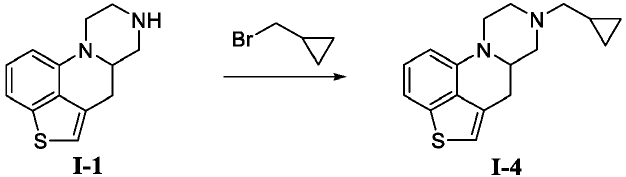

The synthesis was conducted in a manner similar to the synthesis of compound **1-2,** with 1-bromopropane replaced by (bromomethyl)cyclopropane. Compound **1-4** (23 mg, yield of 68%) was obtained as a pale yellow oil. ¹H NMR (800 MHz, CDCl₃) δ 7.26 (t, *J* = 9.3 Hz, 1H), 7.23 (t, *J* = 7.8 Hz, 1H), 6.80 (s, 1H), 6.61 (d, *J* = 7.6 Hz, 1H), 3.78 (d, *J* = 11.7 Hz, 1H), 3.24 (m, 3H), 3.03 (dd, *J* = 15.6, 3.3 Hz, 1H), 2.95 (td, *J =* 11.9, 2.9 Hz, 1H), 2.79 (m, 1H), 2.38 - 2.31 (m, 3H), 2.08 (t, *J* = 10.6 Hz, 1H), 0.96 - 0.90 (m, 1H), 0.60 - 0.55 (m, 2H), 0.16 (q, *J* = 4.9 Hz, 2H). ¹³C NMR (201 MHz, CDCl₃) δ 144.91, 139.62, 129.09, 127.85, 126.21, 115.38, 112.67, 104.42, 63.55, 59.65, 54.92, 52.67, 46.01, 31.03, 8.18, 4.04. HRMS (ESI, m/z): C₁₇H₂₁N₂S⁺ [M+H]⁺ calculated: 285.1420, found: 285.1551.

### Example 5: Synthesis of 8-(4-fluorophenylethyl)-6,6a,7,8,9,10-hexahydropyrazino[1,2-a]thieno[4,3,2-de]quinoline (compound I-5)

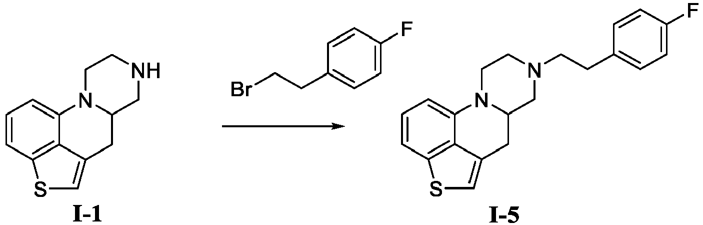

The synthesis was conducted in a manner similar to the synthesis of compound **1-2,** with 1-bromopropane replaced by 1-fluoro-4-(2-bromoethyl)benzene. Compound **1-5** (13 mg, yield of 52%) was obtained as a pale yellow solid. ¹H NMR (800 MHz, CDCl₃) δ 7.27 (d, *J* = 7.9 Hz, 1H), 7.23 (d, *J* = 7.5 Hz, 1H), 7.18 - 7.13 (m, 2H), 6.97 (m, 2H), 6.80 (s, 1H), 6.60 (d, *J* = 7.7 Hz, 1H), 3.77 (m, 1H), 3.20 (tt, *J* = 11.4, 3.2 Hz, 1H), 3.15 - 3.06 (m, 2H), 2.99 (dd, *J =* 15.6, 3.4 Hz, 1H), 2.93 - 2.89 (m, 1H), 2.82 (t, *J* = 9.5 Hz, 2H), 2.80 - 2.75 (m, 1H), 2.63 (t, *J* = 9.3 Hz, 2H), 2.38 (td, *J* = 11.7, 3.2 Hz, 1H), 2.12 (t, *J* = 10.7 Hz, 1H). ¹³C NMR (201 MHz, CDCl₃) δ 171.01, 162.34, 162.10, 161.12, 160.89, 144.87, 139.66, 135.64, 133.54 (d, *J* = 3.2 Hz), 129.01, 127.88, 126.23, 112.79, 104.49, 64.86, 60.20, 59.66, 55.01, 52.67, 46.08, 32.62, 30.97. HRMS (ESI, m/z): C₂₁H₂₀FN₂S⁺ [M+H]⁺ calculated: 353.1482, found: 353.1504.

### Example 6: Synthesis of 8-(2-(thiophen-2-yl)ethyl)-6,6a,7,8,9,10-hexahydropyrazino[1,2-a]thieno[4,3,2-de]quinoline (compound I-6)

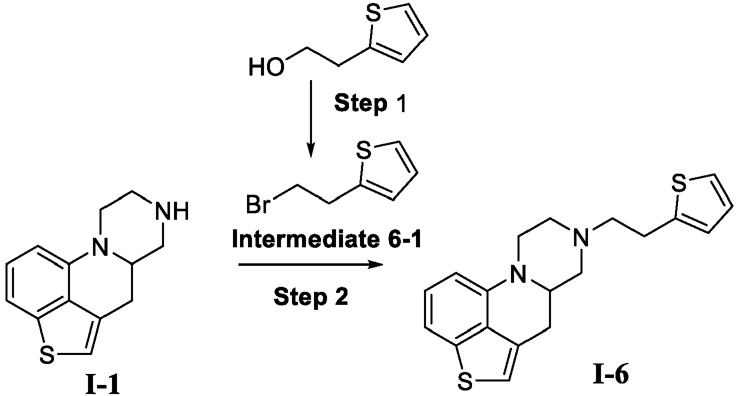

Step 1: 1-(2-Thienyl)ethanol (CAS#2309-47-9) (0.5 g, 3.9 mmol) was dissolved in dichloromethane (10 mL) in a round-bottom flask. CBr₄ (1.94 g, 5.85 mmol) was added thereto, and the round-bottom flask was placed in an ice-water bath. Then, PPh₃ (1.52 g, 5.85 mmol) was added thereto, and the reaction mixture was stirred at room temperature for 16 hours. After the reaction was completed, the solvent was removed, and the residue was purified by flash silica gel column chromatography (eluent: 0 to 10% methanol/dichloromethane) to obtain intermediate **6-1** (0.6 g, 80%) as a yellow oily compound. ¹H NMR (800 MHz, CDCl₃) δ 7.22 - 7.18 (m, 1H), 6.99 - 6.95 (m, 1H), 6.92 - 6.88 (m, 1H), 3.60 - 3.56 (m, 2H), 3.40 - 3.46 (m, 2H).

Step 2: The synthesis was conducted in a manner similar to the synthesis of compound **I-2,** with 1-bromopropane replaced by 2-(2-bromoethyl)thiophene. Compound **I-6** (18 mg, yield of 57%) was obtained as a pale yellow solid. ¹H NMR (800 MHz, CDCl₃) δ 7.28 (d, *J* = 7.9 Hz, 1H), 7.23 (m, 1H), 7.13 (dd, *J* = 5.1, 1.0 Hz, 1H), 6.93 (m, 1H), 6.86 (dd, *J* = 3.3, 0.7 Hz, 1H), 6.81 (d, *J* = 1.2 Hz, 1H), 6.62 (d, *J* = 7.6 Hz, 1H), 3.81 - 3.77 (m, 1H), 3.24 (m, 1H), 3.15 - 3.10 (m, 2H), 3.09 (m, 2H), 3.02 (dd, *J* = 15.6, 3.3 Hz, 1H), 2.94 (td, *J* = 11.8, 3.2 Hz, 1H), 2.80 (m, 1H), 2.76 - 2.71 (m, 2H), 2.43 (td, *J* = 11.7, 3.1 Hz, 1H), 2.17 (t, *J* = 10.7 Hz, 1H). ¹³C NMR (201 MHz, CDCl₃) δ 144.93, 142.42, 139.64, 129.06, 127.88, 126.65, 126.20, 124.79, 123.68, 115.46, 112.74, 104.46, 59.61, 59.57, 55.05, 52.57, 46.12, 30.98, 27.61. HRMS (ESI, m/z): C₁₉H₂₁N₂S₂⁺ [M+H]⁺ calculated: 341.1141, found: 341.1208.

### Example 7: Synthesis of 8-([1,1'-biphenyl]-3-ylmethyl)-6,6a,7,8,9,10-hexahydropyrazino[1,2-a]thieno[4,3,2-de]quinoline (compound I-7)

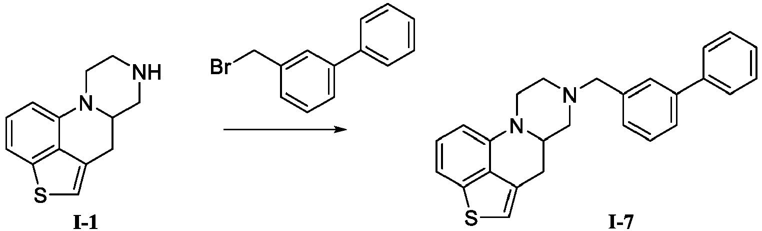

The synthesis was conducted in a manner similar to the synthesis of compound **I-2**, with 1-bromopropane replaced by 3-phenylbenzyl bromide (CAS# 14704-31-5). Compound **I-7** (26 mg, yield of 63%) was obtained as a pale yellow solid. ¹H NMR (800 MHz, CDCl₃) δ 7.63 - 7.58 (m, 3H), 7.51 (d, *J* = 7.7 Hz, 1H), 7.45 - 7.39 (m, 3H), 7.36 - 7.32 (m, 2H), 7.25 (d, *J* = 8.0 Hz, 1H), 7.22 - 7.20 (m, 1H), 6.75 (s, 1H), 6.57 (d, *J* = 7.7 Hz, 1H), 3.73 (d, *J* = 11.6 Hz, 1H), 3.63 (q, *J* = 13.0 Hz, 2H), 3.21 (t, *J* = 10.7 Hz, 1H), 3.05 (dd, *J* = 22.1, 11.0 Hz, 2H), 2.93 (dd, *J* = 15.6, 3.4 Hz, 2H), 2.74 (m, 1H), 2.38 (td, *J* = 11.6, 2.8 Hz, 1H), 2.10 (t, *J* = 10.7 Hz, 1H). ¹³C NMR(201 MHz, CDCl₃) δ 145.04, 141.43, 141.11, 139.69, 129.20, 128.94, 128.89, 128.24, 128.04, 127.95, 127.47, 127.28, 126.28, 115.44, 112.74, 104.52, 62.91, 59.50, 55.12, 52.71, 46.18, 30.99. HRMS (ESI, m/z): C₂₆H₂₅N₂S⁺ [M+H]⁺ calculated: 397.1733, found: 397.1759.

### Example 8: Synthesis of 8-((5-(4-fluorophenyl)pyridin-3-yl)methyl)-6,6a,7,8,9,10-hexahydropyrazino[1,2-a]thieno[4,3,2-de]quinoline (compound I-8)

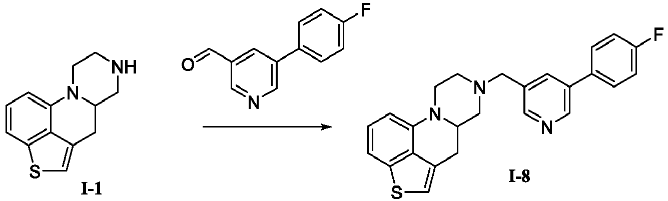

6,6a,7,8,9,10-Hexahydropyrazino[1,2-*a*]thieno[4,3,2-*de*]quinoline (compound **I-1**) (23 mg, 0.1 mmol), sodium cyanoborohydride (13 mg, 0.2 mmol), and 5-(4-fluorophenyl)-3-pyridinecarboxaldehyde (24 mg, 0.12 mmol) were placed in a reaction flask. Methanol was added thereto, then the reaction mixture was stirred at room temperature until dissolved, and acetic acid (0.02 mL) was added dropwise thereto. The reaction mixture was stirred and reacted at room temperature, and the reaction was completed after 6 hours. Water was added to quench the reaction. The reaction mixture was extracted with ethyl acetate and washed with saturated sodium chloride. The organic phase was dried over anhydrous sodium sulfate, concentrated, and purified by column chromatography (dichloromethane/methanol = 95:5). Compound **I-8** (11 mg, yield of 28%) was obtained as a pale yellow solid. ¹H NMR (800 MHz, CDCl₃) δ 8.74 (s, 1H), 8.56 (s, 1H), 7.89 (s, 1H), 7.57 (dd, *J* = 8.6, 5.3 Hz, 2H), 7.27 (d, *J* = 8.0 Hz, 1H), 7.23 (t, *J* = 7.9 Hz, 1H), 7.17 (t, *J* = 8.6 Hz, 2H), 6.79 (s, 1H), 6.60 (d, *J* = 7.7 Hz, 1H), 3.78 (d, *J* = 11.6 Hz, 1H), 3.66 (q, *J* = 13.3 Hz, 2H), 3.22 (t, *J* = 10.8 Hz, 1H), 3.03 (dd, *J* = 29.8, 11.0 Hz, 2H), 2.97 (dd, *J* = 15.6, 3.3 Hz, 1H), 2.95 - 2.89 (m, 1H), 2.79 (ddd, *J* = 15.3, 11.6, 1.5 Hz, 1H), 2.45 (td, *J* = 11.6, 2.8 Hz, 1H), 2.17 (t, *J* = 10.7 Hz, 1H). ¹³C NMR (201 MHz, CDCl₃) δ 163.60, 162.37, 149.07, 147.16, 144.84, 139.64, 135.60, 134.95, 128.95, 128.88 (d, *J =* 8.2 Hz),127.85, 126.18, 116.17, 116.06, 115.48, 112.78, 104.45, 59.95, 59.49, 55.09, 52.72, 46.13, 30.92. HRMS (ESI, m/z): C₂₅H₂₃FN₃ S⁺ [M+H]⁺ calculated: 416.1591, found: 416.1722.

### Example 9: Synthesis of 1-(3-((6a,7,9,10-tetrahydropyrazino[1,2-a]thieno[4,3,2-de]quinolin-8(6H)-yl)methyl)phenyl)imidazolidin-2-one (compound I-9)

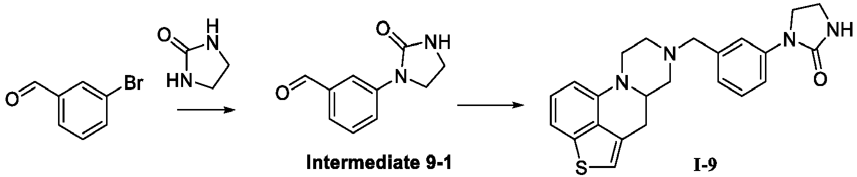

Step 1: Synthesis of intermediate **9-1.** m-Bromobenzaldehyde (360 mg, 2 mmol), 2-imidazolidinone (344 mg, 4 mmol), bis(dibenzylideneacetone)palladium (183 mg, 0.2 mmol), cesium carbonate (1.8 g, 6 mmol), and 4,5-bis(diphenylphosphino)-9,9-dimethylxanthene (125 mg, 0.2 mmol) were placed in a reaction flask. Dioxane was added thereto as the solvent, and the atmosphere in the flask was replaced with argon three times. The reaction mixture was then heated to 110°C and refluxed overnight. After the reaction was completed, the reaction mixture was extracted with ethyl acetate and washed with saturated sodium chloride. The organic phase was dried over anhydrous sodium sulfate, concentrated, and purified by column chromatography (petroleum ether/ethyl acetate = 5:1). A yellow solid (92 mg, yield of 30%) was obtained. HRMS (ESI): C₁₀H11N₂O₂⁺ [M+H]⁺ calculated: 191.0815, found: 191.0917.

Step 2: Synthesis of compound **I-9.** The synthesis was conducted in a manner similar to the synthesis of compound **I-8,** with 5-(4-fluorophenyl)-3-pyridinecarboxaldehyde replaced by 3-(2-oxoimidazolidin-1-yl)benzaldehyde (intermediate **9-1**). Compound I-9 (24 mg, yield of 41%) was obtained as a pale yellow solid. ¹H NMR (800 MHz, CDCl₃) δ 7.52 (s, 1H), 7.48 (d, *J* = 8.1 Hz, 1H), 7.30 (t, *J* = 7.9 Hz, 1H), 7.25 (d, *J* = 8.3 Hz, 1H), 7.21 (t, *J* = 7.8 Hz, 1H), 7.05 (d, *J* = 7.5 Hz, 1H), 6.77 (s, 1H), 6.58 (d, *J* = 7.7 Hz, 1H), 5.74 (s, 1H), 3.93 - 3.88 (m, 2H), 3.73 (d, *J* = 11.6 Hz, 1H), 3.61 (dd, *J* = 28.9, 12.9 Hz, 2H), 3.56 - 3.50 (m, 2H), 3.21 (m, 1H), 3.06 (dd, *J* = 23.3, 11.0 Hz, 2H), 2.93 (m, 2H), 2.78 - 2.71 (m, 1H), 2.44 - 2.36 (m, 1H), 2.11 (t,*J* = 10.7 Hz, 1H). ¹³CNMR (201 MHz, CDCl₃) δ 160.29, 144.93, 140.12, 139.60, 129.09, 128.86, 127.87, 126.18, 123.79, 118.74, 117.19, 115.41, 112.68, 104.47, 77.26, 77.10, 76.94, 62.63, 59.11, 54.88, 52.43, 45.92, 45.40, 37.56, 30.91, 21.30. HRMS (ESI, m/z): C₂₃H₂₅N₄OS⁺ [M+H]⁺ calculated: 405.1744, found: 405.4817.

### Example 10: Synthesis of 1-(4-fluorophenyl)-2-(6a,7,9,10-tetrahydropyrazino[1,2-a]thieno[4,3,2-de]quinolin-8(6H)-yl)ethan-1-one (compound 1-10)

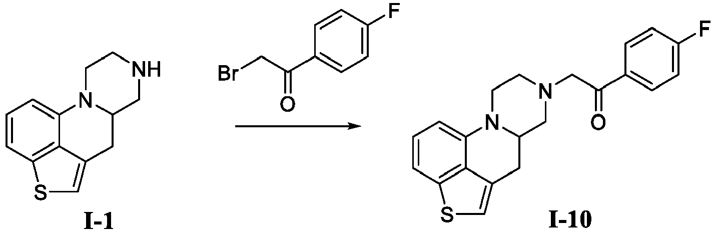

The synthesis was conducted in a manner similar to the synthesis of compound **I-2,** with 1-bromopropane replaced by 2-bromo-4'-fluoroacetophenone (CAS#403-29-2). Compound **I-10** (24 mg, yield of 61%) was obtained as a brown solid. ¹H NMR (800 MHz, CDCl₃) δ 8.10 - 8.05 (m, 2H), 7.27 (d, *J* = 8.0 Hz, 1H), 7.23 (t, *J* = 7.8 Hz, 1H), 7.13 (t, *J* = 8.6 Hz, 2H), 6.80 (s, 1H), 6.61 (d, *J* = 7.7 Hz, 1H), 3.84 (q, *J* = 16.3 Hz, 2H), 3.78 (d, *J* = 11.7 Hz, 1H), 3.33 - 3.26 (m, 1H), 3.15 (dd, *J* = 13.2, 5.3 Hz, 2H), 3.03 - 2.97 (m, 2H), 2.78 (ddd, *J* = 15.4, 11.6, 1.7 Hz, 1H), 2.55 (td, *J* = 11.6, 2.9 Hz, 1H), 2.27 (t, *J* = 10.7 Hz, 1H). ¹³C NMR (201 MHz, CDCl₃) δ 194.69, 166.55, 165.28, 144.78, 139.64, 132.32, 130.97 (d, *J* = 9.2 Hz), 128.87, 127.84, 126.19, 115.81, 115.70, 115.52, 112.81, 104.47, 64.34, 59.76, 54.92, 53.04, 46.02, 30.83. HRMS (ESI, m/z): C₂₁H₂₀FN₂S⁺ [M+H]⁺ calculated: 367.1275, found: 367.1312.

### Example 11: Synthesis of 1-(4-fluorophenyl)-4-(6a,7,9,10-tetrahydropyrazino[1,2-a]thieno[4,3,2-de]quinolin-8(6H)-yl)butan-1-one (compound I-11)

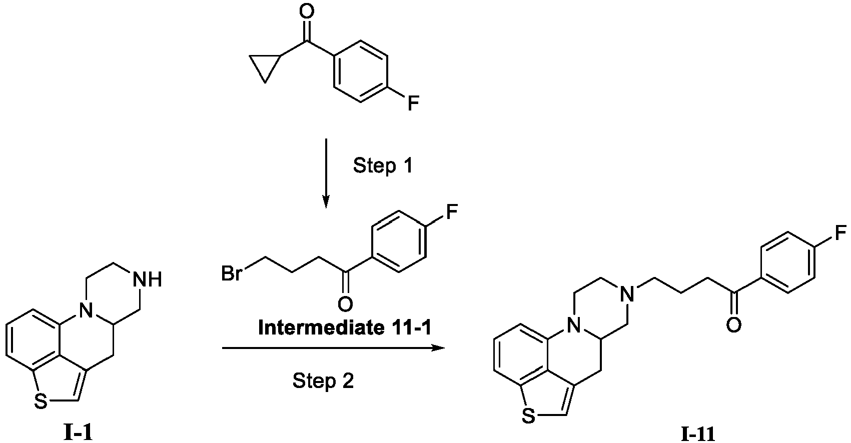

Step 1: Cyclopropyl(4-fluorophenyl)methanone (CAS#772-31-6) (0.50 g, 3.05 mmol) was added to a round-bottom flask, and then hydrobromic acid aqueous solution (3 mL) was added thereto. The reaction mixture was heated at 80°C for 2 hours, then cooled, and extracted with dichloromethane. The organic phases were combined and concentrated to obtain intermediate **11-1** (0.73 g, 98%) as a yellow oil. ¹H NMR (800 MHz, CDCl₃) δ 8.04 - 7.99 (m, 2H), 7.17 - 7.12 (m, 2H), 3.55 (t, *J* = 6.3 Hz, 2H), 3.16 (t, *J* = 6.9 Hz, 2H), 2.31 (p, *J* = 6.6 Hz, 2H).

Step 2: **I-1** (40 mg, 0.174 mmol), intermediate **11-1** (64 mg, 0.26 mmol), and DIPEA (diisopropylethylamine) (1 mL) were sequentially added to a round-bottom flask, and then the solvent DMSO (3 mL) was added thereto. The reaction mixture was heated and stirred at 60°C for 17 hours. After the reaction was completed, the reaction mixture was added with water and extracted three times with ethyl acetate. The organic phases were combined and concentrated to remove the solvent. The residue was purified by flash silica gel column chromatography (eluent: 0 to 10% methanol/dichloromethane) to obtain a compound, which was further purified by preparative liquid chromatography (mobile phase: 20 to 80% MeOH/H₂O, t_{R} = 21 min) to obtain compound **I-11** (35 mg, 51%) as a pale yellow solid. ¹H NMR (800 MHz, CD₃OD) δ 8.12 - 8.06 (m, 2H), 7.36 (d, *J* = 7.9 Hz, 1H), 7.28 (t, *J* = 7.9 Hz, 1H), 7.26 - 7.21 (m, 2H), 7.07 (d, *J* = 1.8 Hz, 1H), 6.79 (d, *J* = 7.8 Hz, 1H), 4.23 (d, *J* = 13.7 Hz, 1H), 3.86 - 3.81 (m, 2H), 3.43 - 3.40 (m, 1H), 3.35 - 3.32 (m, 3H), 3.27 - 3.22 (m, 3H), 3.13 - 3.07 (m, 2H), 2.89 - 2.86 (m, 1H), 2.25 - 2.19 (m, 2H). HR-MS (ESI, m/z): C₂₃H₂₄FN₂OS⁺ [M+H]⁺, calculated: 395.1588; found: 395.1594.

### Example 12: Synthesis of 4-fluoro-N-(2-(6a,7,9,10-tetrahydropyrazino[1,2-a]thieno[4,3,2-de]quinolin-8(6H)-yl)ethyl)benzamide (compound I-12)

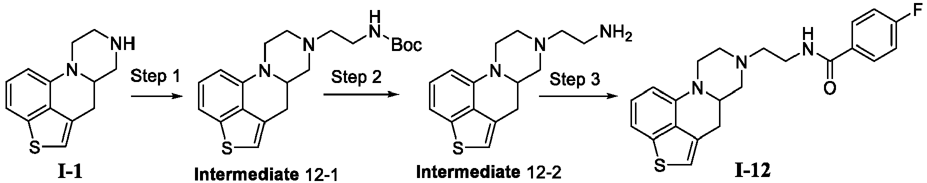

Step 1: Synthesis of **intermediate 12-1.** Compound **I-1** (46 mg, 0.2 mmol), *N-tert-*butoxycarbonyl-bromoethanamine (67 mg, 0.3 mmol), and diisopropylethylamine (0.1 mL, 7.5 mmol) were placed in a reaction flask, then dimethyl sulfoxide was added thereto, and the reaction mixture was stirred at room temperature until dissolved. The reaction mixture was then heated to 60°C, and the reaction was continued with stirring. After 4 hours, the reaction was completed, and the reaction mixture was extracted with ethyl acetate. The organic phase was dried over anhydrous sodium sulfate, concentrated, and purified by column chromatography (petroleum ether/ethyl acetate = 5 :1). A pale yellow oil (97 mg, yield of 69%) was obtained. HRMS (ESI): C₂₀H₂₈N₃O₂S⁺ [M+H]⁺ calculated: 374.1897, found: 374.1980.

Step 2: Synthesis of **intermediate 12-2. Intermediate 12-1** obtained from step 1 was dissolved in dichloromethane, and trifluoroacetic acid (0.2 mL) was added dropwise thereto with stirring at room temperature. After the reaction mixture was stirred and reacted at room temperature for 2 hours, the reaction was completed. The reaction mixture was extracted with ethyl acetate and washed with saturated sodium bicarbonate. The organic phase was dried over anhydrous sodium sulfate and directly used in the next reaction step. A pale yellow solid was obtained. HRMS (ESI): C₁₅H₂₀N₃S' [M+H]⁺ calculated: 274.1372, found: 274.1410.

Step 3: Synthesis of compound **I-12. Intermediate 12-2** (40 mg, 0.15 mmol), *p-*fluorobenzoic acid (25.2 mg, 0.18 mmol), 2-(7-azabenzotriazol-1-yl)-*N,N,N',N'-*tetramethyluronium hexafluorophosphate (HATU) (87 mg, 0.23 mmol), and diisopropylethylamine (0.05 mL, 0.3 mmol) were placed in a reaction flask. Dry *N,N-*dimethylformamide was added thereto, then the reaction mixture was stirred at room temperature until dissolved, and the reaction was continued with stirring. After 5 hours, the reaction was completed. The reaction mixture was extracted with ethyl acetate and washed with saturated sodium chloride. The organic phase was dried over anhydrous sodium sulfate, concentrated, and purified by column chromatography (dichloromethane/methanol = 95:5). Compound **I-12** (36 mg, yield of 67%) was obtained as a brown solid. ¹H NMR (800 MHz, CDCl₃) δ 7.78 (dd, *J* = 8.5, 5.4 Hz, 2H), 7.30 (d, *J* = 8.0 Hz, 1H), 7.24 (t, *J* = 7.9 Hz, 1H), 7.07 (t, *J* = 8.5 Hz, 3H), 6.84 (s, 1H), 6.61 (d, *J* = 7.7 Hz, 1H), 3.87 (d, *J* = 12.1 Hz, 1H), 3.66 (dd, *J* = 10.9, 5.3 Hz, 2H), 3.25 (m, 3H), 3.05 (dd, *J* = 15.5, 2.9 Hz, 1H), 2.96 (td, *J* = 12.0, 2.6 Hz, 1H), 2.86 (m, 2H), 2.82 - 2.78 (m, 1H), 2.62 (t, *J* = 10.9 Hz, 1H), 2.36 (t, *J* = 10.4 Hz, 1H), 2.04 (s, 1H). ¹³C NMR (201 MHz, CDCl₃) δ 165.48, 164.22, 162.77, 139.74, 129.42 (d, *J* = 8.9 Hz), 127.77, 126.19, 116.06, 115.70, 115.59, 113.27, 104.65, 60.40, 58.57, 56.65, 52.24, 45.66, 36.55, 31.47. HRMS (ESI, m/z): C₂₂H₂₃FN₃OS⁺ [M+H]⁺ calculated: 396.1540, found: 396.1687.

### Example 13: Synthesis of N-(2-(6a,7,9,10-tetrahydropyrazino[1,2-a]thieno[4,3,2-de]quinolin-8(6H)-yl)ethyl)thiophene-2-carboxamide (compound I-13)

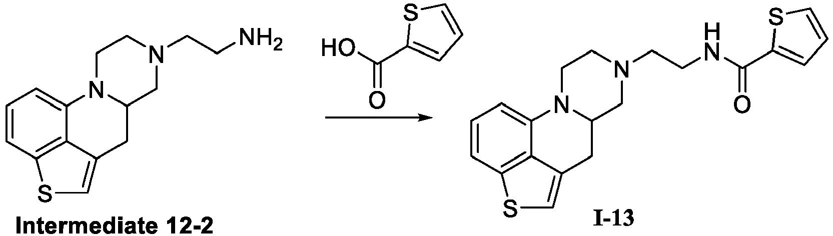

The synthesis was conducted in a manner similar to the synthesis of compound **I-12,** with "*p*-fluorobenzoic acid" in step 3 replaced by "thiophene-2-carboxylic acid". Compound **I-13** (13 mg, yield of 46%) was obtained as a brown solid. ¹H NMR (800 MHz, CDCl₃) δ 7.54 (d, *J* = 3.3 Hz, 1H), 7.46 (dd, *J* = 4.9, 0.8 Hz, 1H), 7.30 (d, *J* = 8.0 Hz, 1H), 7.24 (t, *J* = 7.9 Hz, 1H), 7.05 (dd, *J* = 4.8, 3.9 Hz, 1H), 6.86 (s, 1H), 6.63 (d, *J* = 7.7 Hz, 1H), 3.89 (d, *J* = 12.2 Hz, 1H), 3.38 - 3.28 (m, 3H), 3.08 (dd, *J* = 15.7, 3.2 Hz, 1H), 3.00 (td, *J* = 12.1, 2.9 Hz, 1H), 2.92 (s, 1H), 2.85 - 2.81 (m, 1H), 2.69 (t, *J* = 10.5 Hz, 1H), 2.44 (t, *J* = 10.3 Hz, 1H). ¹³C NMR (201 MHz, CDCl₃) δ 162.67, 144.13, 139.68, 138.62, 130.26, 128.38, 128.29, 127.83, 127.76, 126.18, 116.03, 113.19, 104.68, 55.15, 52.19, 43.19, 38.60, 36.54, 31.44, 30.54. HRMS (ESI, m/z): C₂₀H₂₂N₃OS₂⁺ [M+H]⁺ calculated: 384.1199, found: 384.1237.

### Example 14: Synthesis of N-(2-(6a,7,9,10-tetrahydropyrazino[1,2-a]thieno[4,3,2-de]quinolin-8(6H)-yl)ethyl)tetrahydro-2H-pyran-4-carboxamide (compound I-14)

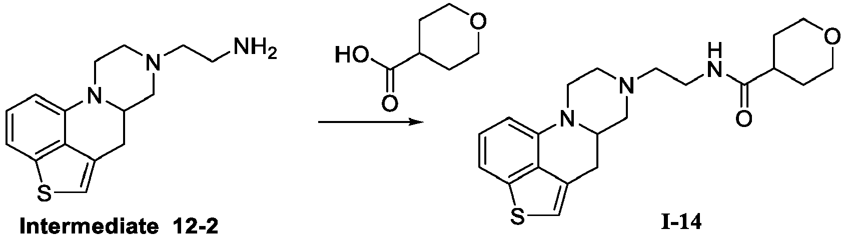

The synthesis was conducted in a manner similar to the synthesis of compound **I-12,** with "*p*-fluorobenzoic acid" in step 3 replaced by "tetrahydropyran-4-carboxylic acid" (CAS# 5337-03-1). Compound **I-14** (15 mg, yield of 39%) was obtained as a brown solid. ¹H NMR (800 MHz, CDCl₃) δ 7.30 (d, *J* = 8.0 Hz, 1H), 7.24 (t, *J* = 7.9 Hz, 1H), 6.84 (s, 1H), 6.62 (d, *J* = 7.7 Hz, 1H), 6.28 (s, 1H), 3.98 (m, 2H), 3.83 (d, *J* = 11.8 Hz, 1H), 3.45 (dd, *J* = 11.2, 5.4 Hz, 2H), 3.39 (td, *J* = 11.5, 1.5 Hz, 2H), 3.23 (t, *J* = 10.8 Hz, 1H), 3.17 - 3.08 (m, 2H), 3.04 (dd, *J* = 15.6, 3.3 Hz, 1H), 2.92 (td, *J* = 11.8, 2.7 Hz, 1H), 2.81 (ddd, *J* = 15.5, 11.5, 1.7 Hz, 1H), 2.66 (t, *J* = 5.1 Hz, 2H), 2.49 (t, *J* = 10.6 Hz, 1H), 2.40 - 2.32 (m, 1H), 2.23 (t, *J* = 10.8 Hz, 1H), 1.81 - 1.71 (m, 4H), 1.43 (d, *J* = 5.9 Hz, 1H). ¹³CNMR (201 MHz, CDCl₃) δ 139.71, 128.57, 127.81, 126.19, 115.82, 113.07, 104.53, 67.26, 58.92, 56.48, 55.09, 54.94, 52.26, 45.93, 42.09, 41.00, 35.64, 30.81, 29.27, 29.25. HRMS (ESI, m/z): C₂₁H₂₈N₃O₂ S⁺ [M+H]⁺ calculated: 386.1897, found: 386.1990.

### Example 15: Synthesis of 8-(2-phenoxyethyl)-6,6a,7,8,9,10-hexahydropyrazino[1,2-a]thieno[4,3,2-de]quinoline (compound I-15)

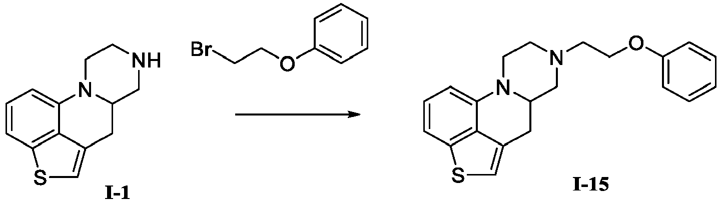

The synthesis was conducted in a manner similar to the synthesis of compound **I-2,** with 1-bromopropane replaced by 2-phenoxyethyl bromide (CAS#589-10-6). Compound **I-15** (14 mg, yield of 48%) was obtained as a colorless oil. ¹H NMR (800 MHz, CDCl₃) δ 7.29 (dd, *J* = 8.4, 7.5 Hz, 2H), 7.27 (d, *J* = 8.0 Hz, 1H), 7.23 (t, *J* = 7.8 Hz, 1H), 6.96 (t, *J* = 7.3 Hz, 1H), 6.93 (d, *J* = 7.9 Hz, 2H), 6.80 (s, 1H), 6.60 (d, *J* = 7.7 Hz, 1H), 4.17 (t, *J* = 5.7 Hz, 2H), 3.78 - 3.75 (m, 1H), 3.22 (m, 1H), 3.19 - 3.13 (m, 2H), 3.00 (dd, *J* = 15.6, 3.3 Hz, 1H), 2.93 (td, *J* = 11.9, 3.2 Hz, 1H), 2.89 (t, *J* = 5.7 Hz, 2H), 2.79 (m, 1H), 2.51 (td*, J* = 11.7, 3.1 Hz, 1H), 2.25 (t, *J =* 10.7 Hz, 1H). ¹³C NMR (201 MHz, CDCl₃) δ 158.69, 144.89, 139.63, 129.53, 129.03, 127.86, 126.20, 120.98, 115.44, 114.66, 112.72, 104.42, 65.87, 60.14, 57.11, 54.98, 53.16, 46.07, 30.96. HRMS (ESI, m/z): C₂₁H₂₃N₂OS⁺ [M+H]⁺ calculated: 351.1526, found: 351.1607.

### Example 16: Synthesis of 8-(3-(benzo[d][1,3]dioxol-5-yloxy)propyl)-6,6a,7,8,9,10-hexahydropyrazino[1,2-a]thieno[4,3,2-de]quinoline (compound I-16)

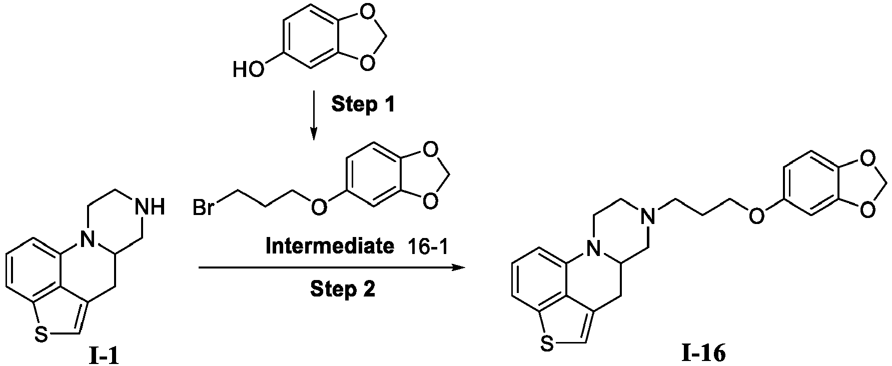

Step 1: Synthesis of intermediate **16-1** (5-(3-bromopropoxy)benzo[*d*][1,3]dioxole). Sesamol (690 mg, 5 mmol) was dissolved in *N,N*-dimethylformamide, and then cesium carbonate (1.9 g, 6 mmol) and 1,3-dibromobutane (2.0 g, 10 mmol) were sequentially added thereto. The reaction mixture was stirred at room temperature for 4 hours, then added with water, and extracted with ethyl acetate. The organic phases were combined, dried over anhydrous sodium sulfate, concentrated, and purified by column chromatography (petroleum ether/ethyl acetate = 5:1) to obtain a colorless oil (201 mg, yield of 78%). HRMS (ESI): C₁₀H₁₂BrO₃⁺ [M+H]⁺ calculated: 258.9964, found: 259.0039.

Step 2: Following the synthesis method of compound I-2, compound I-1 was alkylated with intermediate 16-1 to obtain compound **I-16** (21 mg, yield of 61%) as a pale yellow solid. ¹H NMR (800 MHz, CDCl₃) δ 7.28 - 7.24 (m, 1H), 7.22 (t, *J* = 7.8 Hz, 1H), 6.80 (s, 1H), 6.69 (d, *J* = 8.4 Hz, 1H), 6.60 (d, *J* = 7.7 Hz, 1H), 6.50 (d, *J* = 2.4 Hz, 1H), 6.32 (dd, *J* = 8.4, 2.4 Hz, 1H), 5.89 (s, 2H), 3.96 (t, *J* = 6.3 Hz, 2H), 3.77 (d, *J* = 11.6 Hz, 1H), 3.24 - 3.17 (m, 1H), 3.12 - 3.04 (m, 2H), 3.00 (dd, *J* = 15.6, 3.3 Hz, 1H), 2.89 (td, *J* = 11.8, 3.1 Hz, 1H), 2.78 (m, 1H), 2.58 (m, 2H), 2.34 (td, *J* = 11.7, 2.9 Hz, 1H), 2.09 (t, *J* = 10.7 Hz, 1H), 2.02 - 1.96 (m, 2H). ¹³C NMR (201 MHz, CDCl₃) δ 154.51, 148.27, 144.93, 141.62, 139.62, 129.08, 127.86, 126.19, 115.41, 112.69, 107.97, 105.71, 104.42, 101.13, 98.11, 67.06, 59.77, 55.03, 55.00, 52.74, 46.10, 30.99, 26.71. HRMS (ESI, m/z): C₂₃H₂₅N₂O₃S⁺ [M+H]⁺ calculated: 409.1580, found: 409.1621.

### Example 17: Synthesis of 7-(3-(6a,7,9,10-tetrahydropyrazino[1,2-a]thieno[4,3,2-de]quinolin-8(6H)-yl)propoxy)quinolin-2(1H)-one (compound I-17)

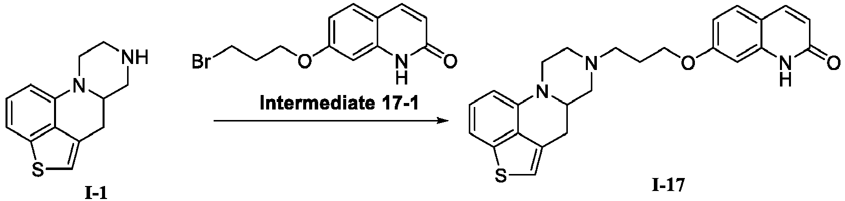

Compound I-1 (50 mg, 0.217 mmol), intermediate **17-1** (prepared according to the method described in CN202110138249.9) (92 mg, 0.326 mmol), and K₂CO₃ (90 mg, 0.65 mmol) were sequentially added to a round-bottom flask. Then, the solvents THF (3 mL) and DMSO (1 mL) were added thereto. The reaction mixture was heated and stirred at 70°C for 20 hours. After the reaction was completed, the solvent was removed. The residue was purified by flash silica gel column chromatography (eluent: 0 to 10% methanol/dichloromethane) and further purified by preparative HPLC (mobile phase: MeOH/H₂O, t_{R} = 24 min) to obtain compound **I-17** (20 mg, 21%) as a white solid. ¹H NMR (800 MHz, CD₃OD) δ 7.89 (d, *J* = 9.5 Hz, 1H), 7.60 (d, *J* = 8.7 Hz, 1H), 7.36 (d, *J* = 8.0 Hz, 1H), 7.28 (t, *J* = 7.9 Hz, 1H), 7.08 (d, *J* = 1.7 Hz, 1H), 6.91 (dd, *J* = 8.7, 2.4 Hz, 1H), 6.87 (d, *J* = 2.4 Hz, 1H), 6.80 (d, *J* = 7.8 Hz, 1H), 6.45 (d, *J* = 9.4 Hz, 1H), 4.27 - 4.22 (m, 3H), 3.87 (t, *J* = 11.5 Hz, 2H), 3.50 - 3.48 (m, 2H), 3.41 - 3.36 (m, 2H), 3.25 (d, *J* = 15.7, 3.7 Hz, 1H), 3.16 - 3.09 (m, 2H), 2.91 - 2.87 (m, 1H), 2.39 - 2.36 (m, 2H). HR-MS (ESI, m/z): C₂₅H₂₆N₃O₂S⁺ [M+H]⁺, calculated: 432.1740; found: 432.1746.

### Example 18: Synthesis of 7-(3-(6a,7,9,10-tetrahydropyrazino[1,2-a]thieno[4,3,2-de]quinolin-8(6H)-yl)propoxy)-3,4-dihydroquinolin-2(1H)-one (compound I-18)

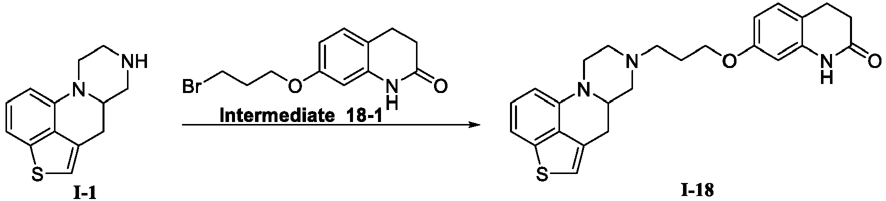

Compound **I-1** (50 mg, 0.217 mmol), intermediate **18-1** (prepared according to the method described in CN202110138249.9) (91 mg, 0.326 mmol), and K₂CO₃ (90 mg, 0.65 mmol) were sequentially added to a round-bottom flask. Then, the solvents THF (3 mL) and DMSO (1 mL) were added thereto. The reaction mixture was heated and stirred at 65°C for 20 hours. After the reaction was completed, the solvent was removed. The residue was purified by flash silica gel column chromatography (eluent: 0 to 10% methanol/dichloromethane) to obtain compound **I-18** (20 mg, 21%) as an off-white solid. ¹H NMR (800 MHz, CDCl₃) δ 8.14 (d, *J* = 6.2 Hz, 1H), 7.27 (d, *J* = 8.0 Hz, 1H), 7.23 (t, *J* = 7.8 Hz, 1H), 7.04 (d, *J* = 8.3 Hz, 1H), 6.82 (d, *J* = 1.9 Hz, 1H), 6.61 (d, *J* = 7.7 Hz, 1H), 6.53 (dd, *J* = 8.3, 2.4 Hz, 1H), 6.34 (d, *J* = 2.5 Hz, 1H), 4.02 (t, *J* = 6.2 Hz, 2H), 3.79 (d, *J* = 11.7 Hz, 1H), 3.21 (t, *J* = 11.0 Hz, 1H), 3.13 - 3.06 (m, 2H), 3.02 (dd, *J* = 15.6, 3.5 Hz, 1H), 2.95 - 2.86 (m, 3H), 2.83 - 2.79 (m, 1H), 2.63 - 2.57 (m, 4H), 2.37 (t, *J* = 11.6 Hz, 1H), 2.12 (t, *J* = 10.7 Hz, 1H), 2.04 - 2.00 (m, 2H). HR-MS (ESI, m/z): C₂₅H₂₈N₃O₂S⁺ [M+H]⁺, calculated: 434.1897; found: 434.1896.

### Example 19: Synthesis of 7-(4-(6a,7,9,10-tetrahydropyrazino[1,2-a]thieno[4,3,2-de]quinolin-8(6H)-yl)butoxy)quinolin-2(1H)-one (compound I-19)

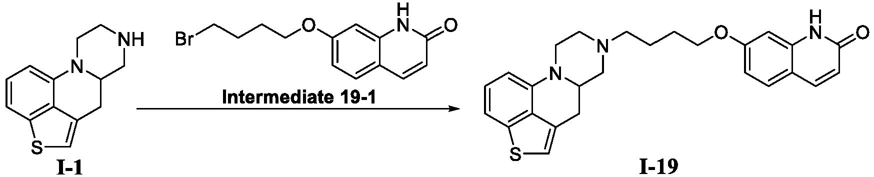

Compound **I-1** (50 mg, 0.217 mmol), intermediate **19-1** (prepared according to the method described in CN202110138249.9) (78 mg, 0.26 mmol), and K₂CO₃ (90 mg, 0.65 mmol) were sequentially added to a round-bottom flask. Then, the solvents THF (3 mL) and DMSO (1 mL) were added thereto. The reaction mixture was heated and stirred at 60°C for 18 hours. After the reaction was completed, the solvent was removed. The residue was purified by flash silica gel column chromatography (eluent: 0 to 10% methanol/dichloromethane) and further purified by preparative HPLC (20 to 80% MeOH/H₂O, t_{R} = 22.5 min) to obtain **I-19** (20 mg, 21%) as a white solid. ¹H NMR (800 MHz, CD₃OD) δ 7.88 (d, *J* = 9.4 Hz, 1H), 7.58 (d, *J* = 8.7 Hz, 1H), 7.37 - 7.33 (m, 1H), 7.27 (t, *J* = 7.9 Hz, 1H), 7.06 (s, 1H), 6.90 (d, *J* = 8.4 Hz, 1H), 6.86 (s, 1H), 6.78 (d, *J* = 7.8 Hz, 1H), 6.45 (d, *J* = 9.4 Hz, 1H), 4.23 (d, *J* = 13.8 Hz, 1H), 4.17 (t, *J* = 6.0 Hz, 2H), 3.81 (t, *J* = 10.8 Hz, 2H), 3.40 (t, *J* = 11.2 Hz, 1H), 3.34 (t, *J* = 7.9 Hz, 4H), 3.23 (d, *J* = 15.3 Hz, 1H), 3.08 (t, *J* = 12.4 Hz, 2H), 2.88 - 2.84 (m, 1H), 2.07 - 2.04 (m, 2H), 1.98 - 1.95 (m, 2H). HR-MS (ESI, m/z): C₂₆H₂₈N₃O₂S⁺ [M+H]⁺, calculated: 446.1897; found: 446.1887.

### Example 20: 7-(4-(6a,7,9,10-tetrahydropyrazino[1,2-a]thieno[4,3,2-de]quinolin-8(6H)-yl)butoxy)-3,4-dihydroquinolin-2(1H)-one (compound I-20)

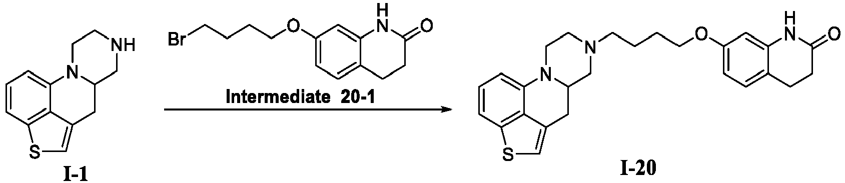

Compound **I-1** (53 mg, 0.23 mmol), intermediate **20-1** (prepared according to the method described in CN202110138249.9) (98 mg, 0.33 mmol), and K₂CO₃ (160 mg, 1.16 mmol) were sequentially added to a round-bottom flask. Then, the solvents THF (3 mL) and DMSO (1 mL) were added thereto. The reaction mixture was heated and stirred at 70°C for 20 hours. After the reaction was completed, the solvent was removed. The residue was purified by flash silica gel column chromatography (eluent: 0 to 10% methanol/dichloromethane) to obtain compound **I-20** (64 mg, 62%) as a white solid. ¹H NMR (800 MHz, CDCl₃) δ 7.38 (d, *J* = 7.7 Hz, 1H), 7.23 (t, *J* = 7.8 Hz, 1H), 7.04 (d, *J* = 8.3 Hz, 1H), 6.81 (d, *J* = 1.9 Hz, 1H), 6.61 (d, *J* = 7.7 Hz, 1H), 6.53 (dd, *J* = 8.3, 2.4 Hz, 1H), 6.27 (d, *J* = 2.4 Hz, 1H), 3.97 (t, *J* = 6.3 Hz, 2H), 3.78 (d, *J* = 11.5 Hz, 1H), 3.17 - 3.15 (m, 1H), 3.07 (t, *J* = 13.4 Hz, 2H), 3.03 - 2.99 (m, 1H), 2.89 (t, *J* = 7.5 Hz, 3H), 2.82 - 2.78 (m, 1H), 2.61 - 2.59 (m, 2H), 2.49 - 2.47 (m, 2H), 2.34 - 2.30 (m, 1H), 2.06 (t, *J =* 10.6 Hz, 1H), 1.86 - 1.82 (m, 2H), 1.75 - 1.72 (m, 2H). ¹³C NMR (201 MHz, CDCl₃) δ 158.72, 145.03, 139.66, 138.32, 129.20, 128.67, 127.92, 126.25, 115.77, 115.42, 112.72, 108.78, 104.46, 102.40, 67.90, 59.82, 58.14, 55.14, 52.84, 46.21, 31.15, 31.06, 27.33, 24.63, 23.38. HR-MS (ESI, m/z): C₂₆H₃₀N₃O₂S⁺ [M+H]⁺ calculated: 448.2053; found: 448.2015.

### Example 21: Synthesis of 8-(4-(benzo[d]thiazol-5-yloxy)butyl)-6,6a,7,8,9,10-hexahydropyrazino[1,2-a]thieno[4,3,2-de]quinoline (compound I-21)

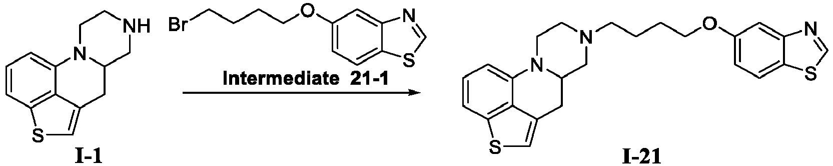

Compound **I-1** (50 mg, 0.217 mmol), intermediate **21-1** (prepared according to the method described in CN202110138249.9) (93 mg, 0.326 mmol), and K₂CO₃ (90 mg, 0.65 mmol) were sequentially added to a round-bottom flask. Then, the solvents THF (3 mL) and DMSO (1 mL) were added thereto. The reaction mixture was heated and stirred at 60°C for 20 hours. After the reaction was completed, the solvent was removed. The residue was purified by flash silica gel column chromatography (eluent: 0 to 10% methanol/dichloromethane) to obtain a compound, which was further purified by preparative HPLC (mobile phase: 20 to 80% MeOH/H₂O, t_{R} = 21.5 min) to obtain compound **I-21** (33 mg, 35%) as an off-white solid. ¹H NMR (800 MHz, DMSO-d₆) δ 9.37 (d, *J* = 1.6 Hz, 1H), 8.06 -8.03 (m, 1H), 7.66 (d, *J* = 2.9 Hz, 1H), 7.39 (dd, *J* = 8.0, 1.2 Hz, 1H), 7.27 (t, *J* = 7.9 Hz, 1H), 7.25 - 7.22 (m, 1H), 7.15 (dd, *J* = 8.7, 2.4 Hz, 1H), 6.80 (dd, *J* = 8.2, 2.3 Hz, 1H), 4.19 - 4.12 (m, 3H), 3.81 (d, *J* = 12.1 Hz, 1H), 3.75 (d, *J* = 12.2 Hz, 1H), 3.45 - 3.39 (m, 1H), 3.34 - 3.16 (m, 4H), 3.03 (t, *J* = 11.2 Hz, 2H), 2.79 - 2.73 (m, 1H), 1.96 - 1.92 (m, 2H), 1.89 - 1.81 (m, 2H). ¹³C NMR (201 MHz, DMSO) δ 157.75, 157.09, 154.50, 143.03, 138.99, 127.35, 127.22, 126.32, 125.43, 122.78, 117.17, 115.82, 113.38, 106.38, 105.48, 67.24, 55.64, 54.44, 52.58, 50.33, 43.28, 29.08, 25.77, 20.27. HR-MS (ESI, m/z): C₂₄H₂₆N₃OS₂⁺ [M+H]⁺ calculated: 436.1512; found: 436.1512.

### Example 22: Synthesis of 2-(4-(6a,7,9,10-tetrahydropyrazino[1,2-a]thieno[4,3,2-de]quinolin-8(6H)-yl)butyl)isoindolin-1-one (compound I-22)

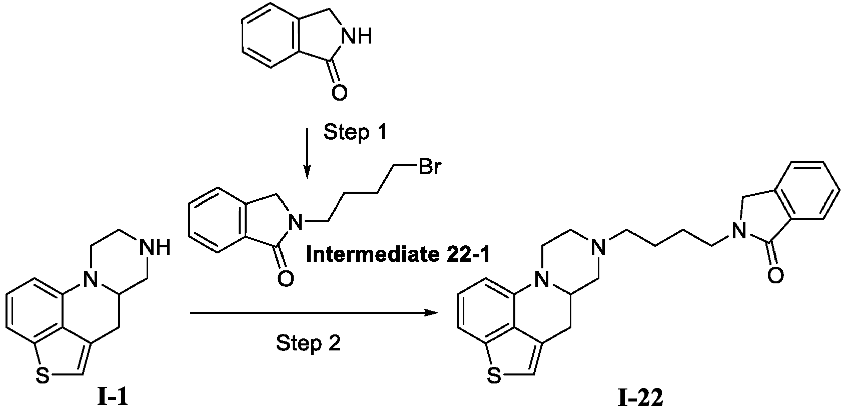

Step 1: Synthesis of intermediate **22-1** (2-(4-bromobutyl)isoindolin-1-one). Isoindolin-1-one (CAS# 480-91-1) (500 mg, 3.76 mmol), 1,4-dibromobutane (2 mL, 37.6 mmol), and sodium hydride (180 mg, 7.5 mmol) were placed in a reaction flask, and *N,N-*dimethylformamide (DMF) was added thereto. The reaction mixture was stirred until dissolved, and stirred and reacted at room temperature. After 4 hours, the reaction was completed. The reaction mixture was added with water and extracted with ethyl acetate. The organic phase was dried over anhydrous sodium sulfate, concentrated, and purified by column chromatography (petroleum ether/ethyl acetate = 5:1). A colorless oil (230 mg, yield of 77%) was obtained. HRMS (ESI, m/z): C₁₂H₁₅BrNO⁺ [M+H]⁺ calculated: 268.0332, found: 268.0561.

Step 2: Compound **I-1** (40 mg, 0.17 mmol), intermediate 22-1 (53 mg, 0.18 mmol), and K₂CO₃ (70 mg, 0.5 mmol) were sequentially added to a round-bottom flask. Then, the solvent DMF (3 mL) was added thereto. The reaction mixture was stirred at room temperature for 10 hours. After the reaction was completed, the solvent was removed. The residue was purified by flash silica gel column chromatography (eluent: 0 to 10% methanol/dichloromethane) to obtain compound **I-22** (16 mg, yield of 33%) as a pale yellow oil. ¹H NMR (800 MHz, CDCl₃) δ 7.85 (d, *J* = 7.5 Hz, 1H), 7.52 (td, *J* = 7.4, 0.9 Hz, 1H), 7.46 (t, *J* = 7.4 Hz, 1H), 7.43 (d, *J* = 7.5 Hz, 1H), 7.27 (s, 1H), 7.22 (t, *J* = 7.8 Hz, 1H), 6.80 (d, *J* = 0.8 Hz, 1H), 6.59 (d, *J* = 7.7 Hz, 1H), 4.39 (s, 2H), 3.76 (d, *J* = 11.7 Hz, 1H), 3.71 - 3.64 (m, 2H), 3.17 (t, *J* = 10.8 Hz, 1H), 3.06 (dd, *J* = 26.0, 10.9 Hz, 2H), 2.97 (dd, *J* = 15.6, 3.3 Hz, 1H), 2.91 - 2.86 (m, 1H), 2.77 (ddd, *J* = 15.5, 11.6, 1.8 Hz, 1H), 2.48 (t, *J* = 7.3 Hz, 2H), 2.32 (t, *J* = 10.5 Hz, 1H), 2.06 (t, *J* = 10.7 Hz, 1H), 1.78 - 1.72 (m, 2H), 1.63 (dt, *J* = 14.5, 7.5 Hz, 2H). ¹³C NMR (201 MHz, CDCl₃) δ 168.58, 144.84, 141.07, 139.59, 132.96, 131.21, 129.01, 128.05, 127.83, 126.15, 123.70, 122.66, 115.41, 112.69, 104.39, 59.60, 57.79, 54.95, 52.69, 49.81, 46.00, 42.05, 30.92, 26.22, 23.87. HRMS (ESI, m/z): C₂₅H₂₈N₃OS⁺ [M+H]⁺ calculated: 418.1948, found: 418.2008.

### Example 23: Synthesis of 5,6-dimethoxy-2-(4-(6a,7,9,10-tetrahydropyrazino[1,2-a]thieno[4,3,2-de]quinolin-8(6H)-yl)butyl)isoindolin-1-one (compound I-23)

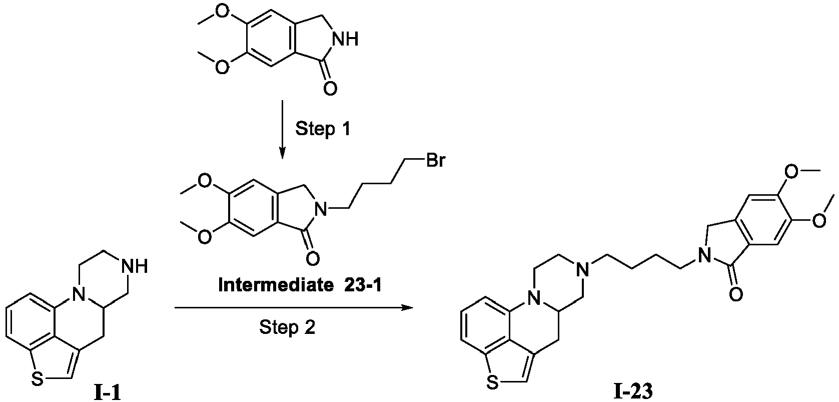

Step 1: Synthesis of intermediate **23-1** (2-(4-bromobutyl)-5,6-dimethoxyisoindolin-1-one). The synthesis was conducted in a manner similar to the synthesis of intermediate 22-1, with "isoindolin-1-one" replaced by "5,6-dimethoxyisoindolin-1-one" (CAS# 59084-72-9). A colorless oil (192 mg, yield of 77%) was obtained. HRMS (ESI, m/z): C₁₄H₁₉BrNO₃⁺ [M+H]⁺ calculated: 328.0543, found: 328.0617.

Step 2: Synthesis of compound **I-23.** The synthesis was conducted in a manner similar to the synthesis of compound **I-2,** with "1-bromopropane" replaced by intermediate **23-1. 1-23** (12 mg, yield of 39%) was obtained as a white solid. ¹H NMR (800 MHz, CDCl₃) δ 7.32 (s, 1H), 7.27 (s, 1H), 7.22 (t, *J* = 7.8 Hz, 1H), 6.89 (s, 1H), 6.80 (s, 1H), 6.59 (d, *J* = 7.7 Hz, 1H), 4.30 (q, *J* = 16.5 Hz, 2H), 3.94 (s, 3H), 3.91 (s, 3H), 3.75 (t, *J* = 9.8 Hz, 1H), 3.71 - 3.59 (m, 2H), 3.16 (s, 1H), 3.04 (dd, *J* = 43.2, 10.8 Hz, 2H), 2.96 - 2.86 (m, 2H), 2.75 (ddd, *J* = 15.5, 11.6, 1.7 Hz, 1H), 2.48 (t, *J* = 6.8 Hz, 2H), 2.32 (t, *J* = 10.6 Hz, 1H), 2.05 (t, *J* = 10.5 Hz, 1H), 1.78 - 1.70 (m, 2H), 1.66 - 1.58 (m, 2H). ¹³C NMR (201 MHz, CDCl₃) δ 168.90, 152.43, 149.69, 144.83, 139.60, 134.57, 128.98, 127.82, 126.15, 125.23, 115.44, 112.70, 105.37, 104.95, 104.39, 59.51, 57.74, 56.24, 54.99, 52.70, 49.48, 46.02, 42.09, 30.90, 26.25, 23.78. HRMS (ESI, m/z): C₂₇H₃₂N₃O₃S⁺ [M+H]⁺ calculated: 448.2159, found: 448.2215.

### Example 24: 2-(4-(6a,7,9,10-tetrahydropyrazino[1,2-a]thieno[4,3,2-de]quinolin-8(6H)-yl)butyl)-3,4-dihydroisoquinolin-1(2H)-one (compound I-24)

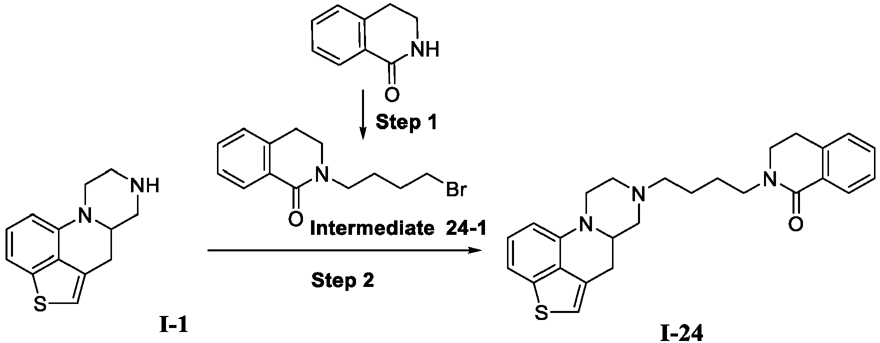

Step 1: Synthesis of intermediate **24-1.** The synthesis was conducted in a manner similar to the synthesis of intermediate **22-1,** with "isoindolin-1-one" replaced by "3,4-dihydroisoquinolin-1(2H)-one". Intermediate **24-1** (168 mg, yield of 68%) was obtained as a colorless oil. HRMS (ESI, m/z): C₁₃H₁₇BrNO⁺ [M+H]⁺ calculated: 282.0488, found: 282.0509.

Step 2: Synthesis of compound **1-24.** The synthesis was conducted in a manner similar to the synthesis of compound **1-2,** with "1-bromopropane" replaced by intermediate **24-1.** Compound **I-24** (10 mg, yield of 32%) was obtained as a pale yellow oil. ¹H NMR (800 MHz, CDCl₃) δ 8.07 (d, *J* = 7.6 Hz, 1H), 7.41 (td, *J* = 7.4, 1.1 Hz, 1H), 7.34 (t, *J* = 7.5 Hz, 1H), 7.27 (s, 1H), 7.23 (t, *J* = 7.8 Hz, 1H), 7.17 (d, *J* = 7.4 Hz, 1H), 6.81 (s, 1H), 6.60 (d, *J* = 7.7 Hz, 1H), 3.77 (d, *J* = 11.7 Hz, 1H), 3.62 (td, *J* = 7.0, 2.1 Hz, 2H), 3.56 (t, *J* = 6.6 Hz, 2H), 3.20 (s, 1H), 3.11 - 3.06 (m, 2H), 3.02 - 2.97 (m, 3H), 2.91 (s, 1H), 2.78 (ddd, *J* = 15.5, 11.6, 1.8 Hz, 1H), 2.49 (s, 2H), 2.33 (m, 1H), 2.08 (t, *J* = 9.9 Hz, 1H), 1.70 (m, 2H), 1.67 - 1.61 (m, 2H). HRMS (ESI, m/z): C₂₆H₃₀N₃OS⁺ [M+H]⁺ calculated: 432.2104, found: 432.2221.

### Example 25: Synthesis of 2-(4-(6a,7,9,10-tetrahydropyrazino[1,2-a]thieno[4,3,2-de]quinolin-8(6H)-yl)butyl)-2,3,4,5-tetrahydro-1H-benzo[c]azepin-1-one (compound I-25)

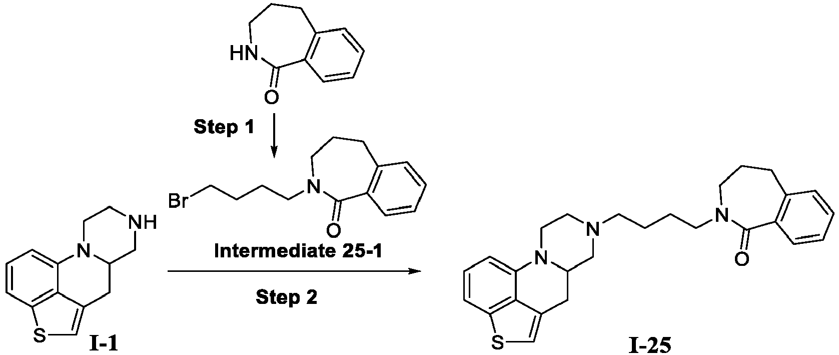

Step 1: Synthesis of intermediate **25-1.** The synthesis was conducted in a manner similar to the synthesis of intermediate **22-1,** with "isoindolin-1-one" replaced by "2,3,4,5-tetrahydro-1*H*-benzo[c]azepin-1-one" (CAS# 6729-50-6). A colorless oil (180 mg, yield of 71%) was obtained. HRMS (ESI, m/z): C₁₄H₁₉BrNO⁺ [M+H]⁺ calculated: 296.0645, found: 296.0728.

Step 2: Synthesis of compound **I-25.** The synthesis was conducted in a manner similar to the synthesis of compound **I-2,** with 1-bromopropane replaced by intermediate **25-1.** Compound **I-25** (36 mg, yield of 67%) was obtained as a pale yellow oil. ¹H NMR (800 MHz, CDCl₃) δ 7.64 (dd, *J* = 7.5, 1.0 Hz, 1H), 7.35 (td, *J* = 7.4, 1.3 Hz, 1H), 7.31 (td, *J* = 7.5, 0.9 Hz, 1H), 7.27 (d, *J* = 7.0 Hz, 1H), 7.22 (t, *J* = 7.8 Hz, 1H), 7.12 (d, *J* = 7.4 Hz, 1H), 6.81 (s, 1H), 6.59 (d, *J* = 7.7 Hz, 1H), 3.79 (d, *J* = 12.0 Hz, 1H), 3.60 (t, *J* = 6.6 Hz, 2H), 3.35 (s, 1H), 3.21 - 3.12 (m, 4H), 3.08 - 3.01 (m, 2H), 2.80 - 2.74 (m, 3H), 2.59 (s, 2H), 2.43 (t, *J* = 10.8 Hz, 1H), 2.17 (t, *J* = 10.7 Hz, 1H), 2.01 (m, 2H), 1.71 (m, 4H). ¹³C NMR (201 MHz, CDCl₃) δ 171.13, 144.42, 139.65, 137.21, 136.35, 130.75, 128.62, 128.50, 128.23, 127.78, 126.97, 126.21, 115.71, 112.87, 104.59, 60.39, 57.84, 54.46, 52.25, 46.83, 46.23, 45.57, 40.99, 30.73, 30.27, 29.93, 26.71, 23.52, 21.07, 14.22. HRMS (ESI, m/z): C₂₇H₃₂N₃OS⁺ [M+H]⁺ calculated: 446.2261, found: 446.2307.

### Example 26: Synthesis of 2-(4-(6a,7,9,10-tetrahydropyrazino[1,2-a]thieno[4,3,2-de]quinolin-8(6H)-yl)butyl)-2,3,4,5-tetrahydro-1H-benzo[c]azepin-1-one (compound I-26)

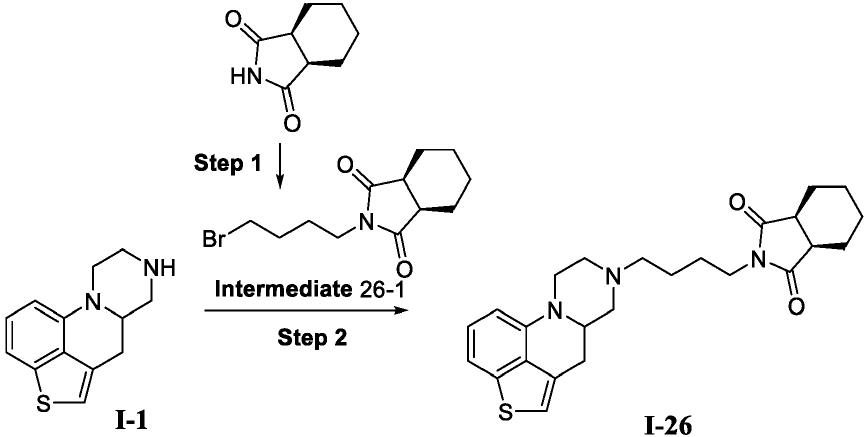

Step 1: Synthesis of intermediate **26-1** (2-(4-bromobutyl)hexahydro-1*H*-isoindole-1,3(2H)-dione). The synthesis was conducted in a manner similar to the synthesis of intermediate **22-1,** with "isoindolin-1-one" replaced by "cis-cyclohexane-1,2-dicarboximide". A colorless oil (201 mg, yield of 78%) was obtained. HRMS (ESI, m/z): C₁₂H₁₉BrNO₂⁺ [M+H]⁺ calculated: 288.0294, found: 288.0332.

Step 2: Synthesis of compound **I-26**. The synthesis was conducted in a manner similar to the synthesis of compound **I-2,** with 1-bromopropane replaced by intermediate 26-1. Compound **I-26** (31 mg, yield of 68%) was obtained as a pale yellow solid. ¹H NMR (800 MHz, CDCl₃) δ 7.27 - 7.25 (m, 1H), 7.22 (t, *J* = 7.8 Hz, 1H), 6.80 (d, *J* = 1.1 Hz, 1H), 6.59 (d, *J* = 7.7 Hz, 1H), 3.75 (d, *J* = 11.6 Hz, 1H), 3.52 (t, *J* = 7.3 Hz, 2H), 3.17 (t, *J* = 10.9 Hz, 1H), 3.07 - 2.97 (m, 3H), 2.88 (td, *J* = 11.7, 2.7 Hz, 1H), 2.83 (dd, *J* = 10.6, 6.1 Hz, 2H), 2.77 (ddd, *J* = 15.5, 11.6, 1.9 Hz, 1H), 2.44 - 2.39 (m, 2H), 2.29 (td, *J* = 11.7, 2.7 Hz, 1H), 2.04 (t, *J* = 10.7 Hz, 1H), 1.90 - 1.83 (m, 2H), 1.78 - 1.69 (m, 2H), 1.63 (dt, *J* = 15.0, 7.5 Hz, 2H), 1.54 (dt, *J* = 15.1, 7.5 Hz, 2H), 1.49 - 1.44 (m, 2H), 1.43 - 1.37 (m, 2H). ¹³C NMR (201 MHz, CDCl₃) δ 179.82, 144.94, 139.58, 129.11, 127.84, 126.17, 115.35, 112.63, 104.38, 99.99, 59.76, 57.80, 55.02, 52.70, 46.10, 39.72, 38.28, 30.97, 25.77, 24.03, 23.77, 21.61. HRMS (ESI, m/z): C₂₅H₃₂N₃O₂S⁺ [M+H]⁺ calculated: 438.2210, found: 438.2381.

### Example 27: Synthesis of 1,1-dimethyl-3-(4-(2-(6a,7,9,10-tetrahydropyrazino[1,2-a]thieno[4,3,2-de]quinolin-8(6H)-yl)ethyl)trans-cyclohexyl)urea (compound I-27)

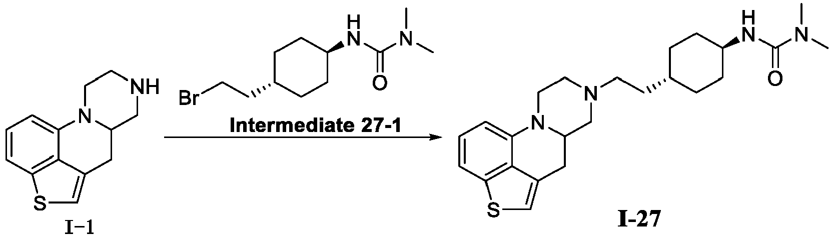

Compound **I-1** (50 mg, 0.217 mmol), intermediate **27-1** (prepared according to the method described in CN202110138249.9) (78.3 mg, 0.283 mmol), and K₂CO₃ (90 mg, 0.65 mmol) were sequentially added to a round-bottom flask. Then, the solvents THF (3 mL) and DMSO (1 mL) were added thereto. The reaction mixture was heated and stirred at 60°C for 18 hours. After the reaction was completed, the solvent was removed. The residue was purified by flash silica gel column chromatography (eluent: 0 to 10% methanol/dichloromethane) to obtain compound **I-27** (22 mg, 24%) as an off-white solid. ¹H NMR (800 MHz, CDCl₃) δ 7.27 (s, 1H), 7.23 (t, *J* = 7.8 Hz, 1H), 6.81 (s, 1H), 6.61 (d, *J* = 7.6 Hz, 1H), 4.11 (d, *J* = 7.6 Hz, 1H), 3.78 (d, *J* = 11.6 Hz, 1H), 3.60 - 3.56 (m, 1H), 3.24 - 3.20 (m, 1H), 3.08 (t, *J* = 13.2 Hz, 2H), 3.03 (dd, *J* = 15.6, 3.4 Hz, 1H), 2.94 - 2.90 (m, 1H), 2.88 (s, 6H), 2.79 (dd, *J* = 15.5, 11.6 Hz, 1H), 2.44 (t, *J* = 8.1 Hz, 2H), 2.34 - 2.30 (m, 1H), 2.08 - 1.98 (m, 3H), 1.81 - 1.76 (m, 2H), 1.49 - 1.46 (m, 2H), 1.29 - 1.24 (m, 1H), 1.14 - 1.04 (m, 3H). HR-MS (ESI, m/z): C₂₄H₃₅N₄OS⁺ [M+H]⁺, calculated: 427.2526; found: 427.2517.

Chiral resolution of compound **I-27**.

Chiral analysis conditions: chiral column: CHIRALPAK IBN-5 (Dacel), 0.46 cm (diameter) x 15 cm (column length) (5 µm particle size filler); mobile phase: methanol/diethylamine = 100/0.1 (V/V); flow rate: 1.0 mL/min; wavelength: UV 214 nm; temperature: 35°C; HPLC instrument: Shimadzu LC-20AD. peak1 (prepeak): t_{R} = 4.335 min; peak2 (postpeak): t_{R} = 5.573 min.

Chiral preparation conditions: chiral column: CHIRALPAK IBN-5 (Dacel), 5 cm (diameter) x 25 cm (column length) (10 µm particle size filler); mobile phase: methanol/diethylamine = 100/0.1 (V/V); flow rate: 60 mL/min; wavelength: UV 254 nm; temperature: 38°C; HPLC instrument: YMC K-Prep 100.

Chiral resolution: racemate compound **I-27** (1.28 g), (+)-**I-27** is the prepeak (peak1), t_{R} = 4.339 min, 0.342 g obtained, > 98% ee, optical rotation [α]_{D}²⁵ = +61.00° (c = 0.1, CHCl₃); (-)-**I-27** is the postpeak (peak2), t_{R} = 5.580 min, 0.347 g obtained, > 98% ee, optical rotation [α]_{D}^{Z5} = -57.00° (c = 0.1, CHCl₃).

### Example 28: Synthesis of N-(4-(2-(6a,7,9,10-tetrahydropyrazino[1,2-a]thieno[4,3,2-de]quinolin-8(6H)-yl)ethyl)trans-cyclohexyl)pyrrolidine-1-carboxamide (compound I-28)

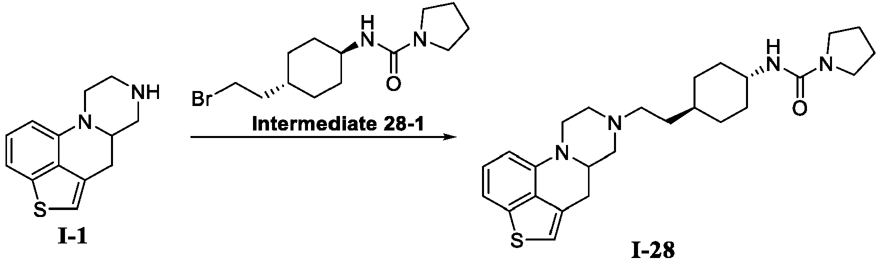

Compound **I-1** (50 mg, 0.22 mmol), intermediate **28-1** (prepared according to the method described in CN202110138249.9) (130 mg, 0.42 mmol), and DIPEA (diisopropylethylamine) (1 mL) were sequentially added to a round-bottom flask. Then, the solvent DMSO (3 mL) was added thereto. The reaction mixture was heated and stirred at 60°C for 17 hours. After the reaction was completed, the reaction mixture was added with water and extracted three times with ethyl acetate. The organic phases were combined and concentrated to remove the solvent. The residue was purified by flash silica gel column chromatography (eluent: 0 to 10% methanol/dichloromethane) to obtain compound **I-28** (70 mg, 70%) as an off-white solid. ¹H NMR (800 MHz, DMSO-d₆) δ 7.28 (d, *J* = 8.0 Hz, 1H), 7.20 (t, *J =* 7.8 Hz, 1H), 7.10 (s, 1H), 6.66 (d, *J* = 7.8 Hz, 1H), 5.63 (d, *J =* 8.0 Hz, 1H), 3.80 - 3.76 (m, 1H), 3.38 - 3.30 (m, 2H), 3.19 - 3.15 (m, 4H), 3.09 - 3.04 (m, 2H), 3.03 - 3.00 (m, 2H), 2.74 - 2.64 (m, 3H), 2.38 - 2.34 (m, 2H), 2.173 - 2.13 (m, 1H), 1.94 - 1.90 (m, 1H), 1.79 - 1.71 (m, 8H), 1.41 - 1.37 (m, 2H), 1.22 - 1.15 (m, 3H). HR-MS (ESI, m/z): C₂₆H₃₇N₄OS⁺ [M+H]⁺ calculated: 453.2683; found: 453.2677.

### Example 29: Synthesis of N-(4-(2-(6a,7,9,10-tetrahydropyrazino[1,2-a]thieno[4,3,2-de]quinolin-8(6H-yl)ethyl)trans-cyclohexyl)piperidine-1-carboxamide (compound I-29)

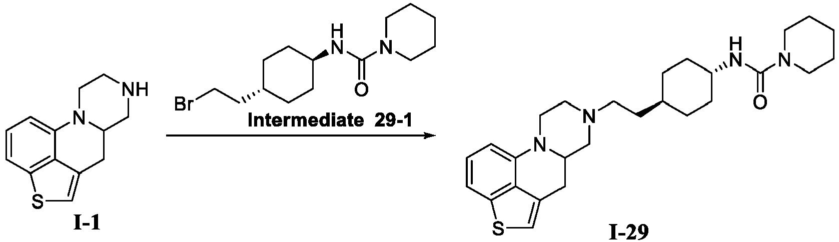

Compound **I-1** (50 mg, 0.217 mmol), intermediate **29-1** (prepared according to the method described in CN202110138249.9) (80 mg, 0.25 mmol), and K₂CO₃ (90 mg, 0.65 mmol) were sequentially added to a round-bottom flask. Then, the solvents THF (3 mL) and DMSO (1 mL) were added thereto. The reaction mixture was heated and stirred at 65°C for 19 hours. After the reaction was completed, the solvent was removed. The residue was purified by flash silica gel column chromatography (eluent: 0 to 10% methanol/dichloromethane) to obtain compound **I-29** (36 mg, 35%) as a white solid. ¹H NMR (800 MHz, CDCl₃) δ 7.27 (s, 1H), 7.23 (t, *J* = 7.8 Hz, 1H), 6.81 (d, *J* = 1.9 Hz, 1H), 6.61 (d, *J* = 7.6 Hz, 1H), 4.20 (d, *J* = 7.6 Hz, 1H), 3.80 - 3.76 (m, 1H), 3.68 (t, *J* = 6.6 Hz, 1H), 3.62 - 3.58 (m, 1H), 3.29 (t, *J* = 5.5 Hz, 4H), 3.21 (d, *J* = 12.5 Hz, 1H), 3.08 (t, *J* = 13.2 Hz, 1H), 3.04 - 3.00 (m, 1H), 2.93 (d, *J* = 12.1 Hz, 1H), 2.80 - 2.77 (m, 1H), 2.62 - 2.60 (m, 1H), 2.44 (t, *J* = 8.0 Hz, 1H), 2.31 (t, *J* = 11.6 Hz, 1H), 2.07 - 1.98 (m, 3H), 1.78 (d, *J* = 13.9 Hz, 2H), 1.60 - 1.56 (m, 2H), 1.55 - 1.50 (m, 4H), 1.50 - 1.46 (m, 2H), 1.28 - 1.24 (m, 1H), 1.10 - 1.06 (m, 4H). HR-MS (ESI, m/z): C₂₇H₃₉N₄OS⁺ [M+H]⁺, calculated: 467.2839; found: 467.2836.

### Test example 1: Affinity of the compounds of the present disclosure for dopamine D₂ receptors

The affinity of the compounds of the present disclosure for the dopamine D₂ receptors was determined by the method of radioligand competition experiment. In the first step, a cell membrane component containing specific dopamine D₂ receptors was prepared. A 10 cm culture dish was used for transfection with 10 ng of the dopamine D₂ receptors and 40 µL of polyethylenimine (PEI hereafter). After 48 hours, the 10 cm culture dish was taken out from the cell culture incubator and the cultured cells had expressed the dopamine D₂ receptors. A vacuum pump was used to suck off the culture medium, 3 mL of lysis buffer was added to each culture dish, and the cells were placed in a 4°C cold room for 10 minutes. After the cells were detached, the cells were transferred to a 15 mL centrifuge tube and centrifuged at 1500 rpm for 5 minutes at 4°C, and the supernatant was discarded. The cell pellet was transferred to a tissue homogenizer, and 3 mL of lysis buffer was added and fully ground until the cells were broken. Then, cell suspension was equally aliquoted into several EP tubes, centrifuged at 12000 rpm for 5 minutes at 4°C, and the supernatant was discarded. The precipitate was the cell membrane component containing the dopamine D₂ receptors. In the second step, a ligandreceptor binding experiment was performed on 293T membrane component transiently expressing the dopamine D₂ receptors. First, a standard binding buffer was added to the cell membrane component containing the dopamine D₂ receptors, and the cell membrane was disrupted and resuspended with an electric tissue homogenizer. 30 µL of membrane protein suspension was added to each well of a 96-well plate. Then, 30 µL of different drugs were added to the 96-well plate sequentially from bottom to top to ensure that the final drug concentrations were 10⁻⁵ M, 10⁻⁶ M, 10⁻⁷ M, 10⁻⁸ M, 10⁻⁹ M, and 0 M, with two replicates per treatment. Next, 30 µL of [³H]-Methylspiperone was added to each well of the 96-well plate. The plate was incubated for 2 hours at room temperature in the dark. Detection was conducted. The machine readout value reflected the amount of [³H]-Methylspiperone bound on the membrane, and after further data processing, the affinity Kᵢ values of different compounds for the dopamine D₂ receptors were obtained.

The results are shown in Table 1.

**Table 1**

| Compound | D₂ affinity Kᵢ (µM) |
|---|---|
| I-1 | >10 |
| I-2 | 0.38 |
| I-3 | >10 |
| I-4 | 0.32 |
| I-5 | 7.50 |
| I-6 | 5.73 |
| I-7 | 0.31 |
| I-8 | 0.44 |
| I-9 | 0.80 |
| I-10 | 2.06 |
| I-11 | 0.47 |
| I-12 | 1.21 |
| I-14 | >10 |
| I-15 | 5.50 |
| I-16 | 1.55 |
| I-17 | 0.29 |
| I-18 | 0.27 |
| I-19 | 0.01 |
| I-20 | 0.03 |
| I-21 | 7.18 |
| I-22 | 0.49 |
| I-23 | 0.75 |
| I-24 | 0.23 |
| I-25 | 0.10 |
| I-26 | 0.09 |
| I-27 | 0.08 |
| (+)-I-27 | >10 |
| (-)-I-27 | 0.12 |
| I-28 | 0.08 |
| I-29 | 0.18 |
| Comparative compound 1 (Haloperidol) | 0.01 |

### Test example 2: Functional activity of the compounds for dopamine D₂ receptors

To examine the downstream G protein signaling pathway mediated by dopamine D₂ receptors, on the first day, a 6 cm culture dish was used for transfection with 1 µg of dopamine D₂ receptors, 1 µg of Gαᵢₗ containing a C-terminal seaweed luciferase (Gαᵢₗ-Rluc), 1 µg of G_{β3}, 1 µg of Gγ₉ containing C-terminal green fluorescent protein (Gγ₉- GFP), and 16 µL of PEI. At the same time, in order to examine the downstream β-arrestin2 signaling pathway mediated by dopamine D₂ receptors, on the first day, a 6 cm culture dish was used for transfection with 500 µg of dopamine D₂ receptors containing C-terminal seaweed luciferase (D₂-Rluc), 500 µg of G protein-coupled receptor kinase 2 (GRK2), 2500 µg of β-arrestin2 containing N-terminal green fluorescent protein (GFP2-ARRB2) mixed with 14 µL of PEI. The next day, the confluent cells of a 6 cm culture dish were digested and reseeded into a 96-well plate with 100 µL of culture medium added to each well. On the third day, the drugs were added for test. The 96-well plate was taken out from the cell incubator to decant the culture medium, 40 µL of the substrate coelenterazine 400a (final concentration of 5 µM) was added to each well, and then 20 µL of different drugs were added sequentially from left to right to ensure that the final concentration of the drug decreased gradually from bottom to top, with two replicates per treatment. Finally, the prepared samples were loaded on machine for test. The machine readout value reflected the recruitment of β-arrestin2 to the membrane or the dissociation of G protein trimer. The former indicated the degree of activation of the β-arrestin2 signaling pathway downstream of the dopamine D₂ receptors, and the latter indicated the degree of activation of the G protein signaling pathway downstream of the dopamine D₂ receptors. Thus, the agonistic effects of different compounds on dopamine D₂ receptors can be revealed. The results are shown in Table 2.

**Table 2**

| Compound | Gαᵢ₁ BRET EC¹₅₀, nM (Emax%) | β-arrestin2 BRET EC₅₀, nM (Emax%) |
|---|---|---|
| I-1 | 129.80 (42.35%) | 24.61 (28.79%) |
| I-2 | 746.80 (12.05%) | 19.28 (27.62%) |
| I-3 | 40.66 (22.54%) | 188.70 (14.23%) |
| I-4 | 36.26 (25.03%) | 11.17 (16.43%) |
| I-5 | 193.50 (53.84%) | 370.30 (27.25%) |
| I-6 | 342.50 (58.44%) | 299.00 (20.21%) |
| I-7 | N.D. | N.D. |
| I-8 | 530.60 (15.86%) | 370.90 (14.14%) |
| I-9 | 281.50 (23.15%) | 28.59 (24.25%) |
| I-10 | 795.70 (17.14%) | 14.31 (21.48%) |
| I-11 | N.D. | N.D. |
| I-12 | N.D. | N.D. |
| I-14 | N.D. | N.D. |
| I-15 | 334.10 (18.55%) | 164.00 (10.54%) |
| I-16 | 44.83 (57.89%) | 289.20 (37.72%) |
| I-17 | 18.55 (81.56%) | 91.56 (66.78%) |
| I-18 | 33.86 (91.30%) | 279.50 (71.91%) |
| I-19 | 8.26 (74.59%) | 43.91 (64.48%) |
| I-20 | 107.40 (63.98%) | 464.20 (35.61%) |
| I-21 | 145.60 (54.05%) | 233.10 (32.58%) |
| I-22 | 160.50 (36.99%) | 328.20 (24.79%) |
| I-23 | 281.80 (61.89%) | 129.60 (38.48%) |
| I-24 | 50.41 (61.63%) | 62.51 (35.19%) |
| I-25 | 27.85 (67.93%) | 61.47 (49.20%) |
| I-26 | 572.60 (47.90%) | 44.78 (19.77%) |
| I-27 | 3.87 (73.59%) | 10.17 (52.08%) |
| (+)-I-27 | 9.16 (69.54%) | 16.03 (45.78%) |
| (-)-I-27 | 3.76 (72.04%) | 10.64 (54.79%) |
| I-28 | 18.03 (30.68%) | 38.74 (34.19%) |
| I-29 | 32.19 (45.23%) | 102.10 (31.82%) |
| Comparative compound 2 (Quinpirole) | 3.73 (99.80%) | 20.17 (99.02%) |

In the tables of the examples, "N.D." or "ND" stands for "Not Detected".

### Test example 3: Affinity of compounds for 5-HT_{2A} receptors

The affinity of the compounds of the present disclosure for the 5-HT_{2A} receptors was determined by the method of radioligand competition experiment. In the first step, a cell membrane component containing specific 5-HT_{2A} receptor was prepared. A 10 cm dish was used for transfection with 10 ng of the 5-HT_{2A} receptor and 40 µL of PEI. After 48 hours, the 10 cm dish was taken out from the cell incubator and the cultured cells had expressed the 5-HT_{2A} receptor. A vacuum pump was used to suck off the culture medium, 3 mL of lysis buffer was added to each culture dish, and the cells were placed in a 4°C cold room for 10 minutes. After the cells were detached, the cells were transferred to a 15 mL centrifuge tube and centrifuged at 1500 rpm for 5 minutes at 4°C, and the supernatant was discarded. The cell pellet was transferred to a tissue homogenizer, and 3 mL of lysis buffer was added and fully ground until the cells were broken. Then, cell suspension was equally aliquoted into several EP tubes, centrifuged at 12000 rpm for 5 minutes at 4°C, and the supernatant was discarded. The precipitate was the cell membrane component containing the 5-HT_{2A} receptor. In the second step, a ligand-receptor binding experiment was performed on 293T membrane component transiently expressing the 5-HT_{2A} receptor. First, a standard binding buffer was added to the cell membrane component containing the 5-HT_{2A} receptor, and the cell membrane was disrupted and resuspended with an electric tissue homogenizer. 30 µL of membrane protein suspension was added to each well of a 96-well plate. Then, 30 µL of different drugs were added to the 96-well plate sequentially from bottom to top to ensure that the final drug concentrations were 10⁻⁵ M, 10⁻⁶ M, 10⁻⁷ M, 10⁻⁸ M, 10⁻⁹ M, and 0 M, with two replicates per treatment. Next, 30 µL of [³H]-ketanserin was added to each well of the 96-well plate. The plate was incubated for 2 hours at room temperature in the dark. Detection was conducted. The machine readout value reflected the amount of [³H]-ketanserin bound on the membrane, and after further data processing, the affinity Kᵢ values of different compounds for the 5-HT_{2A} receptor were obtained. The results are shown in Table 3.

**Table 3**

| Compound | 5-HT_{2A} affinity Kᵢ (µM) |
|---|---|
| I-1 | 0.02 |
| I-2 | 0.71 |
| I-3 | 4.13 |
| I-4 | 4.41 |
| I-5 | 0.92 |
| I-6 | 7.25 |
| I-7 | 0.91 |
| I-8 | 1.46 |
| I-9 | 0.69 |
| I-10 | 1.49 |
| I-11 | 0.32 |
| I-12 | 1.52 |
| I-13 | N.D. |
| I-14 | 1.19 |
| I-15 | 1.71 |
| I-16 | >10 |
| I-17 | 0.75 |
| I-18 | 0.16 |
| I-19 | 1.41 |
| I-20 | 0.64 |
| I-21 | >10 |
| I-22 | 1.20 |
| I-23 | 0.16 |
| I-24 | 0.63 |
| I-25 | 0.30 |
| I-26 | 0.03 |
| I-27 | 0.21 |
| (+)-I-27 | 0.45 |
| (-)-I-27 | 2.29 |
| I-28 | 0.52 |
| I-29 | 0.45 |
| Comparative compound 3 5-HT | 0.02 |

The structure of 5-HT in the example is

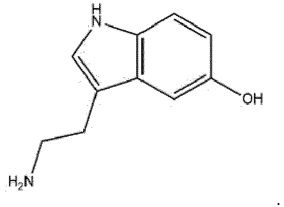

### Test example 4: Functional activity of compounds for 5-HT_{2A} receptors

To examine the downstream G protein signaling pathway mediated by 5-HT_{2A} receptors, on the first day, a 6 cm culture dish was used for transfection with 1 µg of 5-HT_{2A} receptors, 1 µg of Gα_{q} containing C-terminal seaweed luciferase (Gα_{q}-Rluc), 1 µg of G_{β3}, 1 µg of Gγ₉ containing C-terminal green fluorescent protein (G_{γ9}-GFP), and 16 µL of PEI. At the same time, in order to examine the downstream β-arrestin2 signaling pathway mediated by 5-HT_{2A} receptors, on the first day, a 6 cm culture dish was used for transfection with 500 µg of 5-HT_{2A} receptors containing C-terminal seaweed luciferase (5-HT_{2A}-Rluc), 500 µg of G protein-coupled receptor kinase 2 (GRK2), 2500 µg of β-arrestin2 containing N-terminal green fluorescent protein (GFP2-ARRB2), and 14 µL of PEI. The next day, the confluent cells of a 6 cm culture dish were digested and reseeded into a 96-well plate with 100 µL of culture medium was added to each well. On the third day, the drugs were added for test. The 96-well plate was taken out from the cell incubator to decant the culture medium, 40 µL of the substrate coelenterazine 400a (final concentration of 5 µM) was added to each well, and then 20 µL of different drugs were added sequentially from left to right to ensure that the final concentration of the drug decreased gradually from bottom to top, with two replicates per treatment. Finally, the prepared samples were loaded on machine for test. The machine readout value reflected the recruitment of β-arrestin2 to the membrane or the dissociation of G protein trimer. The former indicated the degree of activation of the β-arrestin2 signaling pathway downstream of the 5-HT_{2A} receptors, and the latter indicated the degree of activation of the G protein signaling pathway downstream of the 5-HT_{2A} receptors. Thus, the agonistic effects of different compounds on 5-HT_{2A} receptors can be revealed. The results are shown in Table 4.

**Table 4**

| Compound | Gα_{q} BRET EC¹₅₀, nM (Emax%) | β-arrestin2 BRET EC₅₀, nM (Emax%) |
|---|---|---|
| I-1 | 23.43 (81.63%) | 39.12 (79.00%) |
| I-2 | 2.41 (7.60%) | 298.20 (66.30%) |
| I-3 | 1521.00 (43.57%) | 638.50 (89.97%) |
| I-4 | 1377.00 (50.00%) | 3939.00 (34.10%) |
| I-5 | 1097.32 (50.00%) | 946.00 (27.30%) |
| I-6 | N.D. | 8341.00 (45.40%) |
| I-7 | 140.00 (36.90%) | N.D. |
| I-8 | 4537.00 (20.99%) | 1288.00 (84.15%) |
| I-9 | 746.70 (25.00%) | N.D. |
| I-10 | 14.19 (110.00%) | 393.80 (71.60%) |
| I-11 | N.D. | N.D. |
| I-12 | N.D. | 5244.00 (5.70%) |
| I-14 | 475.10 (29.10%) | 123.20 (21.00%) |
| I-15 | N.D. | 1766.00 (10.00%) |
| I-16 | 340.30 (19.46%) | N.D. |
| I-17 | 279.30 (23.41%) | 1270.00 (92.15%) |
| I-18 | N.D. | N.D. |
| I-19 | 1442.00 (15.55%) | N.D. |
| I-20 | 5590.00 (17.76%) | N.D. |
| I-21 | 4380.00 (16.56%) | N.D. |
| I-22 | 966.10 (13.83%) | 3473.00 (64.59%) |
| I-23 | 245.30 (18.90%) | 8065.00 (138.3%) |
| I-24 | 345.30 (11.61%) | N.D. |
| I-25 | 968.90 (17.83%) | N.D. |
| I-26 | 339.20 (26.03%) | N.D. |
| I-27 | 157.70 (9.57%) | 13.76 (34.67%) |
| (+)-I-27 | N.D. | N.D. |
| (-)-I-27 | N.D. | N.D. |
| I-28 | 183.60 (12.47%) | N.D. |
| I-29 | 299.20 (8.61%) | 30.19 (21.76%) |
| 5-HT | 22.01 (100%) | 21.47 (99.46%) |

### Test example 5: Functional activity and affinity of compounds for 5-HT_{1A} receptors

The affinity of the compounds of the present disclosure for the 5-HT_{1A} receptors was determined by the method of radioligand competition experiment. In the first step, a cell membrane component containing specific 5-HT_{1A} receptor was prepared. A 10 cm culture dish was used for transfection with 10 ng of the 5-HT_{1A} receptor and 40 µL of PEI. After 48 hours, the 10 cm culture dish was taken out from the cell incubator and the cultured cells had expressed the 5-HT_{1A} receptor. A vacuum pump was used to suck off the culture medium, 3 mL of lysis buffer was added to each culture dish, and the cells were placed in a 4°C cold room for 10 minutes. After the cells were detached, the cells were transferred to a 15 mL centrifuge tube and centrifuged at 1500 rpm for 5 minutes at 4°C, and the supernatant was discarded. The cell pellet was transferred to a tissue homogenizer, and 3 mL of lysis buffer was added and fully ground until the cells were broken. Then, cell suspension was equally aliquoted into several EP tubes, centrifuged at 12000 rpm for 5 minutes at 4°C, and the supernatant was discarded. The precipitate was the cell membrane component containing the 5-HT_{1A} receptor. In the second step, a ligand-receptor binding experiment was performed on 293T membrane component transiently expressing the 5-HT_{1A} receptor. First, a standard binding buffer was added to the cell membrane component containing the 5-HT_{1A} receptor, and the cell membrane was disrupted and resuspended with an electric tissue homogenizer. 30 µL of membrane protein suspension was added to each well of a 96-well plate. Then, 30 µL of different drugs were added to the 96-well plate sequentially from bottom to top to ensure that the final drug concentrations were 10⁻⁵ M, 10⁻⁶ M, 10⁻⁷ M, 10⁻⁸ M, 10⁻⁹ M, and 0 M, with two replicates per treatment. Next, 30 µL of [³H]-LSD was added to each well of the 96-well plate. The plate was incubated for 2 hours at room temperature in the dark. Detection was conducted. The machine readout value reflected the amount of [³H]-LSD bound on the membrane, and after further data processing, the affinity Kᵢ values of different compounds for the 5-HT_{1A} receptor were obtained. The results are shown in Table 5. To examine the downstream G protein signaling pathway mediated by 5-HT_{1A} receptors, on the first day, a 6 cm culture dish was used for transfection with 1 µg of 5-HT_{1A} receptors, 1 µg of Gαᵢ₁ containing C-terminal seaweed luciferase (Gαᵢ₁-Rluc), 1 µg of G_{β3}, 1 µg of Gγ₉ containing C-terminal green fluorescent protein (G_{γ9}-GFP), and 16 µL of PEI. The next day, the confluent cells of a 6 cm culture dish were digested and reseeded into a 96-well plate with 100 µL of culture medium was added to each well. On the third day, the drugs were added for test. The 96-well plate was taken out from the cell incubator to decant the culture medium, 40 µL of the substrate coelenterazine 400a (final concentration of 5 µM) was added to each well, and then 20 µL of different drugs were added sequentially from left to right to ensure that the final concentration of the drug decreased gradually from bottom to top, with two replicates per treatment. Finally, the prepared samples were loaded on machine for test. The machine readout value reflected the dissociation of G protein trimer, and thereby indicated the degree of activation of the G protein signaling pathway downstream of the 5-HT_{1A} receptors. Thus, the agonistic effects of different compounds on 5-HT_{1A} receptors can be revealed. The results are shown in Table 5.

**Table 5**

| Compound | 5-HT_{1A} affinity Kᵢ, µM | Gαᵢ₁ BRET EC¹₅₀, nM (Emax%) |
|---|---|---|
| I-1 | 0.14 | 1055.60 (86%) |
| I-2 | 3.42 | 7722.36 (72%) |
| I-3 | 4.41 | 24182.43 (62%) |
| I-4 | 0.59 | 12764.39 (69%) |
| I-5 | 0.28 | 3126.08 (91%) |
| I-6 | 2.73 | 3228.49 (90%) |
| I-7 | 39.31 | N.D. |
| I-8 | 9.85 | 16302.34 (53%) |
| I-9 | 5.46 | 14446.08 (55%) |
| I-10 | 0.97 | 7198.63 (71%) |
| I-11 | 4.54 | 3865.89 (28%) |
| I-12 | 0.59 | 1324.34 (93%) |
| I-13 | N.D. | N.D. |
| I-14 | 1.30 | 1996.41 (86%) |
| I-15 | 0.84 | 5260.17 (57%) |
| I-16 | 0.56 | 2716.44 (88%) |
| I-17 | 0.34 | 753.78 (86%) |
| I-18 | 0.01 | 695.60 (82%) |
| I-19 | 0.03 | 3674.94 (81%) |
| I-20 | 0.16 | 1536.38 (72%) |
| I-21 | 3.22 | 8175.23 (68%) |
| I-22 | 0.35 | 1468.93 (87%) |
| I-23 | 4.13 | 6702.70 (71%) |
| I-24 | 0.53 | 946.69 (79%) |
| I-25 | 0.77 | 1319.78 (82%) |
| I-26 | 0.40 | 488.93 (88%) |
| I-27 | 0.16 | 163.96 (64%) |
| (+)-I-27 | 0.17 | 205.83 (60%) |
| (-)-I-27 | 0.08 | 166.15 (76%) |
| I-28 | 0.05 | 142.07 (59%) |
| I-29 | 0.12 | 1477.41 (62%) |
| 5-HT | 0.01 | 6.21 (100%) |

### Test example 6: Pharmacodynamic test of compounds on schizophrenia-like animal behavioral model

### 1. Open field test

Experimental method: The experimental animals were C57B6 male mice, n = 8 in each group. This model used C57B6 mice as experimental animals. Hyperlocomotion of the mice in open field was induced by acute injection of a NMDA antagonist MK801. and modeled to test the inhibitory effect of different compounds on hyperkinesia phenotype induced by MK801. All mouse behavior experiments were carried out during the light period, and the whole process was recorded by the camera and locomotive data were tracked and analyzed by behavioral tracking software. The compounds were administered intraperitoneally. Immediately after the injection, the mice entered the open field and their movement trajectories were recorded by the camera. After 30 minutes, the mice received 0.2 mg/kg of MK801 by intraperitoneal injection and returned to the open field immediately after administration and the movement trajectories were further recorded for 120 minutes. The accumulative travelled distance of the mice was counted in a 5-minute bin. Data statistics were performed using Student-t-test analysis, *p < 0.05, **p < 0.01, ***p < 0.001, ****p < 0.0001. The results of the open field test showed that compound (-)-I-27 at 0.4/0.2/0.1 mg/kg could significantly inhibit hyperlocomotion of the mice induced by MK801.

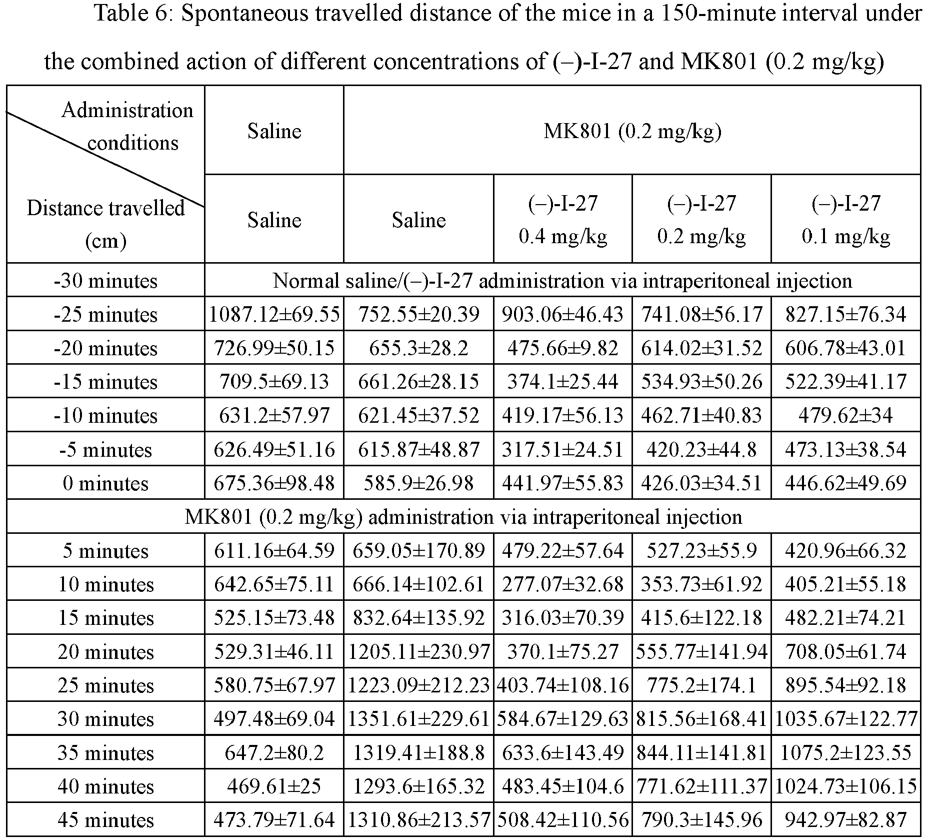

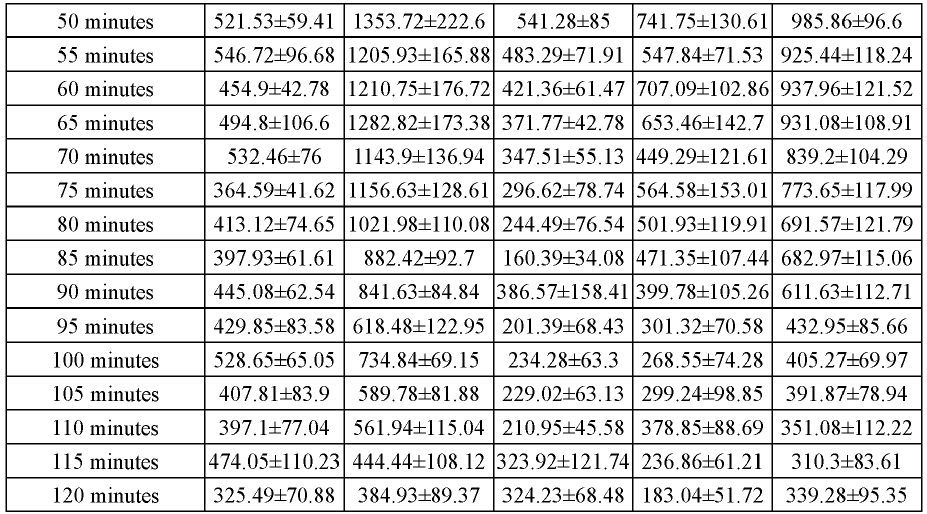

**Table 7: Total travelled distance of the mice in the 0-45 minute interval after MK-801 administration under the combined action of different concentrations of (-)-I-27 and MK801 (0.2 mg/kg)**

| Administration conditions | Saline | MK801 (0.2 mg/kg) | | | |
|---|---|---|---|---|---|
| | Saline | Saline | (-)-1-27 0.4 mg/kg | (-)-1-27 0.2 mg/kg | (-)-1-27 0.1 mg/kg |
| Distance (cm) travelled in 45 minutes | 4977.09±413.12 | 8904.49±1080.00 | 4056.3±773.61 | 5849.12±990.80 | 6882.3±616.06 |

### 2. Animal "depression-like" behavioral test

The experimental animals were C57B6 male mice, n = 8 in each group. 5-hour restraint were used to develop depression-like behavioral representations of the mice, then the effects of the compounds on their "depression-like" behaviors were measured according to behavioral performance in tail suspension test and forced swimming test. The specific experimental procedures were as follows: Firstly, the mice were restrained using a mouse fixator for tail vein injection. During the restraint process, all movement abilities of the mice were restricted under the condition of introducing minimal pain to the mice. Compounds were administered intraperitoneally twice, before and after restraint, respectively. After compound administration, the mice returned to the cage to recover for 30 minutes. After 30 minutes recovery, the mice in different groups were subjected to tail suspension test or forced swimming test to measure their "depression-like" behaviors.

Tail suspension test: mouse tails were fixed a cantilever arm on an iron rack using adhesive tape, and the mouse was maintained in a suspension position for 6 minutes. The first 2 minutes were the adaptation period and no data collection was performed. Subsequently, the mouse's immobility behavior was recorded intermittently for the remaining 4 minutes. The duration of immobility was measured to assess the level of behavioral despair in the mice. Data statistics were performed using Student-t-test analysis, *p < 0.05, **p < 0.01, ***p < 0.001, ****p < 0.0001.

Forced swimming test: the mice were placed in a 5 L glass beaker filled with water, with the water level at 15 cm height, and the mice were forced to swim continuously in the beaker for 6 minutes. The first 2 minutes were the adaptation period and no data collection was performed. The duration of the immobility behavior was recorded intermittently for the remaining 4 minutes, and the immobility behavior of the mice was defined as: the mice float passively on the surface of the water without movement, with only small local movements required to maintain afloat. The duration of this immobility was measured to assess the level of behavioral despair in the mice. Data statistics were performed using Student-t-test analysis, *p < 0.05, **p < 0.01, ***p < 0.001, ****p < 0.0001. The results of the "depression-like" behavioral tests showed that compound (-)-I-27 at 0.1 mg/kg and 0.025 mg/kg could significantly inhibit the restraint-induced "depression-like" behaviors.

**Table 8: Statistical data of restrained and unrestrained mouse tail suspension test and forced swimming test**

| Treatment conditions | | Naïve | 5 hour restraint | | | |
|---|---|---|---|---|---|---|
| | | Saline | Saline | **(-)-I-27** 0.1 mg/kg | **(-)-I-27** 0.025 mg/kg | **(-)-I-27** 0.00625 mg/kg |
| Immobility time (seconds) | Tail suspension test | 52.17±5.06 | 126.5±3.54 | 54.13±6.99 | 87.04±7.69 | 125.81±5.69 |
| | Forced swimming test | 53.44±7.18 | 119.37±4.06 | 56.89±4.80 | 91.83±6.80 | 120.57±4.21 |

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

1. A compound of formula I, a pharmaceutically acceptable salt thereof, a solvate thereof, or a solvate of the pharmaceutically acceptable salt thereof: 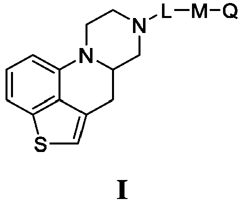
wherein L is absent, C₁₋₁₀ alkylene, C₂₋₁₀ alkenylene, C₂₋₁₀ alkynylene, or -C₁₋₆ alkylene-C₃₋₆ cycloalkylene-;
M is absent, -O-, -NH-, -CH₂-, -(CH-OH)-, or -C(=O)-;
Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, 5- to 12-membered heteroaryl substituted by one or more than one Q¹⁻², or - C(=O)R¹; the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3;
Q¹⁻¹ is halogen, C₁₋₄ alkyl, C₁₋₄ alkoxy, C₆₋₁₈ aryl, 5- to 12-membered heterocycloalkyl, 5-to 12-membered oxo-heterocycloalkyl, hydroxyl, or C₆₋₁₈ aryl substituted by one or more than one Q¹⁻¹⁻¹; the heteroatoms in the 5- to 12-membered heterocycloalkyl are one or more than one of N, S, or O, and the number is 1, 2, or 3;
Q¹⁻² is halogen, oxo, C₁₋₄ alkyl, C₁₋₄ alkoxy, 5- to 12-membered heterocycloalkyl, C₆₋₁₈ aryl substituted by one or more than one Q¹⁻²⁻¹, C₆₋₁₈ aryl, or hydroxyl; the heteroatoms in the 5- to 12-membered heterocycloalkyl are one or more than one of N, S, or O, and the number is 1, 2, or 3;
R¹ is -NR¹⁻¹R¹⁻², 3- to 6-membered heterocycloalkyl, C₆₋₁₈ aryl, 5- to 12-membered heteroaryl, 5- to 12-membered heteroaryl substituted by one or more than one R¹⁻⁴, or C₆₋₁₈ aryl substituted by one or more than one R¹⁻³; the heteroatoms in the 3- to 6-membered heterocycloalkyl are one or more than one of N, S, or O, and the number is 1, 2, or 3; the heteroatoms in the 5- to 12-membered heteroaryl are one or more than one of N, S, or O, and the number is 1, 2, or 3;
R¹⁻¹ and R¹⁻² are independently hydrogen or C₁₋₄ alkyl;
R¹⁻³, Q¹⁻¹⁻¹, and Q¹⁻²⁻¹ are independently halogen;
R¹⁻⁴ is halogen or oxo.

2. The compound of formula I, the pharmaceutically acceptable salt thereof, the solvate thereof, or the solvate of the pharmaceutically acceptable salt thereof according to claim 1, wherein in L, the C₁₋₁₀ alkylene is C₁₋₄ alkylene, such as methylene, 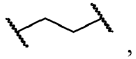 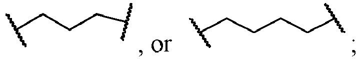
and/or, in L, in the -C₁₋₆ alkylene-C₃₋₆ cycloalkylene-, the C₁₋₆ alkylene is connected to N, and the C₃₋₆ cycloalkylene is connected to Q;
and/or, in L, in the -C₁₋₆ alkylene-C₃₋₆ cycloalkylene-, the C₁₋₆ alkylene is C₁₋₄ alkylene, such as methylene or ethylene;
and/or, in L, in the -C₁₋₆ alkylene-C₃₋₆ cycloalkylene-, the C₃₋₆ cycloalkylene is cyclopropylene, cyclobutylene, cyclopentylene, or cyclohexylene, such as cyclopropylene or cyclohexylene; furthermore, the -C₁₋₆ alkylene-C₃₋₆ cycloalkylene is -methylene-cyclopropylene- or -ethylene-cyclohexylene-, such as 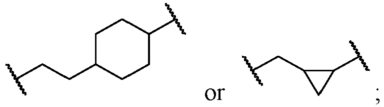 and/or, in Q, the C₆₋₁₈ aryl is C₆₋₁₀ aryl, such as phenyl or naphthyl;
and/or, in Q, the C₆₋₁₈ aryl, as described in the C₆₋₁₈ aryl substituted by one or more than one Q¹⁻¹, is C₆₋₁₀ aryl; further preferably, the C₆₋₁₈ aryl substituted by one or more than one Q¹⁻¹ is phenyl substituted by fluorine or phenyl substituted by imidazolidinone, such as 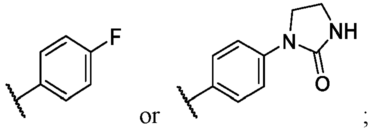
and/or, in Q, the 5- to 12-membered heteroaryl has 1 or 2 heteroatoms; further preferably, the 5- to 12-membered heteroaryl is thienyl, pyridyl, benzothiazolyl, or benzodioxolane, such as 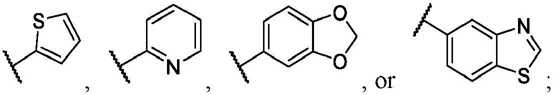
and/or, in Q, the 5- to 12-membered heteroaryl, as described in the 5- to 12-membered heteroaryl substituted by one or more than one Q¹⁻², is pyridyl, tetrahydro-benzazepine, dihydroisoquinolinyl, or indolyl; further preferably, the 5- to 12-membered heteroaryl substituted by one or more than one Q¹⁻²⁻¹ is pyridyl substituted by fluorine-substituted phenyl, indolyl substituted by methoxy, indolyl substituted by oxo, dihydroisoquinolinyl substituted by oxo, or tetrahydro-benzazepine substituted by oxo, such as 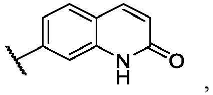 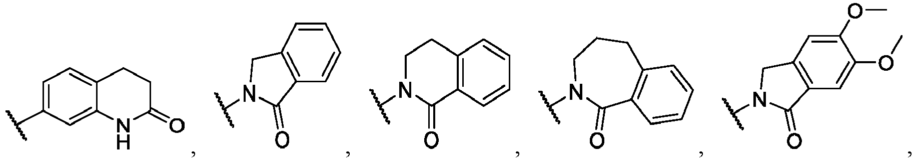 or 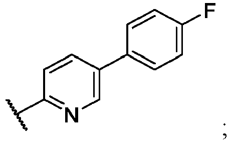
and/or, in Q, the 5- to 12-membered heterocycloalkyl is piperidinyl, pyrrolidinyl, imidazolidinyl, or hexahydroisoindolyl, such as 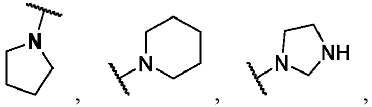 or 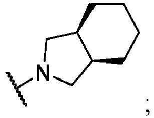
and/or, in Q, the 5- to 12-membered oxo-heterocycloalkyl is oxo-piperidinyl, oxo-pyrrolidinyl, oxo-imidazolidinyl, or oxo-hexahydroisoindolyl; the 5- to 12-membered oxo-heterocycloalkyl is 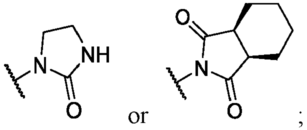
and/or, in Q¹⁻¹, the halogen is F, Cl, Br, or I, such as F;
and/or, in Q¹⁻¹, the C₁₋₄ alkyl is methyl, ethyl, *n*-propyl, isopropyl, *n*-butyl, isobutyl, *sec-*butyl, or *tert*-butyl, such as methyl or ethyl;
and/or, in Q¹⁻¹, the C₁₋₄ alkoxy is methoxy, ethoxy, *n*-propoxy, isopropoxy, *n*-butoxy, isobutoxy, *sec*-butoxy, or *tert*-butoxy, such as methoxy or ethoxy;
and/or, in Q¹⁻¹, the 5- to 12-membered heterocycloalkyl is imidazolidinyl, such as 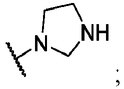
and/or, in Q¹⁻¹, the 5- to 12-membered oxo-heterocycloalkyl is oxo-imidazolidinyl, such as 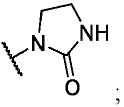
and/or, in Q¹⁻¹, the C₆₋₁₈ aryl is C₆₋₁₀ aryl, such as phenyl or naphthyl;
and/or, in Q¹⁻¹, the C₆₋₁₈ aryl, as described in the C₆₋₁₈ aryl substituted by one or more than one Q¹⁻¹⁻¹, is C₆₋₁₀ aryl; further preferably, the C₆₋₁₈ aryl substituted by one or more than one Q¹⁻¹⁻¹ is phenyl substituted by fluorine, such as 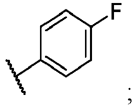
and/or, in Q¹⁻², the C₁₋₄ alkyl is methyl, ethyl, *n*-propyl, isopropyl, *n*-butyl, isobutyl, *sec-*butyl, or *tert*-butyl, such as methyl or ethyl;
and/or, in Q¹⁻², the C₁₋₄ alkoxy is methoxy, ethoxy, *n*-propoxy, isopropoxy, *n*-butoxy, isobutoxy, *sec*-butoxy, or *tert*-butoxy, such as methoxy or ethoxy;
and/or, in Q¹⁻², the C₆₋₁₈ aryl is C₆₋₁₀ aryl, such as phenyl or naphthyl;
and/or, in Q¹⁻², the C₆₋₁₈ aryl, as described in the C₆₋₁₈ aryl substituted by one or more than one Q¹⁻²⁻¹, is C₆₋₁₀ aryl; further preferably, the C₆₋₁₈ aryl substituted by one or more than one Q¹⁻²⁻¹ is phenyl substituted by fluorine, such as 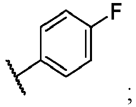
and/or, in R¹, the 3- to 6-membered heterocycloalkyl is piperidinyl, tetrahydropyranyl, or pyrrolidinyl, such as 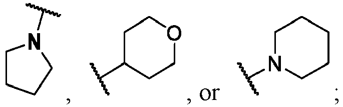
and/or, in R¹, the C₆₋₁₈ aryl is C₆₋₁₀ aryl, such as phenyl or naphthyl;
and/or, in R¹, the C₆₋₁₈ aryl, as described in the C₆₋₁₈ aryl substituted by one or more than one R¹⁻³, is C₆₋₁₀ aryl; further preferably, the C₆₋₁₈ aryl substituted by one or more than one R¹⁻³ is phenyl substituted by fluorine, such as 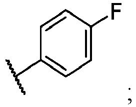
and/or, in R¹⁻¹, the C₁₋₄ alkyl is methyl, ethyl, *n*-propyl, isopropyl, *n*-butyl, isobutyl, *sec-*butyl, or *tert*-butyl, such as methyl or ethyl;
and/or, in R¹⁻², the C₁₋₄ alkyl is methyl, ethyl, *n*-propyl, isopropyl, *n*-butyl, isobutyl, *sec-*butyl, or *tert*-butyl, such as methyl or ethyl;
and/or, in R¹⁻³, the halogen is F, Cl, Br, or I, such as F;
and/or, in Q¹⁻¹⁻¹, the halogen is F, Cl, Br, or I, such as F;
and/or, in Q¹⁻²⁻¹, the halogen is F, Cl, Br, or I, such as F;
and/or, in R¹⁻⁴, the halogen is F, Cl, Br, or I, such as F.

3. The compound of formula I, the pharmaceutically acceptable salt thereof, the solvate thereof, or the solvate of the pharmaceutically acceptable salt thereof according to claim 1, wherein L is C₁₋₁₀ alkylene or -C₁₋₆ alkylene-C₃₋₆ cycloalkylene-;
and/or, M is absent, -O-, -NH-, -CH₂-, or -C(=O)-;
and/or, Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, 5- to 12-membered heteroaryl substituted by one or more than one Q¹⁻², or - C(=O)R¹; the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3;
and/or, Q¹⁻¹ is halogen, C₁₋₄ alkyl, C₆₋₁₈ aryl, 5- to 12-membered heterocycloalkyl, 5- to 12-membered oxo-heterocycloalkyl, or C₆₋₁₈ aryl substituted by one or more than one Q¹⁻¹⁻¹; the heteroatoms in the 5- to 12-membered heterocycloalkyl are one or more than one of N, S, or O, and the number is 1, 2, or 3;
and/or, Q¹⁻² is C₁₋₄ alkyl, C₁₋₄ alkoxy, C₆₋₁₈ aryl substituted by one or more than one Q¹⁻²⁻¹, or C₆₋₁₈ aryl;
and/or, R¹ is -NR¹⁻¹R¹⁻², 3- to 6-membered heterocycloalkyl, C₆₋₁₈ aryl, or C₆₋₁₈ aryl substituted by one R¹⁻³; the heteroatom in the 3- to 6-membered heterocycloalkyl is N, and the number is 1.

4. The compound of formula I, the pharmaceutically acceptable salt thereof, the solvate thereof, or the solvate of the pharmaceutically acceptable salt thereof according to any one of claims 1 to 3, wherein the compound of formula I is scheme i or scheme ii:
scheme i:
the compound of formula I is a compound of formula Ie, a compound of formula If, or a mixture thereof; 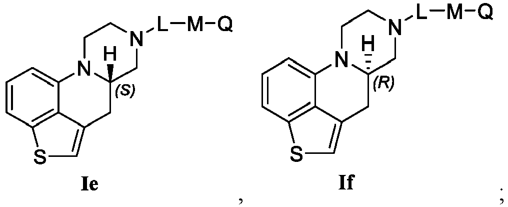
scheme i:
the compound of formula I is a compound of formula Ia, Ib, Ic, or Id:

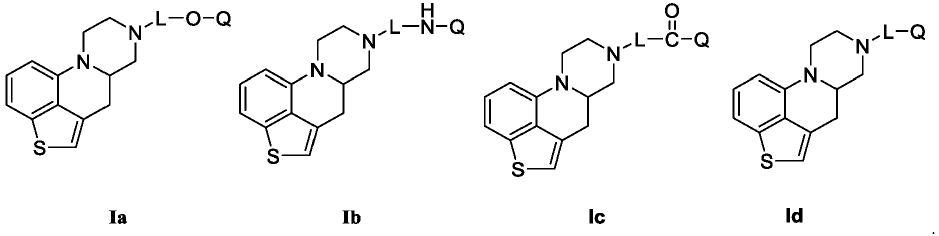

5. The compound of formula I, the pharmaceutically acceptable salt thereof, the solvate thereof, or the solvate of the pharmaceutically acceptable salt thereof according to any one of claims 1 to 3, wherein the compound of formula I is any one of the following schemes:
scheme 1: the compound of formula I is a compound of formula Ia: 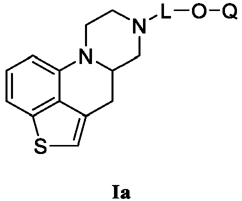
L is C₁₋₁₀ alkylene;
Q is hydrogen, C₆₋₁₈ aryl, or 5- to 12-membered heteroaryl; the heteroatoms in the 5- to 12-membered heteroaryl are one or more than one of N, S, or O, and the number is 1, 2, or 3;
scheme 2: the molecular structure of formula I is as shown in formula Ib: 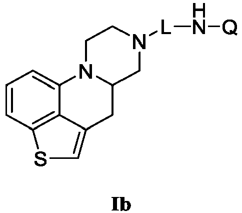
L is C₁₋₁₀ alkylene or -C₁₋₆ alkylene-C₃₋₆ cycloalkylene-;
Q is -C(=O)R¹;
R¹ is -NR¹⁻¹R¹⁻², 3- to 6-membered heterocycloalkyl, C₆₋₁₈ aryl, 5- to 12-membered heteroaryl, 5- to 12-membered heteroaryl substituted by one or more than one R¹⁻⁴, or C₆₋₁₈ aryl substituted by one or more than one R¹⁻³; the heteroatoms in the 3- to 6-membered heterocycloalkyl are one or more than one of N, S, or O, and the number is 1, 2, or 3; the heteroatoms in the 5- to 12-membered heteroaryl are one or more than one of N, S, or O, and the number is 1, 2, or 3; the heteroatoms in the 5- to 12-membered heteroaryl substituted by one or more than one R¹⁻⁴ are one or more than one of N, S, or O, and the number is 1, 2, or 3;
R¹⁻¹ and R¹⁻² are independently C₁₋₄ alkyl;
R¹⁻³ and R¹⁻⁴ are independently halogen;
scheme 3: the molecular structure of formula I is as shown in formula Ic: 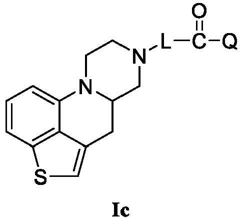
L is C₁₋₁₀ alkylene;
Q is C₆₋₁₈ aryl or C₆₋₁₈ aryl substituted by one or more than one Q¹⁻¹, Q¹⁻¹ is halogen;
scheme 4: the molecular structure of formula I is as shown in formula Id: 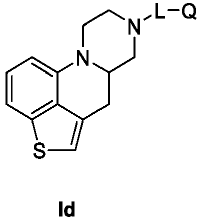
L is absent, C₁₋₁₀ alkylene, or -C₁₋₆ alkylene-C₃₋₆ cycloalkylene-;
Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, or 5- to 12-membered heteroaryl substituted by one or more than one Q¹⁻²; the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3;
Q¹⁻¹ is halogen, C₁₋₄ alkyl, C₁₋₄ alkoxy, C₆₋₁₈ aryl, 5- to 12-membered oxo-heterocycloalkyl, 5- to 12-membered heterocycloalkyl, or C₆₋₁₈ aryl substituted by one or more than one Q¹⁻¹⁻¹; the heteroatoms in the 5- to 12-membered heterocycloalkyl are one or more than one of N, S, or O, and the number is 1, 2, or 3;
Q¹⁻² is halogen, C₁₋₄ alkyl, C₁₋₄ alkoxy, 5- to 12-membered heterocycloalkyl, C₆₋₁₈ aryl substituted by one or more than one Q¹⁻²⁻¹, C₆₋₁₈ aryl, or hydroxyl; the heteroatoms in the 5- to 12-membered heterocycloalkyl are one or more than one of N, S, or O, and the number is 1, 2, or 3;
Q¹⁻¹⁻¹ and Q¹⁻²⁻¹ are independently halogen;
scheme 5: L is C₁₋₁₀ alkylene or -C₁₋₆ alkylene-C₃₋₆ cycloalkylene-;
M is absent, -O-, -NH-, -CH₂-, or -C(=O)-;
Q is hydrogen, C₆₋₁₈ aryl, C₆₋₁₈ aryl substituted by one or more than one Q¹⁻¹, 5- to 12-membered heteroaryl, 5- to 12-membered heterocycloalkyl, 5- to 12-membered oxo-heterocycloalkyl, 5- to 12-membered heteroaryl substituted by one or more than one Q¹⁻², or - C(=O)R¹; the heteroatoms in the 5- to 12-membered heteroaryl are one or more than one of N, S, or O, and the number is 1, 2, or 3; the heteroatoms in the 5- to 12-membered heterocycloalkyl are one or more than one of N, S, or O, and the number is 1, 2, or 3;
Q¹⁻¹ is halogen, C₁₋₄ alkyl, C₆₋₁₈ aryl, 5- to 12-membered heterocycloalkyl, 5- to 12-membered oxo-heterocycloalkyl, or C₆₋₁₈ aryl substituted by one or more than one Q¹⁻¹⁻¹; the heteroatoms in the 5- to 12-membered heterocycloalkyl are one or more than one of N, S, or O, and the number is 1, 2, or 3;
Q¹⁻² is C₁₋₄ alkyl, C₁₋₄ alkoxy, C₆₋₁₈ aryl substituted by one or more than one Q¹⁻²⁻¹, or C₆₋₁₈ aryl;
R₁ is -NR¹⁻¹R¹⁻², 3- to 6-membered heterocycloalkyl, C₆₋₁₈ aryl, or C₆₋₁₈ aryl substituted by one R¹⁻³; the heteroatom in the 3- to 6-membered heterocycloalkyl is N, and the number is 1;
R¹⁻¹ and R¹⁻² are independently C₁₋₄ alkyl;
R¹⁻³, Q^{1-1-1,} and Q¹⁻²⁻¹ are independently halogen;
R¹⁻⁴ is halogen or oxo.

6. The compound of formula I, the pharmaceutically acceptable salt thereof, the solvate thereof, or the solvate of the pharmaceutically acceptable salt thereof according to claim 5, wherein L is C₁₋₄ alkylene or -C₁₋₄ alkylene-C₃₋₆ cycloalkylene;
and/or, M is absent, -O-, -NH-, -CH₂-, or -C(=O)-;
preferably, -L-M- is absent, 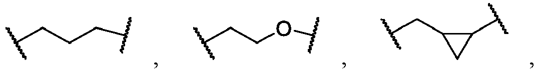 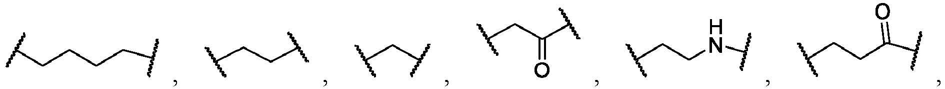 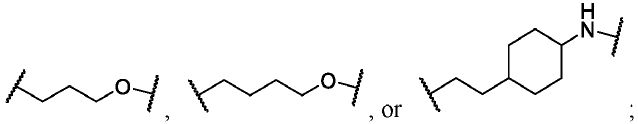
preferably, Q is hydrogen, hydroxyl, cyclopropyl,

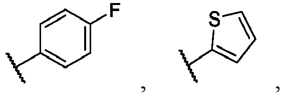

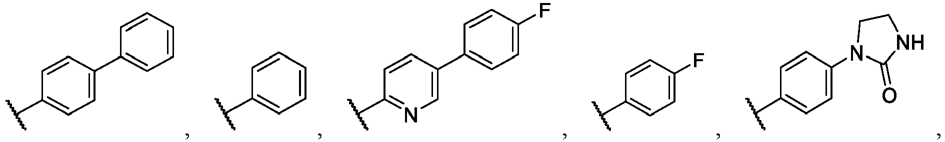

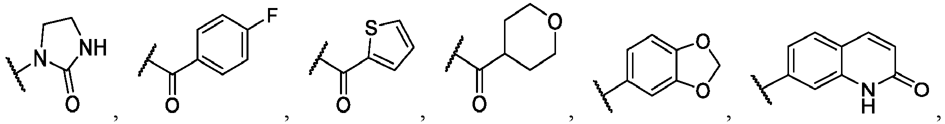

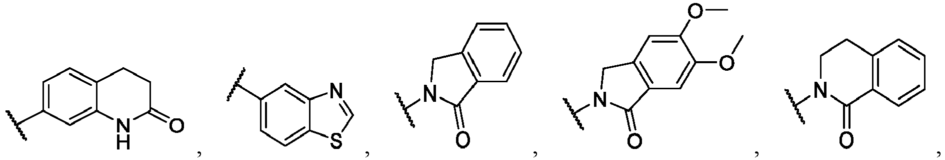

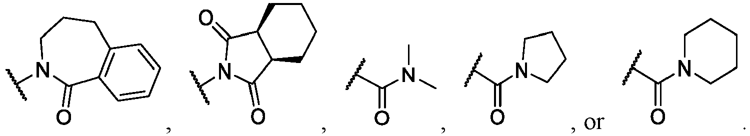

7. The compound of formula I, the pharmaceutically acceptable salt thereof, the solvate thereof, or the solvate of the pharmaceutically acceptable salt thereof according to claim 1, wherein the compound of formula I is selected from any one of the following compounds: 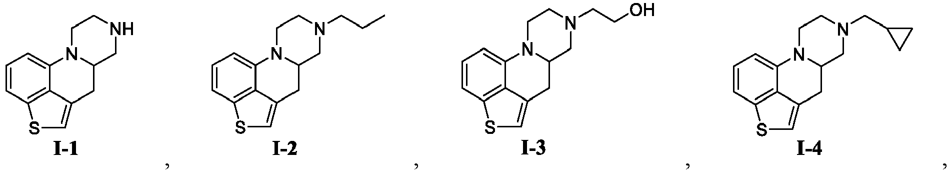 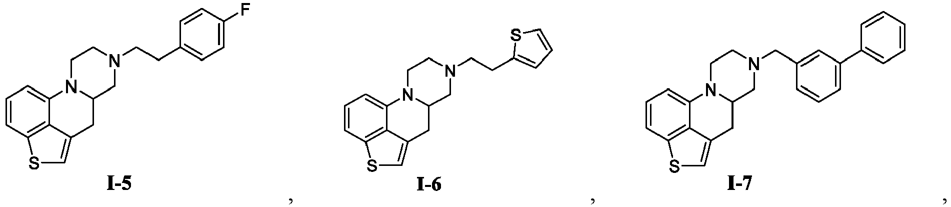 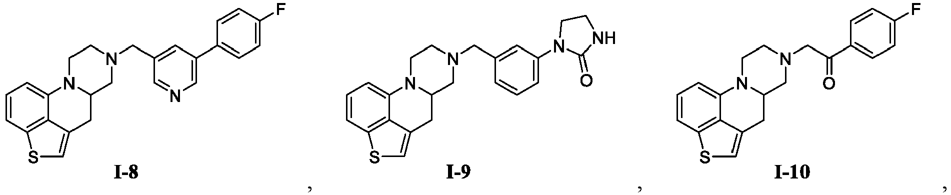 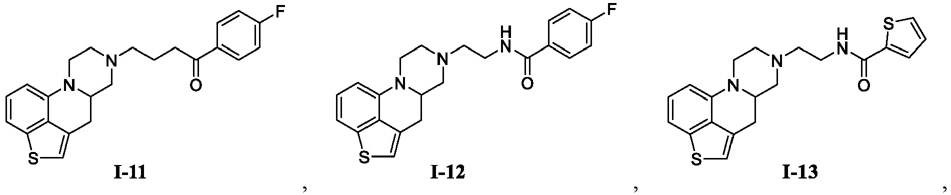 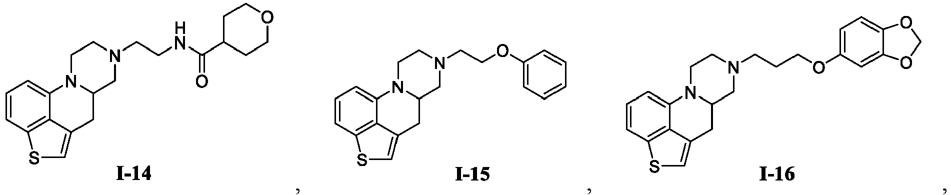 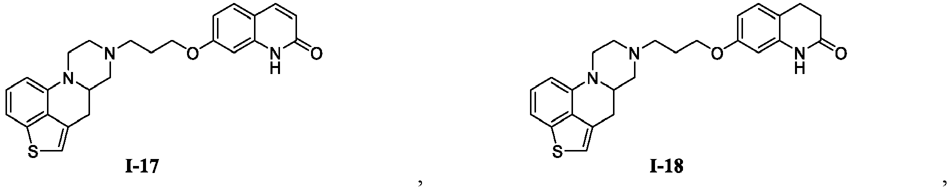 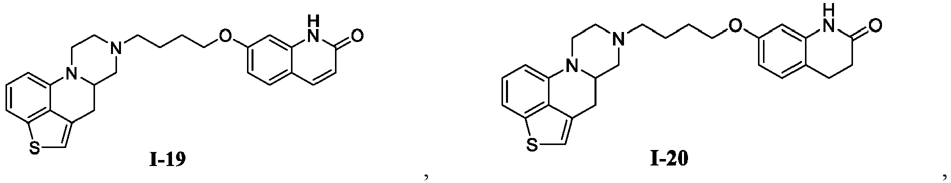 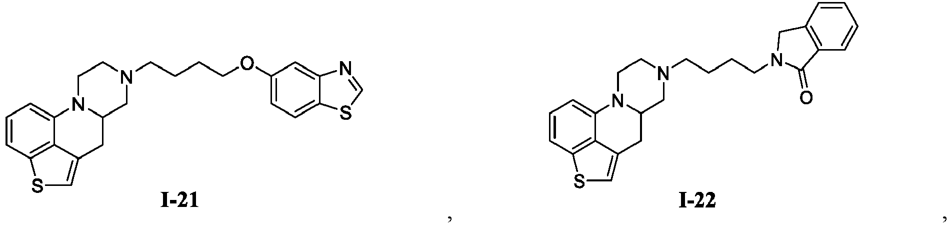 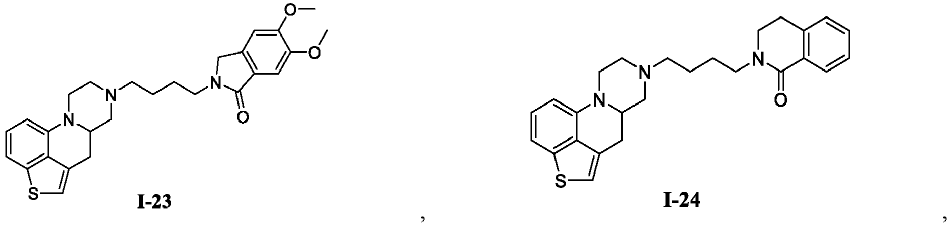 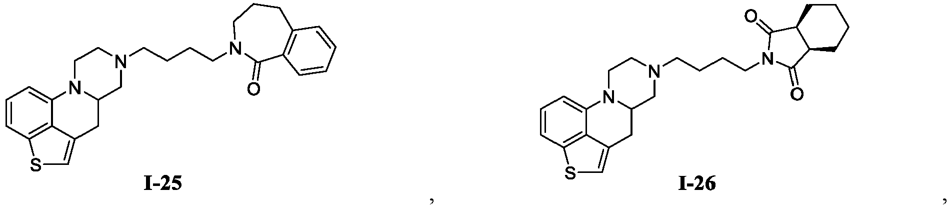 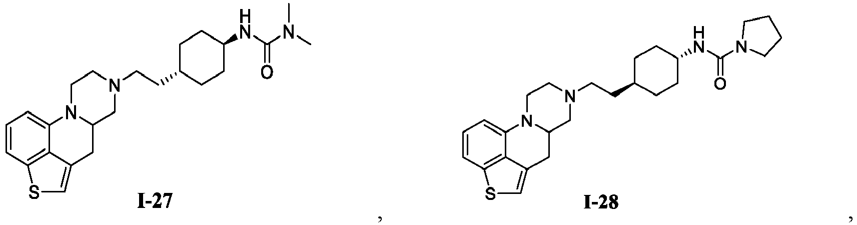 or

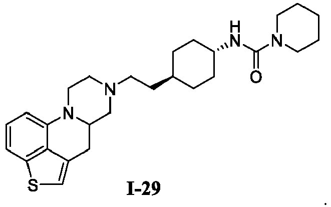

8. A compound of any one of the following formulas:

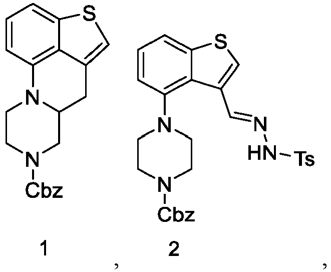

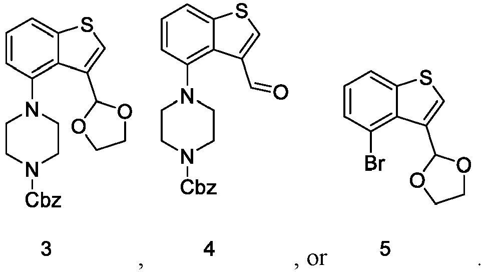

9. A pharmaceutical composition, wherein the pharmaceutical composition comprises the compound of formula I, the pharmaceutically acceptable salt thereof, the solvate thereof, or the solvate of the pharmaceutically acceptable salt thereof according to any one of claims 1 to 7, and a pharmaceutical excipient.

10. A use of substance **A** in the preparation of a G protein-coupled receptor agonist or partial agonist, wherein the substance **A** is the compound of formula I, the pharmaceutically acceptable salt thereof, the solvate thereof, or the solvate of the pharmaceutically acceptable salt thereof according to any one of claims 1 to 7, or the pharmaceutical composition according to claim 9; the G protein-coupled receptor is a dopamine receptor and/or a 5-hydroxytryptamine receptor;
preferably, the dopamine receptor is a dopamine D₂ receptor;
preferably, the 5-hydroxytryptamine receptor is a 5-hydroxytryptamine 5-HT_{2A} receptor and/or a 5-hydroxytryptamine 5-HT_{1A} receptor.

11. A use of substance **A** in the preparation of a medicament for treating and/or preventing a disease associated with a G protein-coupled receptor; the substance **A** is the compound of formula I, the pharmaceutically acceptable salt thereof, the solvate thereof, or the solvate of the pharmaceutically acceptable salt thereof according to any one of claims 1 to 7, or the pharmaceutical composition according to claim 9; the G protein-coupled receptor is a dopamine receptor and/or a 5-hydroxytryptamine receptor;
preferably, the dopamine receptor is a dopamine D2 receptor;
preferably, the 5-hydroxytryptamine receptor is a 5-hydroxytryptamine 5-HT_{2A} receptor and/or a 5-hydroxytryptamine 5-HT_{1A} receptor;
preferably, the disease associated with a G protein-coupled receptor refers to one or more than one of a neurodegenerative disease, a mental disorder, and a metabolic disease related to a mental disorder, such as Parkinson's disease, Alzheimer's disease, dementia, schizophrenia, bipolar disorder, depression, attention deficit hyperactivity disorder (ADHD), restless legs syndrome, Huntington's disease, erectile dysfunction, prolactinoma, or drug addiction.

12. A use of substance **A** in the preparation of a medicament for treating and/or preventing disease **M;** the substance **A** is the compound of formula I, the pharmaceutically acceptable salt thereof, the solvate thereof, or the solvate of the pharmaceutically acceptable salt thereof according to any one of claims 1 to 7, or the pharmaceutical composition according to claim 9; the disease **M** refers to one or more than one of a neurodegenerative disease, a mental disorder, and a metabolic disease related to a mental disorder;
preferably, in the use, the disease M is preferably Parkinson's disease, Alzheimer's disease, dementia, schizophrenia, bipolar disorder, depression, attention deficit hyperactivity disorder, restless legs syndrome, Huntington's disease, erectile dysfunction, prolactinoma, or drug addiction.

13. A preparation method for a compound of formula I, a pharmaceutically acceptable salt thereof, a solvate thereof, or a solvate of the pharmaceutically acceptable salt thereof, wherein the preparation method comprises the following steps:
in the presence of an alkaline reagent, carrying out an alkylation reaction between a compound of formula II and a compound of formula III in a solvent as shown below to obtain the compound of formula I; 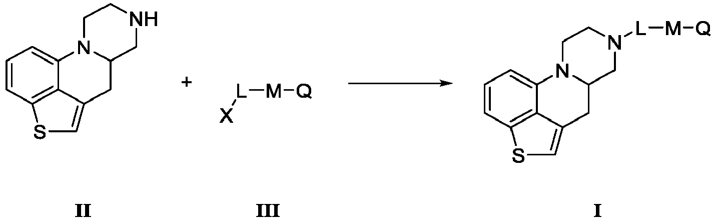
wherein X is halogen; L, M, Q, and R are as defined in any one of claims 1 to 7.
